# EUROPEAN PATENT SPECIFICATION

(11) **EP 1 986 697 B1**
(45) Date of publication and mention of the grant of the patent: **29.06.2016**
(21) Application number: 07751016.2
(22) Date of filing: 16.02.2007
(51) Int. Cl.: C12N 15/113

(54) **COMPOSITIONS AND METHODS FOR INHIBITING GENE SILENCING BY RNA INTERFERENCE**
ZUSAMMENSETZUNGEN UND VERFAHREN ZUR HEMMUNG EINER GENVERSTUMMUNG DURCH RNA-INTERFERENZ
COMPOSITIONS ET PROCÉDÉS PERMETTANT L'INHIBITION DE SILENÇAGE DE GÈNES PAR L'INTERFÉRENCE ARN

(30) Priority: 17.02.2006 US 774350 P; 22.09.2006 US 826702 P; 13.11.2006 US 865508 P; 19.12.2006 US 870815 P
(43) Date of publication of application: 05.11.2008
(73) Proprietor: GE Healthcare Dharmacon, Inc., Lafayette, CO 80026 (US)
(72) Inventor: VERMEULEN, Annaleen, Lafayette, CO 80026 (US); ROBERTSON, Barbara, Boulder, CO 80304 (US); BASKERVILLE, Scott, Louisville, CO 80027 (US); YAMADA, Christina, Boulder, CO 80404 (US); LEAKE, Devin, Denver, CO 80238 (US); FEDOROV, Yuriy, Superior, CO 80027 (US); KARPILOW, Jon, Boulder, CO 80304 (US); KHVOROVA, Anastasia, Boulder, CO 80305 (US)
(74) Representative: Maughan, Sophie Louise
(86) International application number: PCT/US2007/004223
(87) International publication number: WO 2007/095387

(56) References cited:
- WO-A1-92/17484
- WO-A1-94/01550
- WO-A1-94/23026
- WO-A1-03/050285
- WO-A2-2004/076622
- WO-A2-2005/079397
- US-A1- 2006 014 289
- US-B1- 6 369 038
- KRÜTZFELDT JAN ET AL.: "Silencing of microRNAs in vivo with 'antagomirs'." NATURE, vol. 438, no. 7068, 1 December 2005 (2005-12-01), pages 685-689, XP002600600 ISSN: 1476-4687
- LAKKA S.S. ET AL.: 'Inhibition of cathepsin B and MMP-9 gene expression in glioblastoma cell line via RNA interference reduces tumor cell invasion, tumor growth and angiogenesis' ONCOGENE vol. 23, 10 June 2004, pages 4681 - 4689, XP002998518
- STOVE ET AL. JOURNAL OF BIOTECHNOLOGY vol. 9, no. 5, 2006, pages 573 - 579
- ANDERSON ET AL. AIDS RESEARCH AND THERAPY vol. 2, no. 1, 2005, pages 1 - 12
- G. HARTH ET AL: 'Hairpin extensions enhance the efficacy of mycolyl transferase-specific antisense oligonucleotides targeting Mycobacterium tuberculosis' PROCEEDINGS OF THE NATIONAL ACADEMY OF SCIENCES vol. 104, no. 17, 24 April 2007, pages 7199 - 7204, XP055010941 DOI: 10.1073/pnas.0701725104 ISSN: 0027-8424

## Description

### CROSS-REFERENCE TO RELATED APPLICATIONS

This PCT patent application claims benefit of U.S. Provisional Serial No. 60/870,815, which was filed on December 19, 2006, U.S. Provisional Serial No. 60/865,508, which was filed on November 13, 2006, U.S. Provisional Serial No. 60/826,702, which was filed on September 22, 2006, and U.S. Provisional Serial No. 60/774,350, which was filed on February 17, 2006,

### FIELD OF THE INVENTION

The present invention relates to the field of modified polynucleotides configured to inhibit gene silencing by RNA interference. More particularly, the present invention relates to polynucleotides that can interact with target miRNA or siRNA so as to inhibit silencing of a target gene.

### BACKGROUND

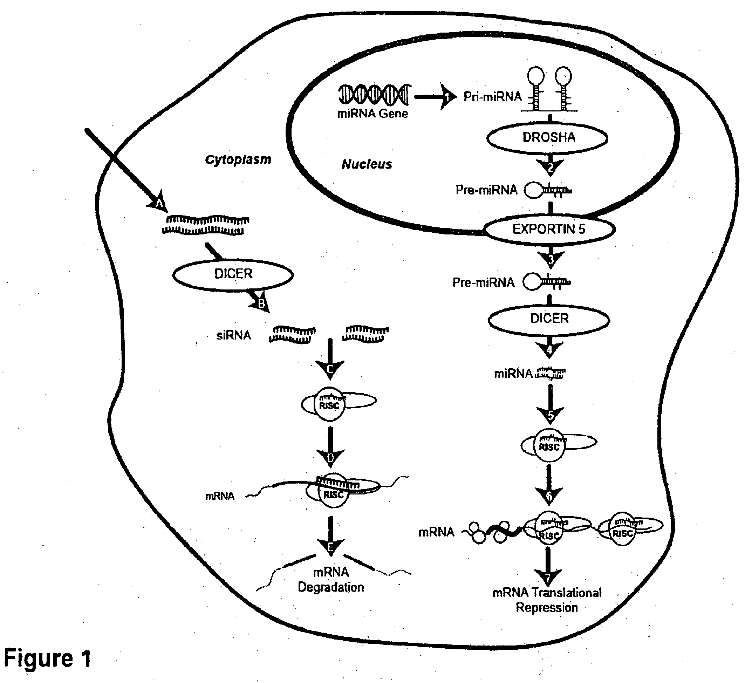

RNA interference (RNAi) is a near-ubiquitous pathway involved in post-transcriptional gene modulation. The key effector molecule of RNAi is the microRNA (miRNA or miR). These small, non-coding RNAs are transcribed as primary miRNAs (pri-miRNA, Figure 1) and processed in the nucleus by Drosha (*e.g.*, Type III ribonuclease) to generate pre-miRNAs. The resulting hairpin molecules are then transported to the cytoplasm and processed by a second nuclease (Dicer) before being incorporated into the RNA Induced Silencing Complex (RISC). Interactions between the mature miRNA-RISC complex and messenger RNA (mRNA), particularly between the seed region of the miRNA guide strand (*e.g.*, 5' nucleotides 2-7) leads to gene knockdown by transcript cleavage and/or translation attenuation. While study of native substrates (*e.g.*, miRNA) has garnered considerable interest in recent years, the RNAi pathway has also been recognized as a powerful research tool. Small double stranded RNAs (*e.g*., small interfering RNAs or siRNA) generated by synthetic chemistries or enzymatic methods can enter the pathway and target specific gene transcripts for degradation. As such, the RNAi pathway serves as a potent tool in the investigation of gene function, pathway analysis, and drug discovery.

To better understand the mechanism of RNAi, the targets of microRNAs, and the roles that miRNAs and their targets play in disease, cellular differentiation and homeostasis, development of molecular tools, such as miRNA inhibitors and mimics, are valuable. Inhibitors should be potent, stable, highly specific, and easily introduced into cells under *in vivo* (*e.g.,* whole animal and in culture), and induce silencing for extended periods of times.

Several groups have previously described a class of miRNA inhibitors (see Meister, G. et al, (2004) RNA 10(3):544-50; Hutvagner, G. et al. (2004) PLoS Biol. Apr;2(4):E98. Epub 2004 Feb 24). These molecules are single stranded, range in size from 21-31 nucleotides (nts) in length, and contain O-methyl substitutions at the 2' position of the ribose ring. More recently, a variant of this original design called "antagomirs" were developed and include the addition of a cholesterol to single stranded 21-23 nt inhibitors (Krutzfeldt, J. et al (2005) Nature 438:685-689), and novel designs that include the incorporation of locked nucleic acids (LNAs, see Orom et al, (2006) Gene 372:137-141).

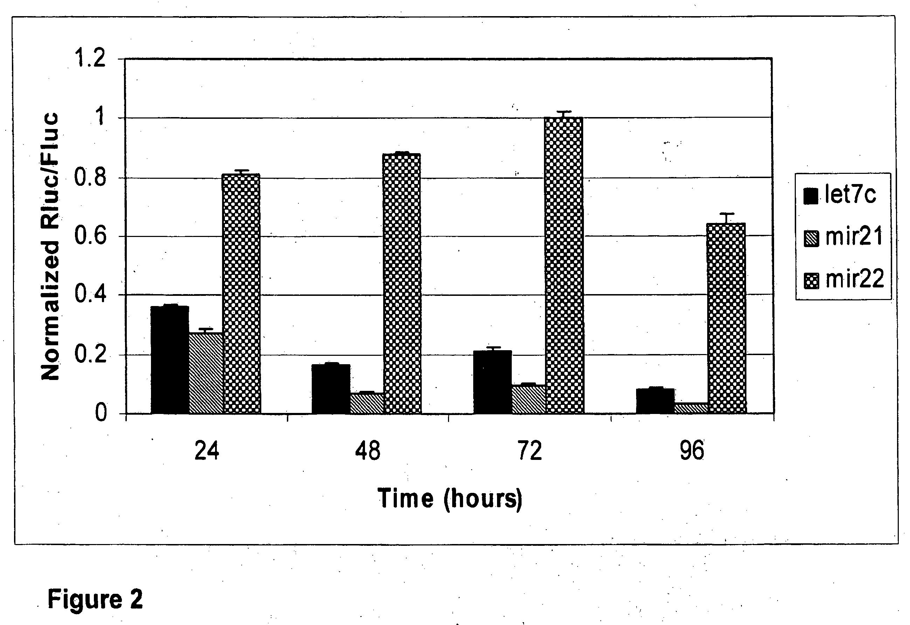

Short, single stranded RNAi inhibitors and single stranded RNAi inhibitors conjugated to cholesterol suffer from dissimilar shortcomings. The short single stranded molecules described by Hutvagner (2004) and Meister (2004) are as a whole fairly ineffectual in inhibiting the intended target. As shown in Figure 2, inhibitors of this design predominantly induce low to moderate (*e.g.,* 0-30%) levels of silencing when transfected at moderate (*e.g*., 25-50nM) concentrations and sustain silencing for limited periods (*e.g*., about 1-3 days). While conjugation of cholesterol to single stranded 21-23 nt inhibitors (*e.g*., antagomirs) alters pharmacokinetic behavior and secures some degree of functionality *in vivo* in mouse livers, the manufacturing of these molecules is tedious. Specifically, due to the single stranded nature of the antagomir design, each of the hundreds of sequences must be synthesized individually and separately conjugated with cholesterol. For these reasons, designs that exhibit enhanced activity and allow for streamlined manufacturing procedures are highly desirable.

Krützfeld, J. et al: "silencing of micro-RNAs in vivo with antagomirs" NATURE, vol. 438, no. 7068, December 2005, pages 685-689 and WO 2007/079397 disclose antisense oligonucleotides directed towards a microRNA sequence. WO 94/01550 discloses different ways of stablising antisense oligonucleotides. WO 94/23026 discloses an antisense oligonucleotide stabilized by introducing two hairpins at its 5' and 3' ends.

### SUMMARY OF THE INVENTION

To address the shortcomings of the currently available miRNA inhibitors, the inventors have identified a design that represents a double stranded RNAi inhibitor that significantly enhances overall functionality. Incorporation of regions of double stranded oligonucleotides into the inhibitor design greatly increases overall potency and longevity of the molecules without altering specificity. In addition, the second design is compatible with manufacturing processes that greatly minimize the complications associated with previous designs. The polynucleotides of the double stranded inhibitors can be modified or unmodified.

In the most general of terms, the molecules of the invention can be used to inhibit gene silencing by the RNAi pathway. The inhibitors described herein can inhibit endogenous targets, including but not limited to microRNAs, or piRNAs, or can be used to inhibit the effects of exogenously introduced molecules, such as synthetic siRNAs, siRNAs expressed from vector constructs (*e.g*., viral expression systems), or siRNAs generated by enzymatic methods. In addition, the molecules of the invention can be used to inhibit microRNAs that are expressed by pathogens. Inhibition is specific, potent, prolonged, and can be performed on a single target or multiple targets simultaneously.

The molecules of the invention can be RNA, modified RNA, DNA, modified DNA, or any combination thereof. Nucleotide modifications applicable for the inhibitors of the invention are disclosed in WO2005/097992 and WO2005/078094. In addition, the molecules of the invention can be conjugated to one or more molecules that enhance cellular delivery. This conjugate can be attached directly to the inhibitor or associated through a linker molecule.

The present invention provides miRNA inhibitors in accordance with the claims.

In one embodiment, one or more of the nucleotides of the inhibitor molecule of the invention are modified and/or contain a conjugate. The preferred modifications include: (a) 2'-O-alkyl modifications; (b) 2'-oithoester modifications, and/or (c) 2'-ACE *(i.e.,* 2'-O-acetoxyethoxy) modifications of the ribose ring of some or all nucleotides. Preferably, the oligonucleotide(s) are modified polyribonucleotides, and the conjugates are hydrophobic molecules. More preferably, the modification is a 2'-O-alkyl modification of the ribose ring of some or all nucleotides and the conjugate is cholesterol. Conjugates can be attached directly to the 5' end, 3' end, or internal regions of any of the oligonucleotides or be attached through a linker molecule associated with the 5' end, 3' end, and/or internal regions of any of the oligonucleotides of the invention.

According to another embodiment, the invention describes methods of inhibiting the ability of an miRNA, a piRNA, or siRNA to modulate gene expression using compositions described in the previous embodiments.

Also described herein are kits and pharmaceutical compositions containing the inventive inhibitors.

### BRIEF DESCRIPTION OF THE FIGURES AND TABLES

The preferred embodiments of the present invention have been chosen for purposes of illustration and description, but are not intended to restrict the scope of the invention in any way. The benefits of the preferred embodiments of certain aspects of the invention are shown in the accompanying figures, wherein:
Figure 1 is a diagram showing some of the underlying steps in the RNAi pathway.
Figure 2 is a bar graph showing the performance of single stranded, 21 nucleotide, 2'-O-methyl modified inhibitor molecules targeting let-7c, miR21, and miR22. Sequences used in these studies include: let-7c: 5'-AACCAUACAACCUACUACCUCA (SEQ ID NO: 1); miR-21: 5'-UCAACAUCAGUCUGAUAAGCUA (SEQ ID NO: 2); and miR-22: 5'-ACAGUUCUUCAACUGGCAGCUU (SEQ ID NO: 3) inhibitors. All sequences used in this study were fully 2'-O-methylated.

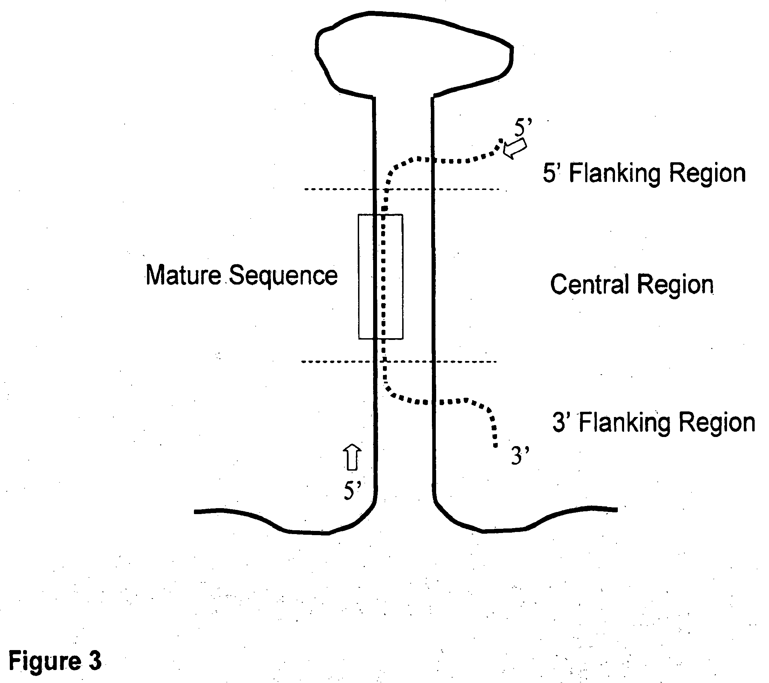

Figure 3 is a depiction of the single stranded inhibitor design as it relates to primary miR sequence and structure. Box indicates the position of the mature miRNA. Central, 5' flanking, and 3' flanking regions are indicated as dotted line.

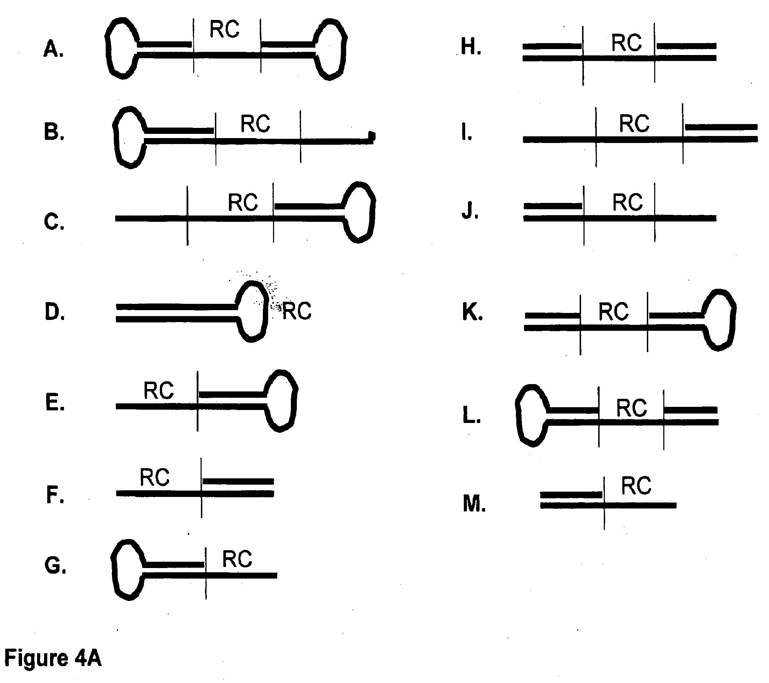

Figure 4A shows schematic illustrations of embodiments of various inhibitors of the present invention that contain double stranded regions. All of the diagrammed structures contain 2'-O-methyl modifications and can be modified with one or more conjugates. (A) an inhibitor molecule that has an RC region and hairpin structures in both the 5' and 3' flanking regions, (B) an inhibitor molecule with an RC region and a hairpin structure in the 5' flanking regions and an unstructured (not duplexed) 3' flanking region, (C) an inhibitor molecule with an RC region and a hairpin structure in the 3' flanking regions and an unstructured 5' flanking region, (D) an inhibitor molecule that has an RC region and forms a double stranded region by having the 5' and 3' flanking regions anneal together, (E) an inhibitor molecule that has an RC region and a hairpin structure in the 3' flanking region, but no 5' flanking region, (F) an inhibitor molecule that has an RC region and a double stranded region in the 3' flanking region resulting from annealing to a complementary sequence, but no 5' flanking region, (G) an inhibitor molecule that has an RC region, a hairpin structure in the 5' flanking region, but no 3' flanking region, (H) an inhibitor molecule that has an RC region and double stranded regions in the 5' and 3' flaking regions resulting from addition of second and third oligonucleotides that anneal to the flanks, (I) an inhibitor molecule that has an RC region, a 5' flanking region, and a double stranded 3' flanking region resulting from addition of a complementary oligonucleotide, (J) an inhibitor molecule that has an RC region, a 3' flanking region, and a double stranded 5' flanking region resulting from addition of a complementary oligonucleotide, (K) an inhibitor with an RC region, a double stranded 5' flanking region resulting from addition of a complementary oligonucleotide, and a 3' flanking region that contains a hairpin, (L) an inhibitor with an RC region, a double stranded 3' flanking region resulting from addition of a complementary oligonucleotide, and a 5' flanking region that contains a hairpin, and (M) an inhibitor molecule that contains an RC region and a double stranded 5' flanking region resulting from addition of a complementary oligonucleotide, but no 3' flanking region. In all cases, RC region is synonymous with "central region". The vertical lines demark the boundaries between regions.

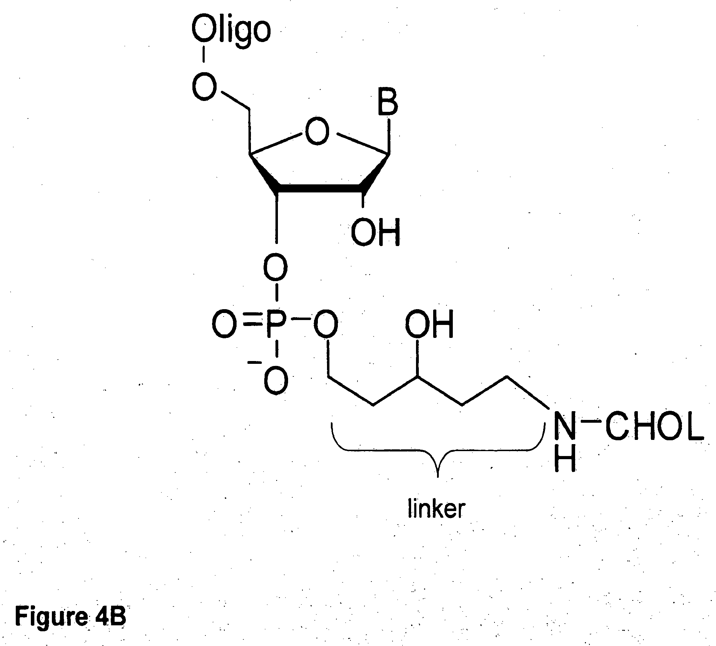

Figure 4B is an illustration of an embodiment of an inhibitor having a conjugate linked to an oligonucleotide through a linker.

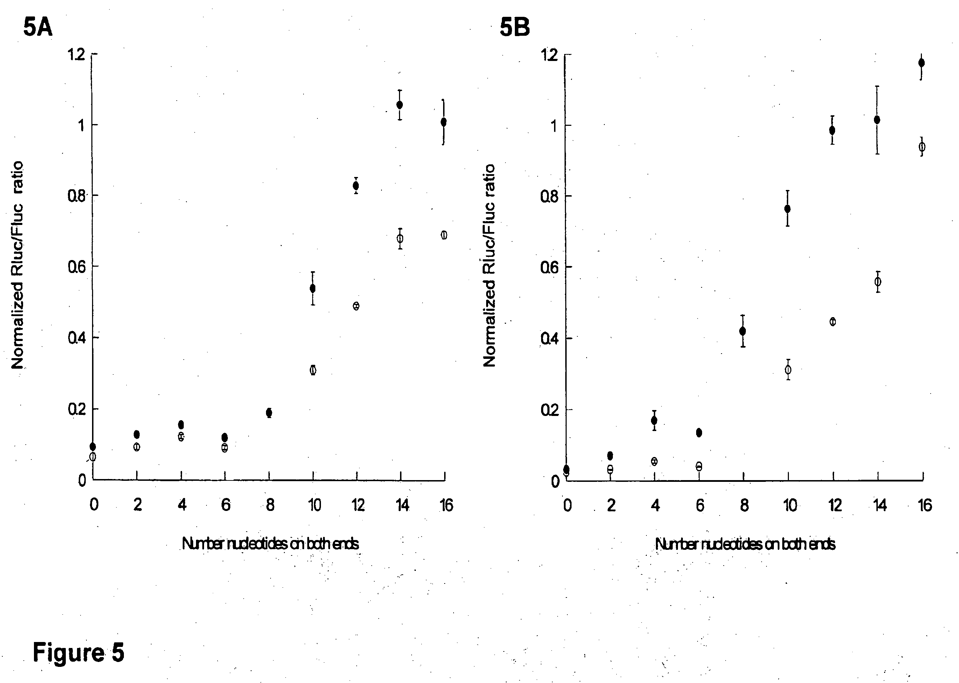

Figures 5A-5B are graphs showing that overall length of 2'-O-methylated miRNA inhibitors affects functionality. Inhibitors targeting let-7c (Figure 5A) and miR-21 (Figure 5B) were assayed by co-transfecting into HeLa cells with reporters at 25 (open circles) and 50 nM (filled circles) concentrations. The dual luciferase ratio was measured 48 hrs post-transfection to determine the effectiveness of each design. The experiment demonstrates a strong correlation between functionality and length.

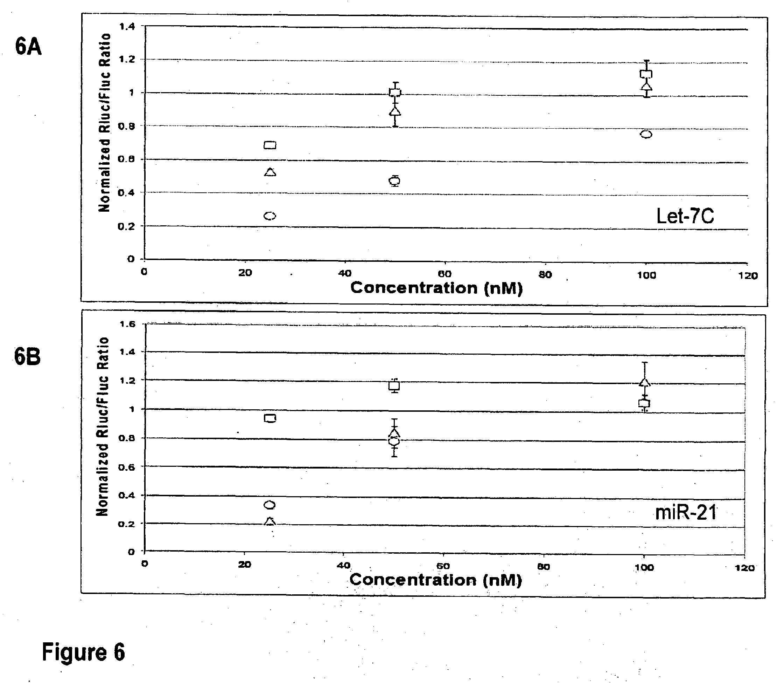

Figures 6A-6B are graphs showing the effects of adding flanking sequences to varying positions. Accordingly, Figures 6A-6B depict the effectiveness of Let-7c (Figure 6A) and miR-21 (Figure 6B) 2'-O-methyl modified inhibitors that have 1) sequences complementary to the mature miRNA, plus 2) sequences complementary to regions bordering the mature miRNA sequence, attached on the 5' (circles), 3' (triangles), and 5' and 3' ends (squares). Results demonstrate that the 16 + RC + 16 design consistently provides superior performance.

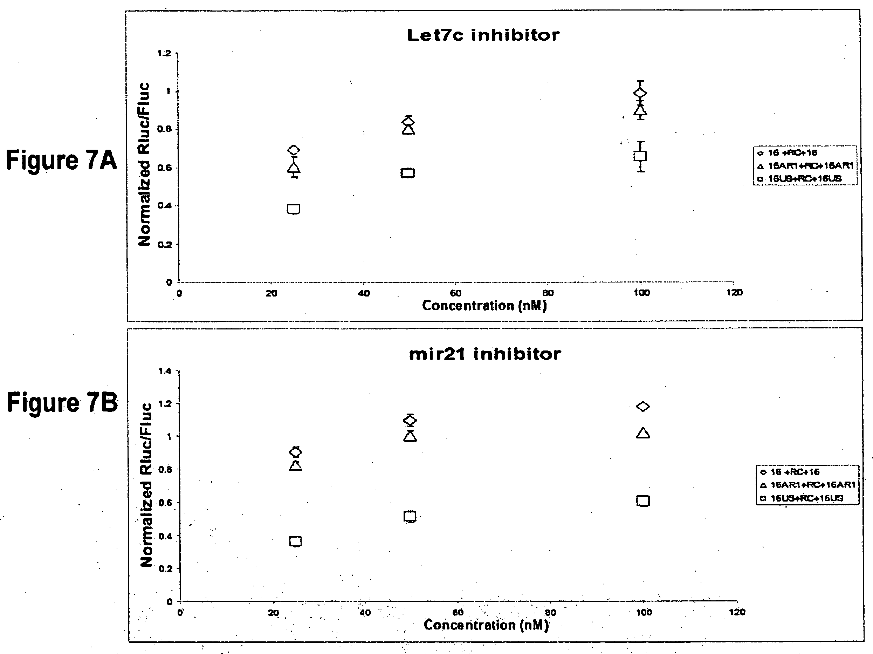

Figures 7A-7B are graphs showing the effect of inhibitor flanking sequence composition on inhibitor functionality.

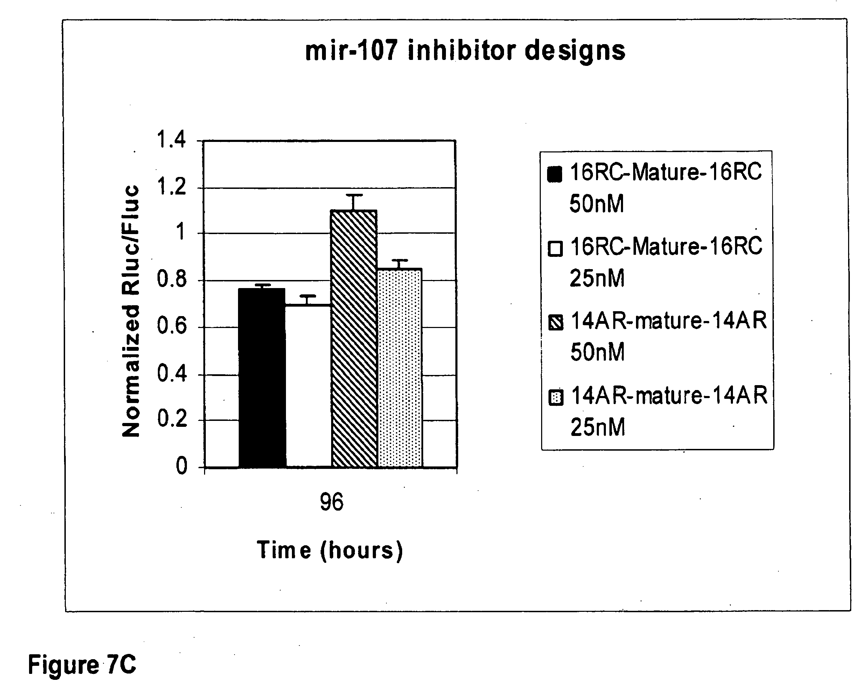

Figure 7C is a graph showing the effect of inhibitor flanking sequence composition on inhibitor functionality.

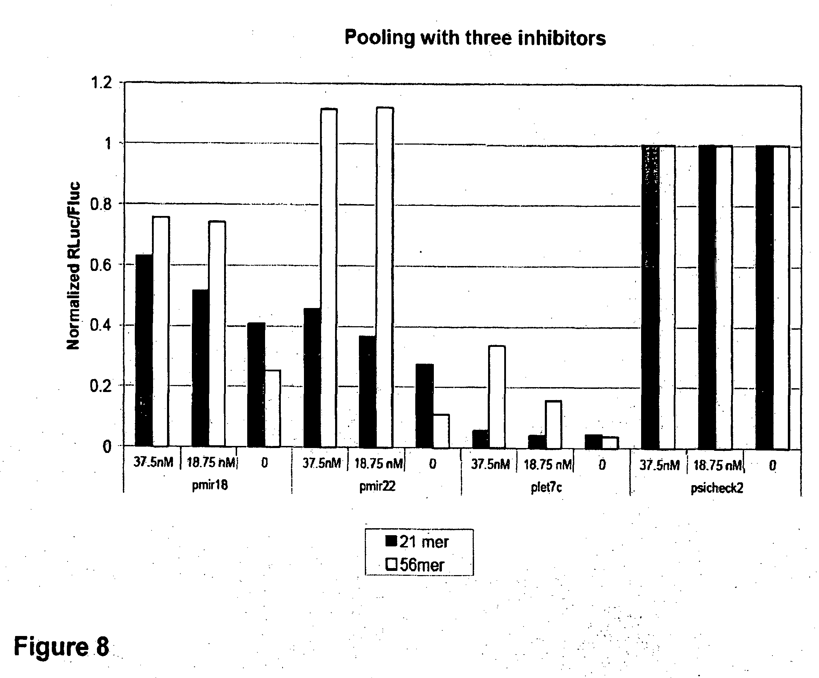

Figure 8 is a graph showing the effect of multi-miRNA targeting with long and short single stranded inhibitors. The graph shows the differences in the ability of 21 nts 2'-O-methyl inhibitors and 56 nts 2'-O-metbyl modified inhibitors (*e.g*., 28 nts central region, 14 nts 5' flanking region (5'-AGCUCUCAUCCAUG; SEQ ID NO: 4) and 14 nts 3' flanking regions (5'-GUACCUACUCUCGA; SEQ ID NO: 5)) targeting miR-18, miR-22, and Let-7c to simultaneously inhibit multiple miRNAs.

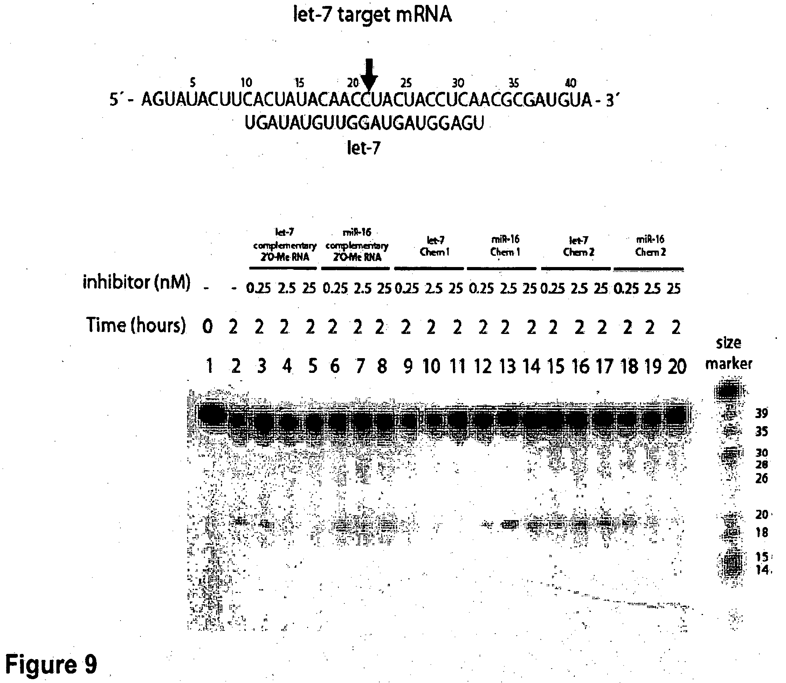

Figure 9 compares the effectiveness of 2'-ACE and 2'-O-Me modified 31 nts inhibitors in preventing the cleavage of an artificial substrate.

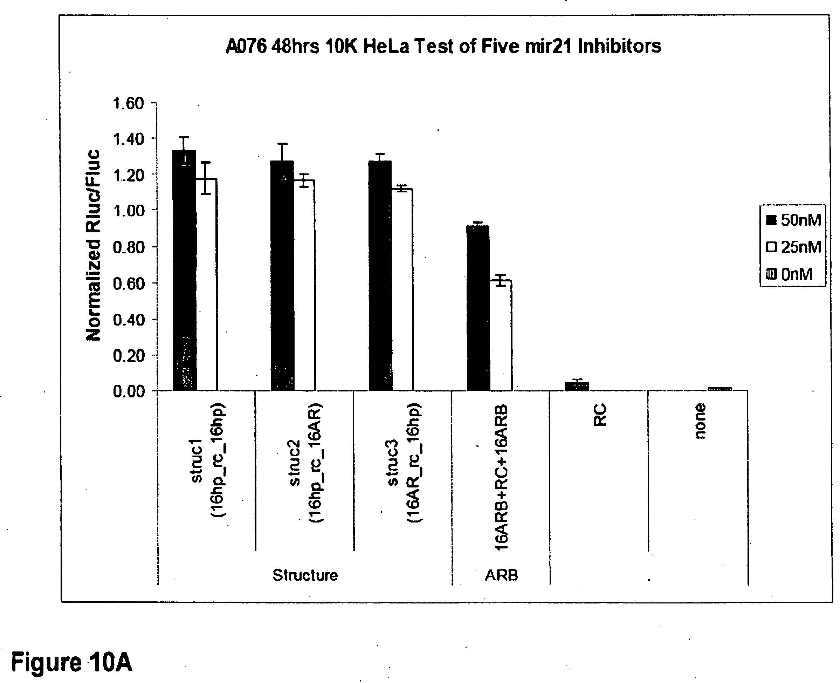

Figures 10A-10B are bar graphs showing the performance of five different inhibitor designs at 50 and 25 nM and targeting: A) miR21, and B) let7c miRNAs. "Struc1" = 5' and 3' flanking region hairpins. "Struc2" = 5' flanking region hairpin, 3' flanking region unstructured. "Struc3" = 5' flanking region unstructured, 3' flanking region hairpin. "ARB" = unstructured 5' and 3' flanking regions. "RC" = short (21 nt) reverse complement. The study shows that incorporation of double stranded regions in inhibitor design enhances functionality.

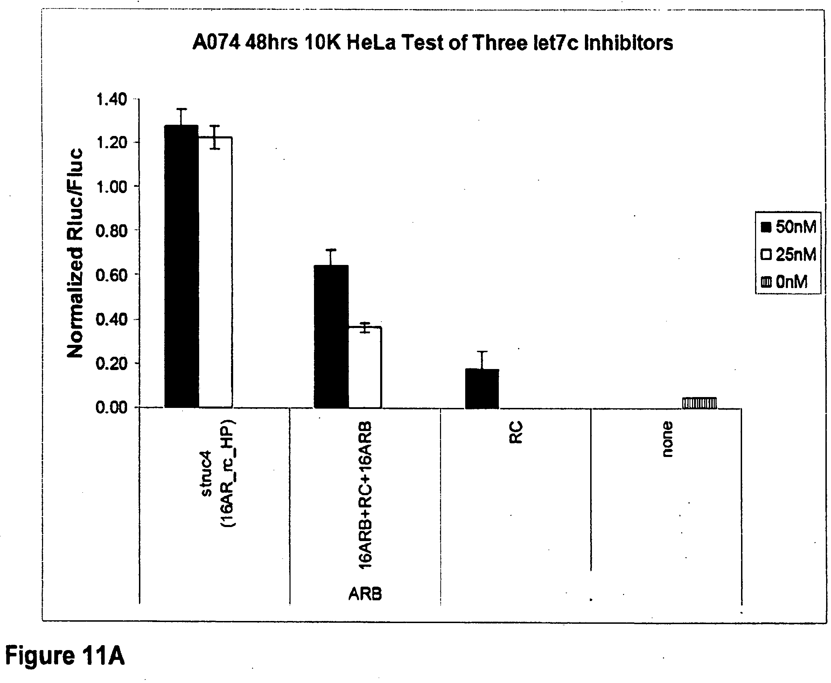

Figures 11A-11B are bar graphs showing the performance of three different inhibitor designs targeting: A) miR21, and B) let7c miRNAs at 50 and 25 nM. "Struc4" represents inhibitor designs that include 5' and 3' flanking region annealing (also referred to as "lollipop" designs). "ARB" represents inhibitors of equivalent length that do not form lollipop structures. "RC" = short 21 nt reverse complement. The study demonstrates that in the absence of RC secondary structures, incorporation of double stranded regions into inhibitor design by this method can enhance functionality.

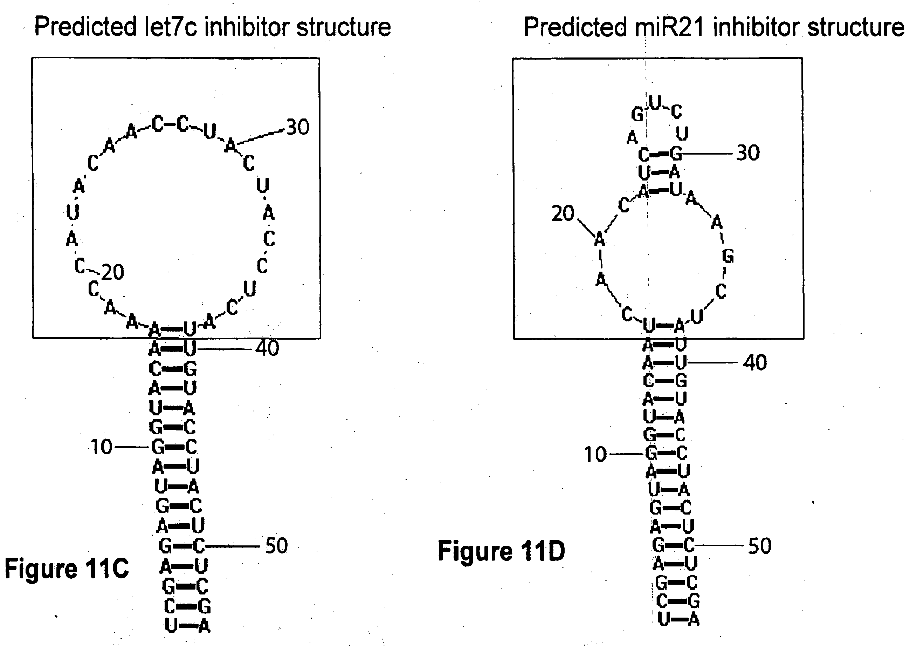

Figure 11C is a schematic representation of a predicted folding structure of an embodiment of a Let7c inhibitor (5'-UCGAGAGUAGGUACAAAACCAACAACCUACUACCUCAUUGUACCUACUC UCGA; SEQ ID NO: 6). Highlighted regions represent the position of the critical reverse complement.
Figure 11D is a schematic representation of a predicted folding structure of the miR21 inhibitor (5'-UCGAGAGUAGGUACAAUCAACAUCAGUCUGAUAAGCUAUUGUACCUAC UCUCGA; SEQ ID NO: 7). Highlighted regions represent the position of the critical reverse complement.

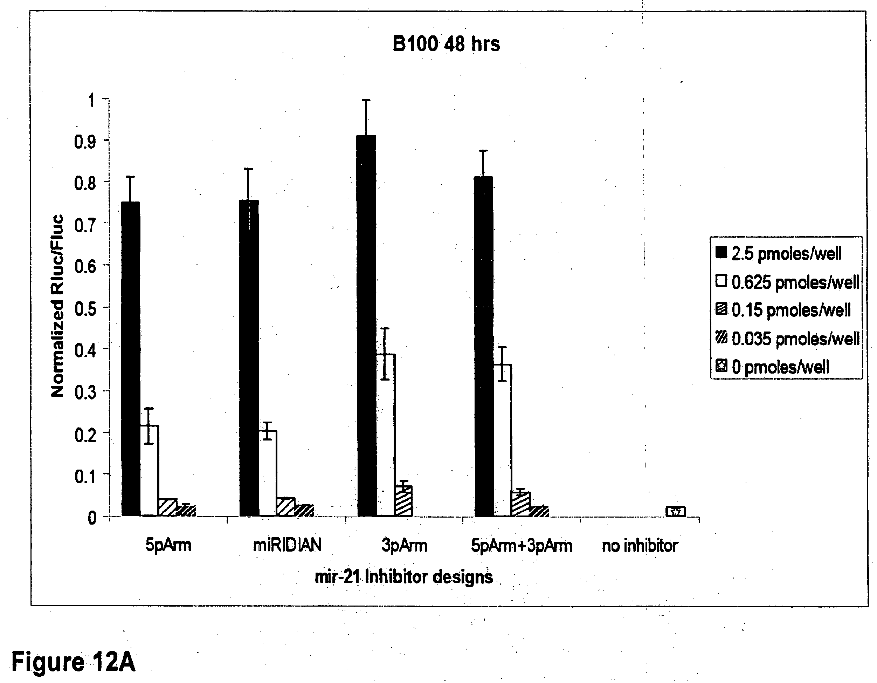

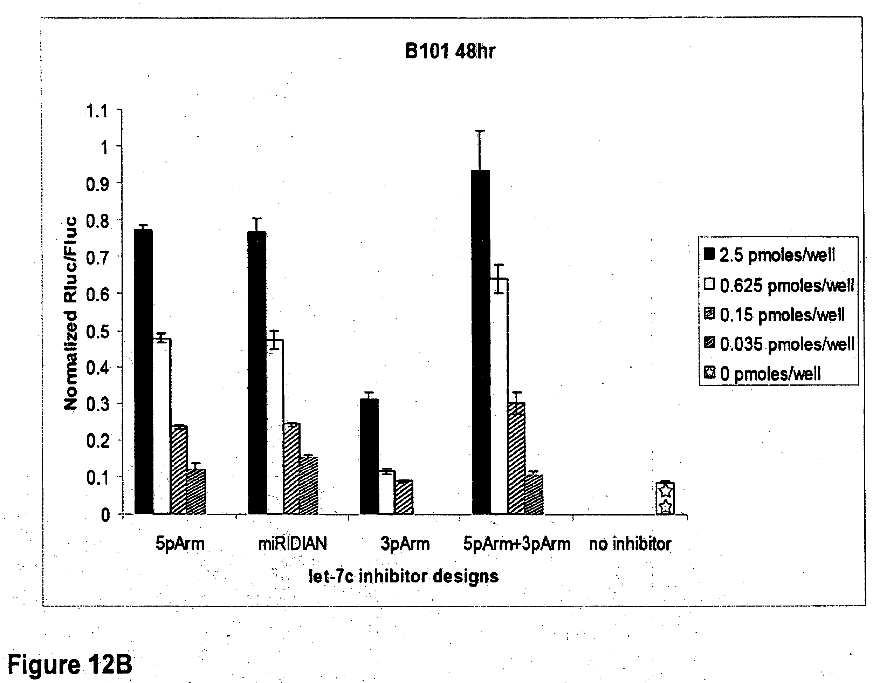

Figures 12A-12B are bar graphs showing the performance of four different inhibitor designs targeting: A) miR21 (Figure 12A), and B) let7c miRNAs (Figure 12B). "5pARM" represents inhibitor designs that include a first oligonucleotide containing a central region, a 5' flanking region, and a 3' flanking region plus a first enhancer sequence capable of annealing to the 5' flanking region. "3pARM" represents inhibitor designs that include a first oligonucleotide containing a central region, a 5' flanking region, and a 3' flanking region plus a second enhancer sequence capable of annealing to the 3' flanking region. "5pARM+3pARM" represents inhibitor designs that include a first oligonucleotide (containing a central region, a 5' flanking region, and a 3' flanking region) plus a first enhancer sequence capable of annealing to the 5' flanking region, and a second enhancer sequence capable of annealing to the 3' flanking region. "miRIDIAN" represents single stranded (1^{st} oligonucleotide) inhibitors of equivalent length and sequence. Study demonstrates the enhanced potency of inhibitors having double stranded structures.

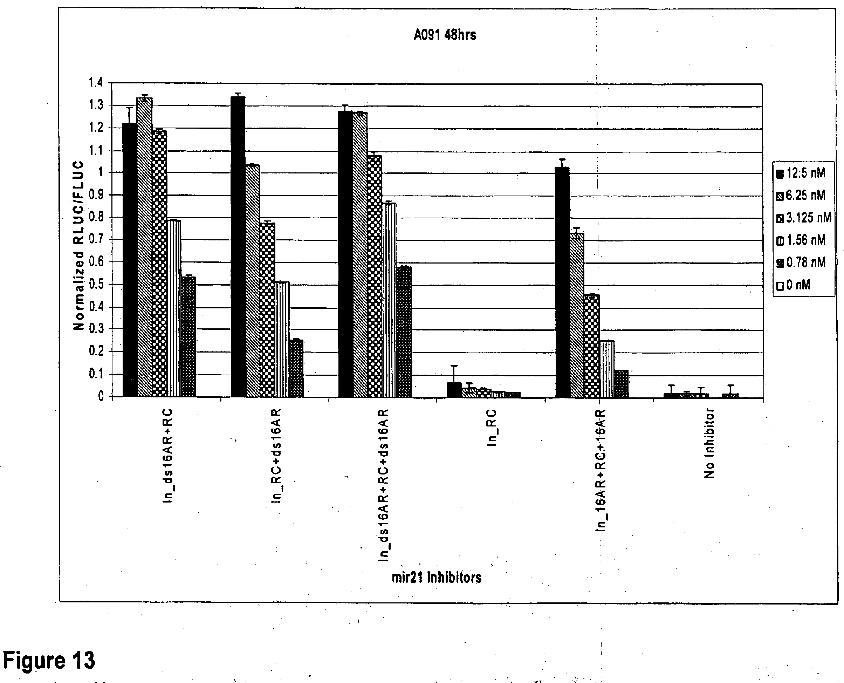

Figure 13 is a bar graph showing the performance of five different inhibitor designs targeting miR21. From left to right: 1) a truncated inhibitor consisting of 5' flanking region-a central region plus an enhancer sequence annealed to the 5' flank (ds16AR+RC); 2) a truncated inhibitor consisting of a central region & a 3' flanking region plus an enhancer sequence annealed to the 3' flank (RC + ds16AR); 3) an inhibitor consisting of 5' flanking region-a central region-a 3' flanking region plus an enhancer sequence annealed to the 5' and 3' flanking regions (ds16AR+RC+ds16AR); 4) a short, 21 nt single stranded reverse complement (RC); and 5) a long single stranded inhibitor containing a 5' flanking region, a central region, and a 3' flanking region (16AR+RC+16AR). Study demonstrates the enhanced potency of double stranded inhibitors.

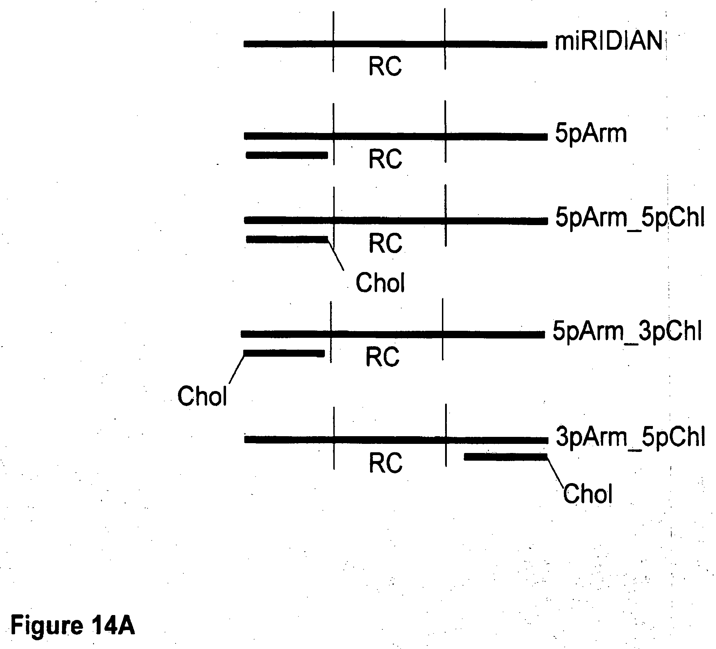

Figures 14A-14B are schematic representations of embodiments of inhibitors having conjugate structures that were tested in Example 10.

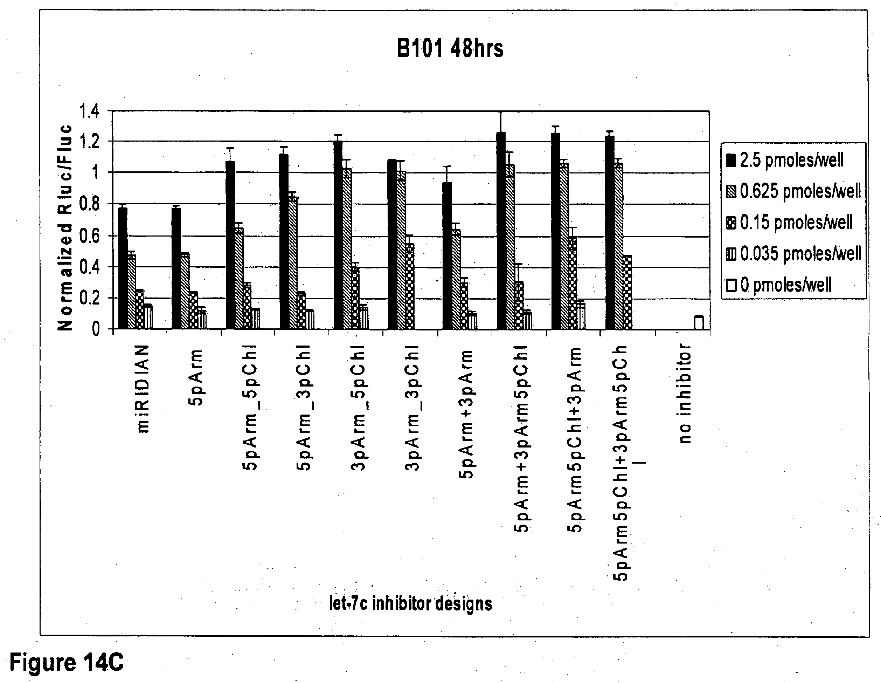

Figures 14C-14F are bar graphs showing the performance of multiple double stranded inhibitor designs conjugated to cholesterol (C and D), or Cy3 (E and F). All designs were tested for efficacy against let-7c and miR21. For the cholesterol experiments, long single stranded inhibitors (5' flanking region-central region-3' flanking region, miRIDIAN), were compared to double stranded inhibitors conjugated to cholesterol including those that contain: 1) a first oligonucleotide plus a 5' enhancer sequence with a 5' cholesterol modification (5pArm_5pChl), 2) a first oligonucleotide plus a 5' enhancer sequence with a 3' cholesterol modification (5pArm_3pChl), 3) a first oligonucleotide plus a 3' enhancer sequence with a 5' cholesterol modification (3pArm_5pChl), 4) a first oligonucleotide plus a 3' enhancer sequence with a 3' cholesterol modification (3pArm_3pChl), 5) a first oligonucleotide plus a 3' enhancer sequence with a 5' cholesterol modification plus a 5' enhancer sequence(5pArm+3pArm5pChl), 6) a first oligonucleotide plus a 5' enhancer sequence with a 5' cholesterol modification plus a 3' enhancer sequence (5pArm5pChl+3pArm), and 7) a first oligonucleotide plus both 3' and 5' enhancer sequences, both of which are modified with a 5' cholesterol modification. For the Cy3 experiments, simple, single stranded (miRIDIAN) designs were compared with double stranded designs having 5' flanking regions- central regions- 3' flanking regions plus enhancer sequences annealed to both flanks (5pArm-3pArm), and the same double stranded designs having Cy3 conjugates, including: 1) 5' flanking regions- central regions- 3' flanking regions plus enhancer sequences annealed to both flanks with the first enhancer sequence having a Cy3 on the 5' terminus (SpArm5pCy3+3pArm), or 2) 5' flanking regions- central region- 3' flanking regions plus enhancer sequences annealed to both flanks with the second enhancer sequence having a Cy3 on the 5' terminus (SpArm+3pArm5pCy3). Study demonstrates the superior performance of double stranded inhibitors and double stranded inhibitors with cholesterol or Cy3 conjugates.

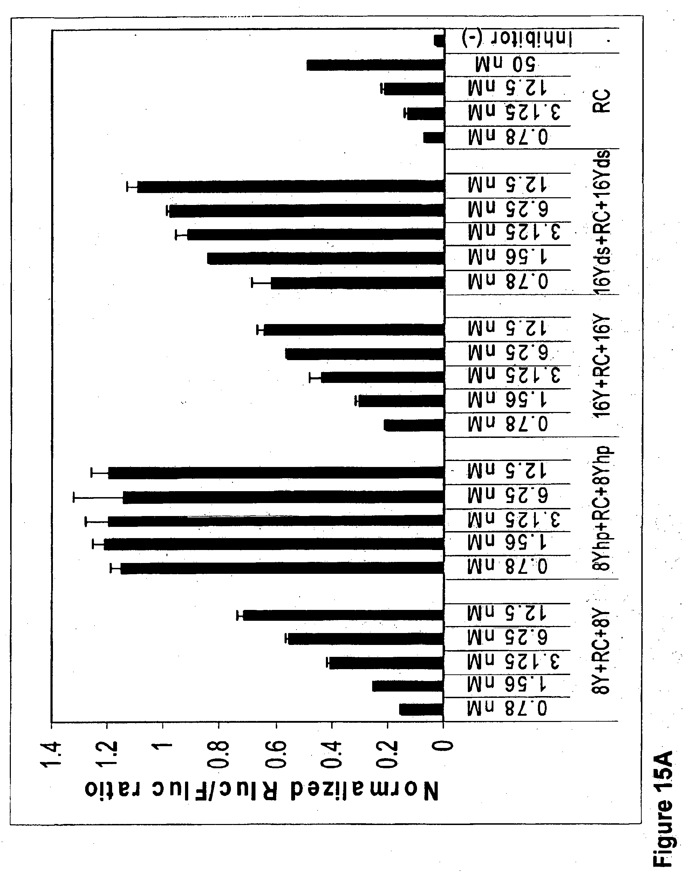

Figure 15A is a bar graph comparing the performance of simple single stranded miR21-targeting inhibitor designs having: 1) 8 polypyrimidine nucleotide flanks (8Y+RC+8Y), or 2) 16 polypyrimidine nucleotide flanks (16Y+RC+16Y), with 3) double stranded polypyrimidine flanks having stem-loop structures containing 8 polypyrimidine base pairs in the stems (8Yhp+RC+8Yhp), or 4) 16 polypyrimidine flanks with complementary enhancer sequences (16Yds+RC+16Yds). The graph demonstrates the superior performance and sequence independence of double stranded inhibitor designs.

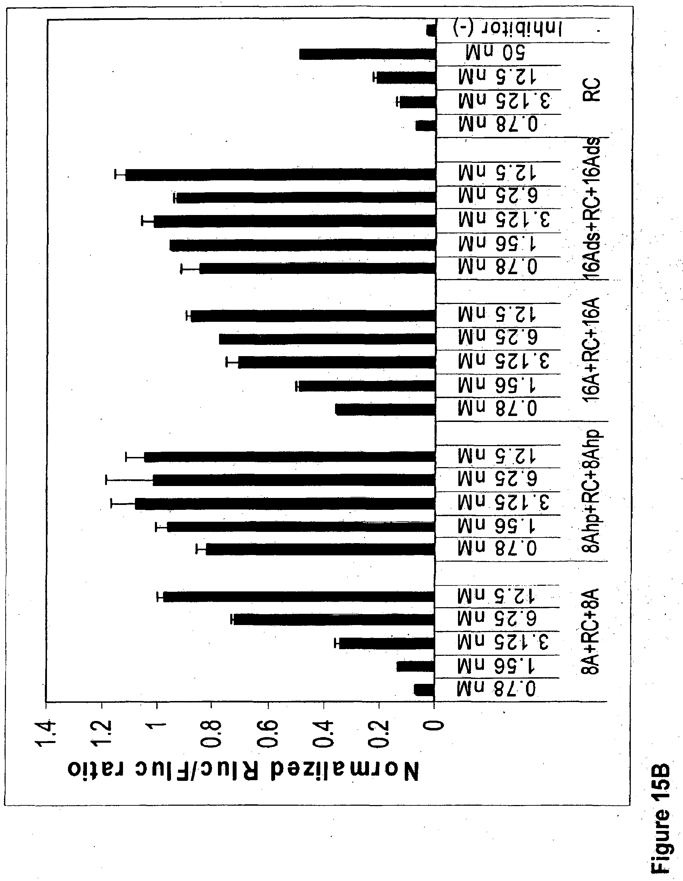

Figure 15B is a bar graph comparing the performance of simple single stranded inhibitor designs having: 1) 8 arbitrary nucleotide flanks (8A+RC+8A), or 2) 16 arbitrary nucleotide flanks (16A+RC+16A), with 3) double stranded arbitrary flanks having stem-loop structures containing 8 arbitrary base pairs in the stems (8Ahp+RC+8Ahp), or 4) 16 double stranded arbitrary flanks resulting from addition of enhancer sequences (16Ads+RC+16Ads). The graph demonstrates the superior performance and sequence independence of double stranded inhibitor designs.

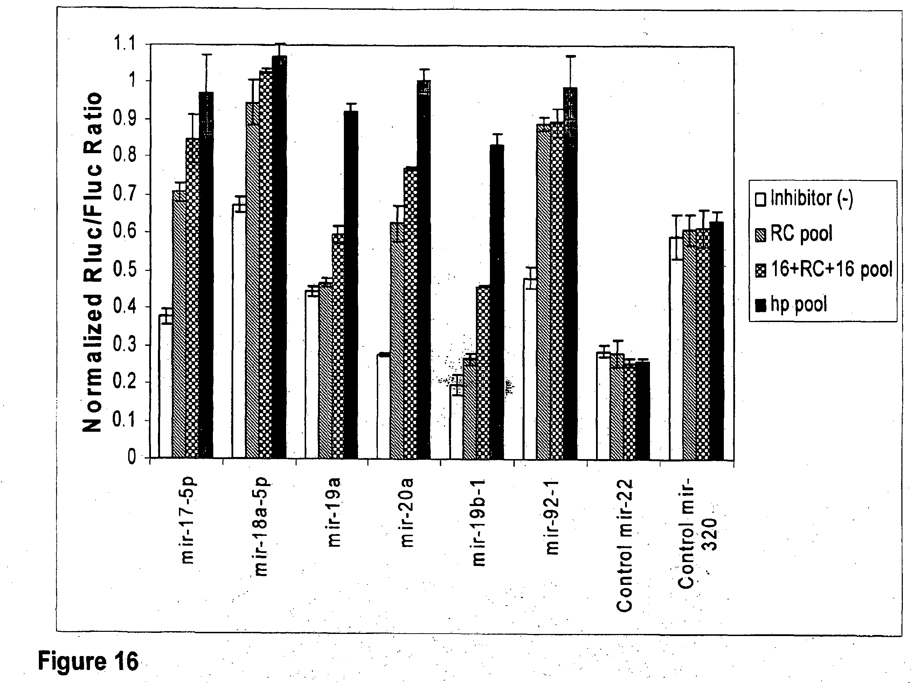

Figure 16 is a graph of a comparison between short 21 nt single stranded inhibitors, long single stranded inhibitors, and double stranded (hairpin) inhibitors in multi-miR studies. The study demonstrates that unlike single stranded inhibitor designs, double stranded inhibitors of the invention are capable of multi-miR knockdown.

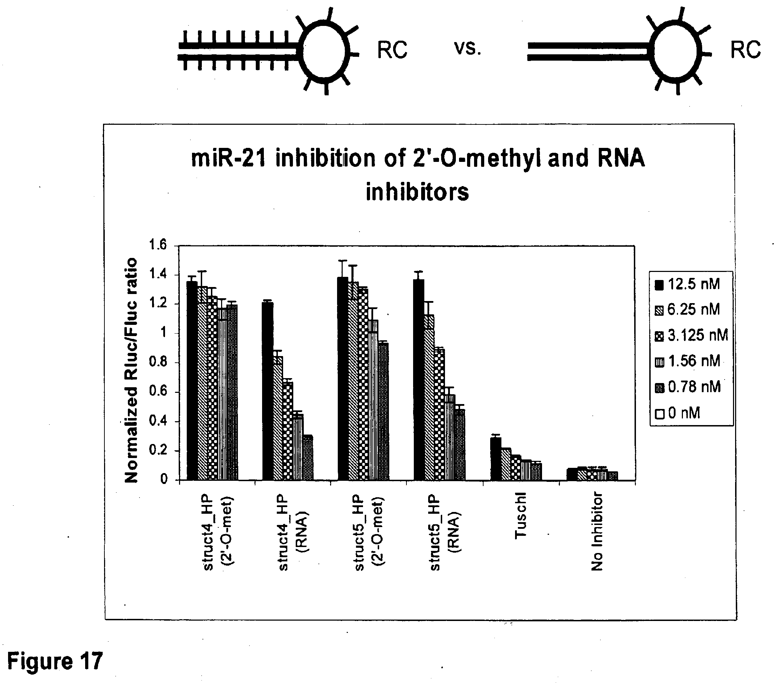

Figure 17 is a graph of a comparison study to test the performance of double stranded inhibitors containing mixtures of modified and unmodified nucleotides. miR21-targeting inhibitors in which: 1) the 3' flanking region was altered so as to promote annealing with the 5' flanking region (structure 4), or 2) the 5' flanking region was altered so as to promote annealing with the 3' flanking region (structure 5), were designed. Both designs were tested as: 1) fully 2'-O-methylated double stranded inhibitors, or 2) partially 2'-O-methylated double stranded inhibitors. Short single stranded, fully 2'-O-methylated inhibitors, targeting miR21 were incorporated in the experiment for comparison. Results demonstrate that both fully and partially modified double stranded inhibitor designs exhibit superior performance to short, single-stranded 21 nucleotide designs.

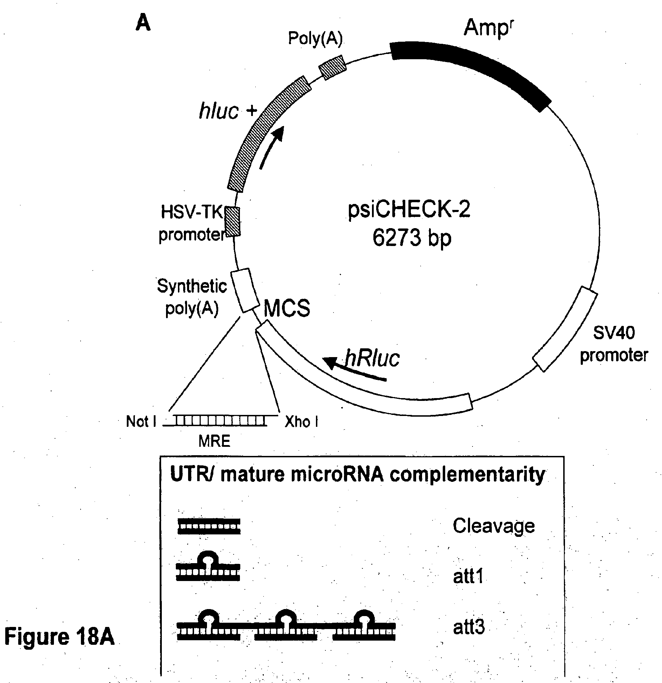

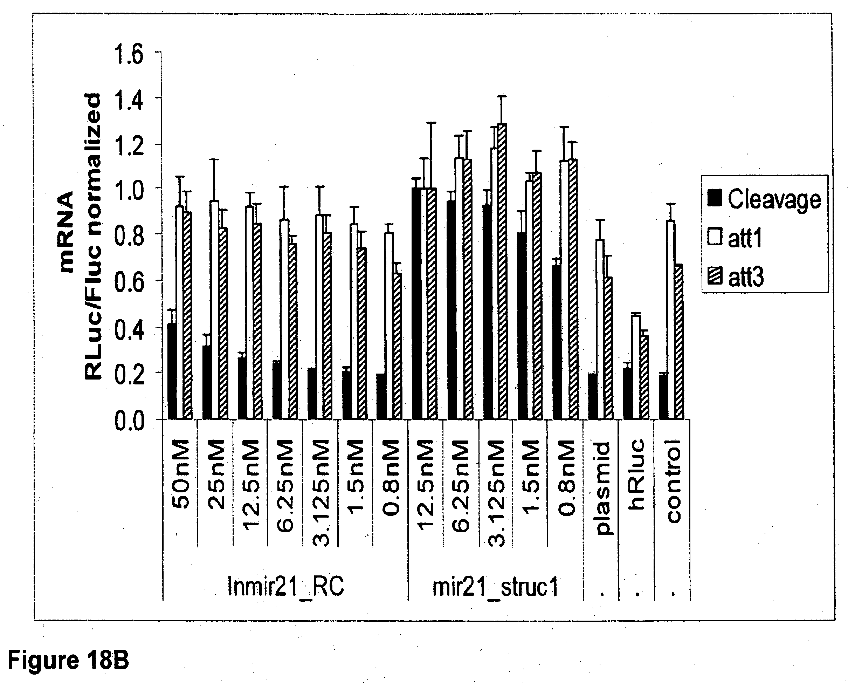

Figure 18A is a schematic representation of the reporter plasmid used in the experiments that generated the data shown in the graphs of Figures 18B-18C.
Figure 18B is a graph showing a comparison of the affects of short single stranded inhibitors and double stranded inhibitors on reporter constructs containing: 1) a single cleavage site, 2) a single attenuation site, or 3) three attenuation sites, as measured by branched DNA assays. "mir21-strucl" represents the double stranded inhibitor design tested in these studies. "Inmir21_RC" represents the short single stranded inhibitor design used in these studies. Results demonstrate the enhanced potency of double stranded inhibitors in both assays.

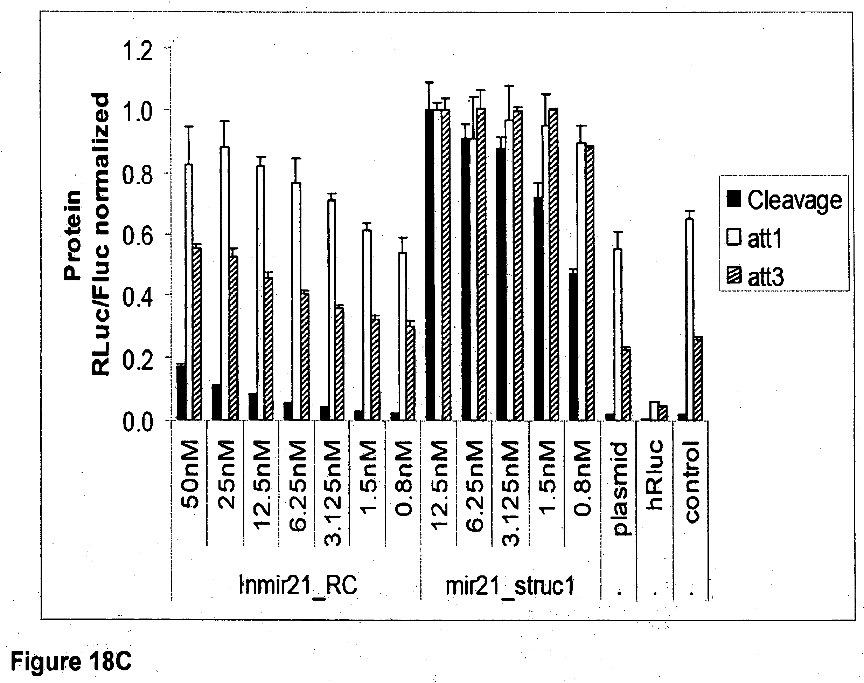

Figure 18C is a graph showing a comparison of the effects of short single stranded inhibitors and double stranded inhibitors on reporter constructs containing 1) a single cleavage site, 2) a single attenuation site, or 3) three attenuation sites, as measured by the dual luciferase assay. "mir21-struc1" represents the double stranded inhibitor design tested in these studies. "Inmir21_RC" represents the short single stranded inhibitor design used in these studies. Results demonstrate the enhanced potency of double stranded inhibitors in both assays.

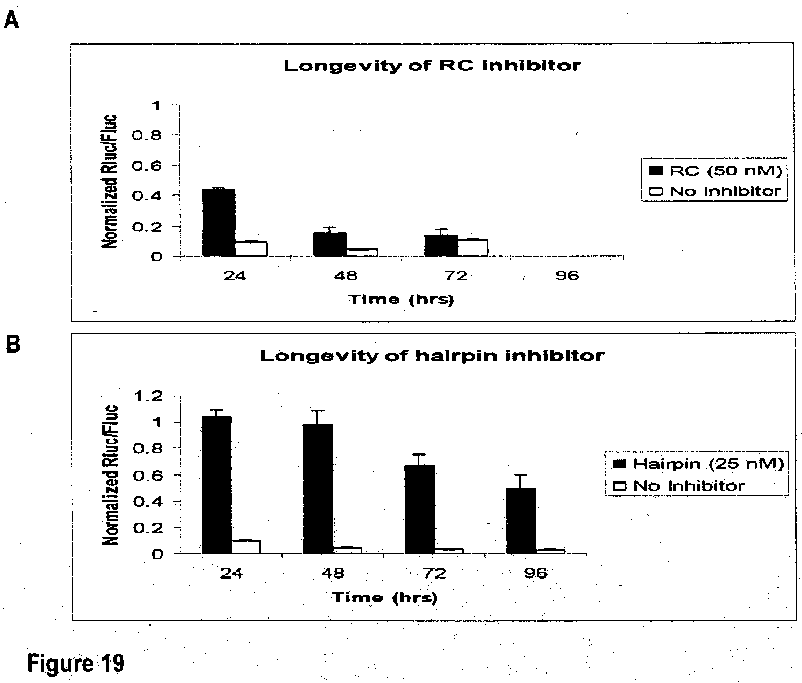

Figures 19A-19B are graphs showing a study of the longevity of silencing by comparing simple RC and double stranded inhibitor designs. Study examines the relative longevity of silencing by short, single-stranded 21 nucleotide 2'-O-Me modified inhibitors (Figure 19A), and double stranded inhibitors of the invention having hairpins in the flanking regions targeting let-7 (Figure 19B). Results show the enhance longevity of inhibition by double stranded inhibitors.

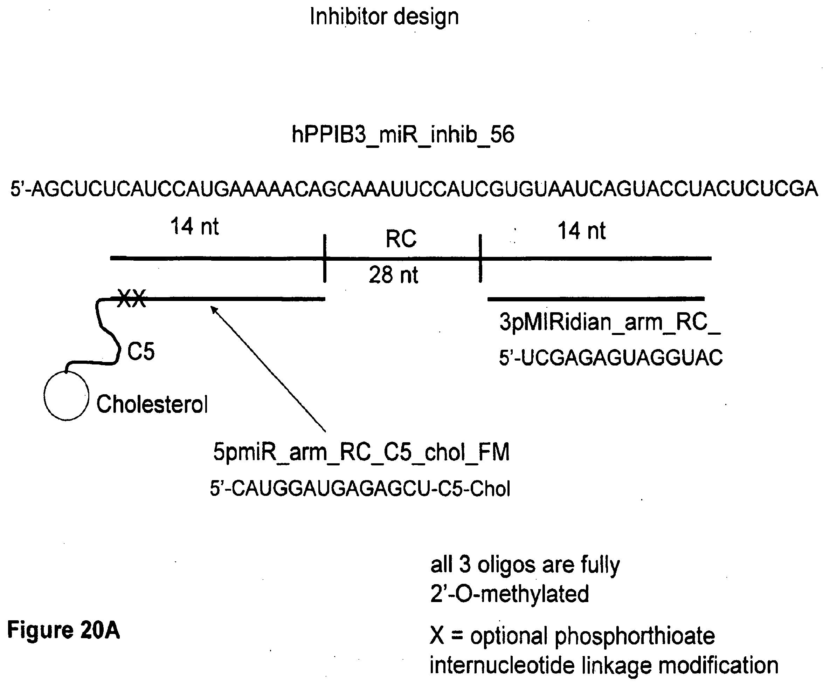

Figure 20A is a schematic representation of a design of the cholesterol modified double stranded inhibitor used in Example 16. The long oligonucleotide described by "hPPIB3_miR_inhib_56" has a polynucleotide sequence of 5'-AGCUCUCAUCCAUGAAAAACAGCAAAUUCCAUCGUGUAAUCAGUACCU ACUCUCGA (SEQ ID NO: 8). The short oligonucleotide described by "5pmiR_arm_RC_C5_chol_FM" has a polynucleotide sequence of 5'-CAUGGAUGAGAGCU (SEQ ID NO: 9). The short oligonucleotide described by "3pMIRidian_arm_RC_" has a polynucleotide sequence of 5'-UCGAGAGUAGGUAC (SEQ ID NO: 10).

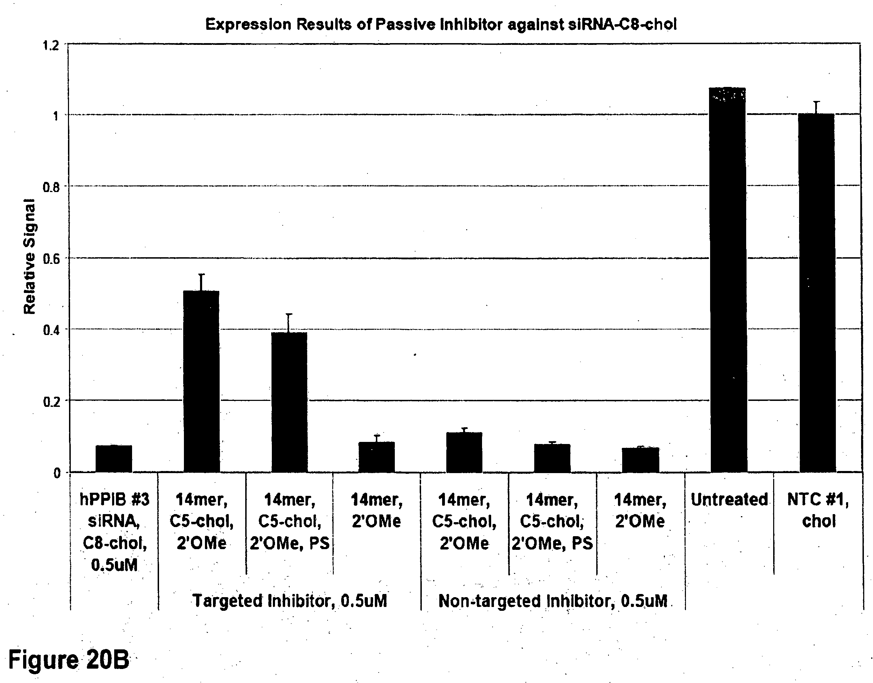

Figure 20B is a graph that demonstrates the utility of multiple cholesterol modified double stranded inhibitor designs. In particular, these experiments demonstrate that in the absence of the inhibitor molecule, the siRNA knocks down its respective target by greater than 90% (see lane 1). Addition of the cholesterol conjugated inhibitor molecule (with or without the optional phosphorothioate modification) severely limits the ability of the siRNA to act (see lanes 2 and 3). Similar experiments with control, non-targeting inhibitors, or targeting inhibitors that are un-conjugated to cholesterol, fail to prevent the siRNA from knocking down its target.

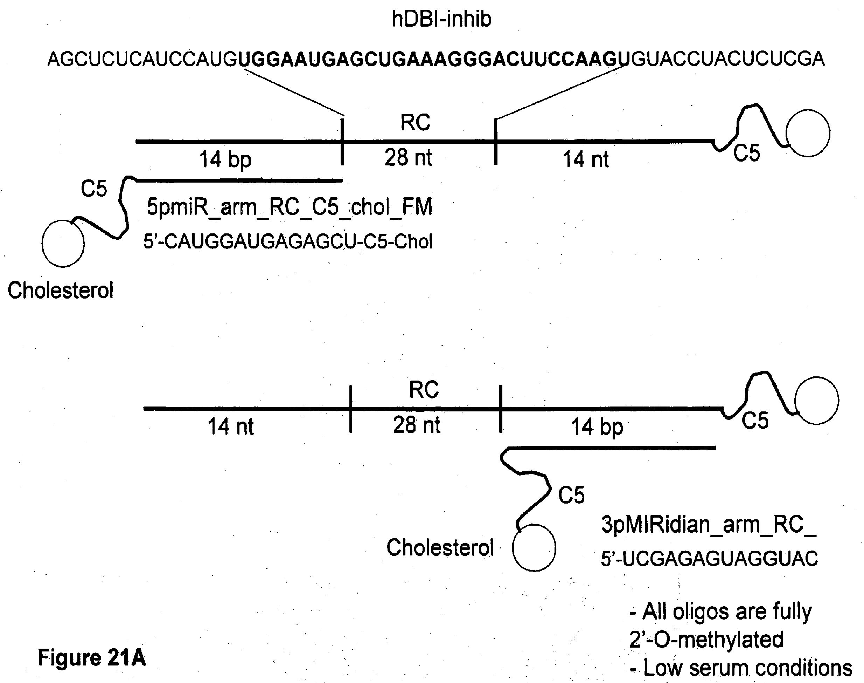

Figure 21A is a schematic representation of double stranded inhibitor designs that were tested for compatibility with passive delivery. The long oligonucleotide described by "hDBI-inhib" has a polynucleotide sequence of 5'-AGCUCUCAUCCAUGUGGAAUGAGCUGAAAGGGACUUCCAAGUGUACCU ACUCUCGA (SEQ ID NO: 11). The short oligonucleotide described by "5pmiR_arm_RC_C5_chol_FM" has a polynucleotide sequence of 5'-CAUGGAUGAGAGCU (SEQ ID NO: 9). The short oligonucleotide described by "3pMIRidian_arm_RC_" has a polynucleotide sequence of 5'-UCGAGAGUAGGUAC (SEQ ID NO: 10).

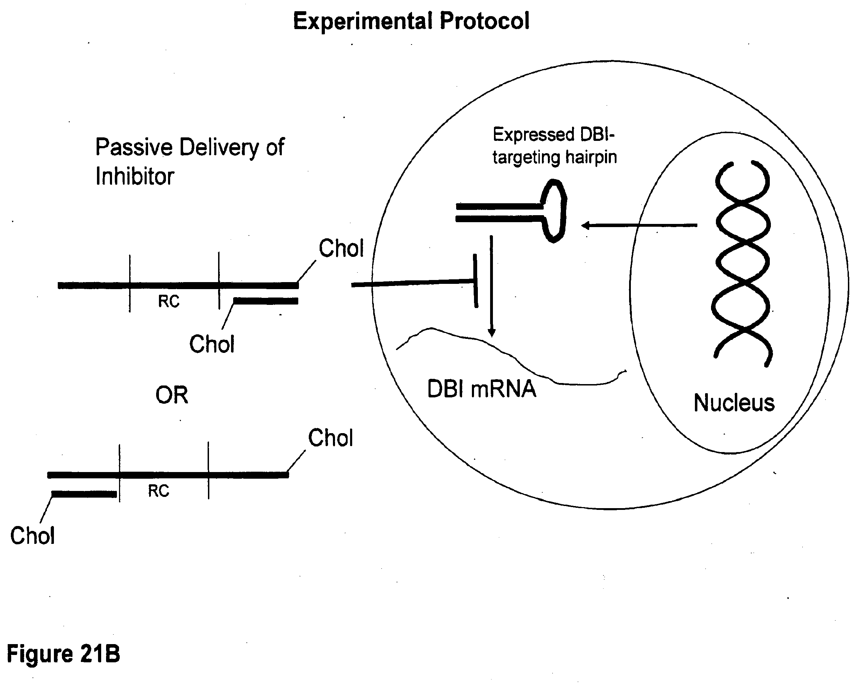

Figure 21B is a schematic representation of a protocol that was used to test passive delivery of cholesterol modified double stranded inhibitors.

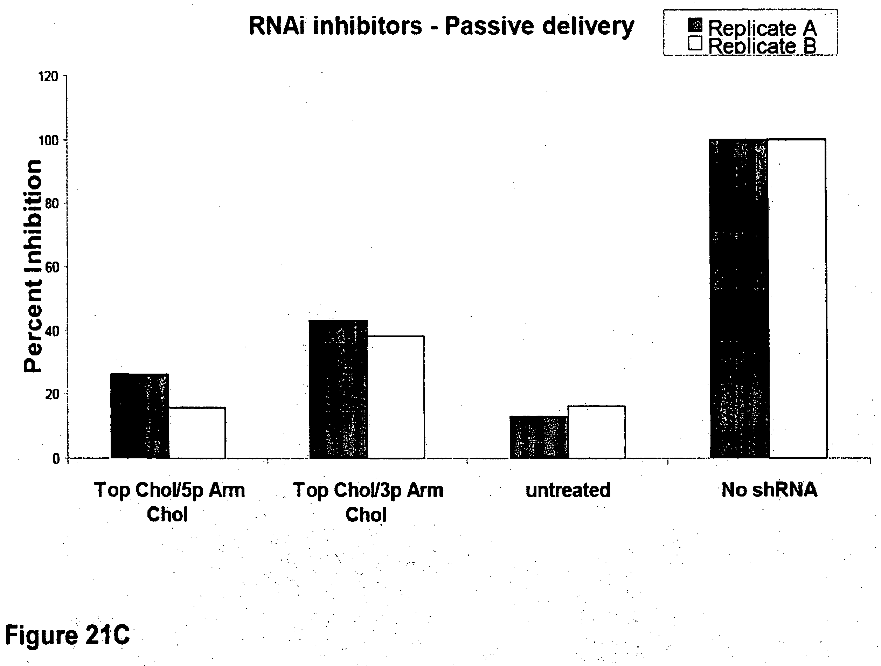

Figure 21C is a graph illustrating the results from passive delivery of two double stranded inhibitors. Findings demonstrate that inhibitors comprised of a cholesterol, modified first oligonucleotide, and a cholesterol modified 3^{rd} oligonucleotide provide strong inhibition of the hairpin design targeting DBI.

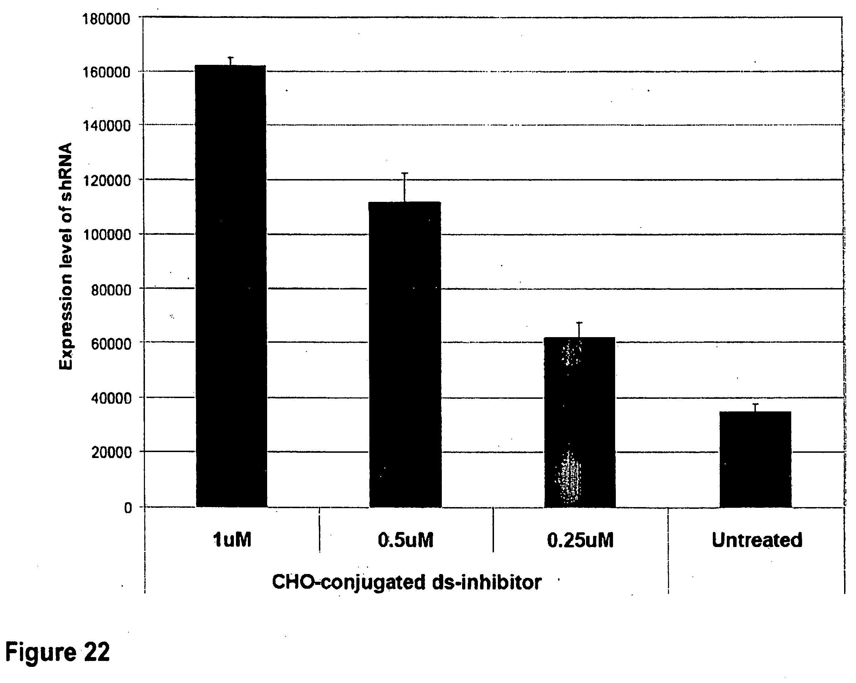

Figure 22 is a graph illustrating the results of short double stranded inhibitor designs having cholesterol conjugates that were tested for the ability to inhibit RNAi. Findings show that inhibitors built around this design provide strong inhibition of shRNAs targeting the DBI gene by passive delivery.

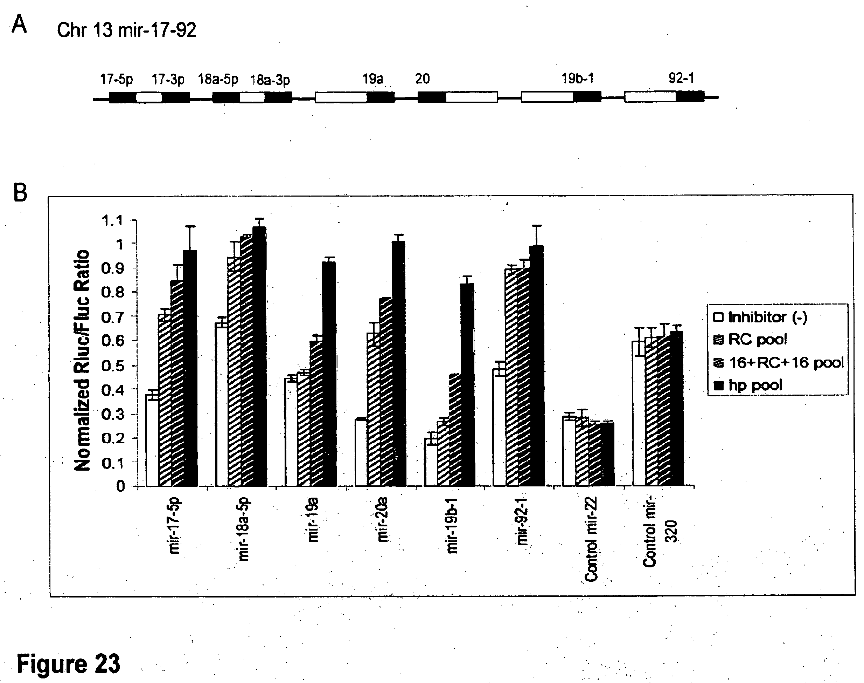

Figure 23A is a schematic representation of a cluster of microRNA that is coexpressed on chromosome 13 (miR~17~18a~19a~20~19b~92).
Figure 23B is a bar graph illustrating results of double stranded inhibitors that were tested for the ability to simultaneously target six distinct miRNAs. The results of these experiments are shown in Figure 23B and demonstrate that only the double stranded inhibitors are potent enough to simultaneously target all of the molecules.

Table 1 represents a list of preferred inhibitor sequences targeting miRNAs from the human, mouse, and rat genomes. The sequences consist of the central or reverse complement (RC) region and can be associated with either the single stranded or double stranded designs. For long single stranded inhibitors, the full inhibitor sequences contain the central region, as well as common 5' flanking (5') and 3' flanking (3') regions. In this case, all of the nucleotides in the inhibitor central and flanking sequences are O-methylated at the 2' carbon of the ribose ring. Table 1 also provides the accession number of the mature and precursor miR to which each inhibitor targets.
Table 2 provides the list of sequences that were tested to determine the optimal length of the inhibitors.
Table 3 provides the list of sequences that were tested to determine the optimal position of flanking sequences.
Table 4 provides a list of sequences that were used to test the importance of flanking sequence content.
Table 5 provides the list of sequences that were used in these studies to test the efficacy of different double stranded inhibitor designs.

### DETAILED DESCRIPTION OF THE INVENTION

The present invention will now be described in connection with preferred embodiments. These embodiments are presented to aid in an understanding of the present invention and are not intended, and should not be construed, to limit the invention in any way. All alternatives, modifications and equivalents that may become apparent to those of ordinary skill upon reading this disclosure are included within the spirit and scope of the present invention. This disclosure is not a primer on compositions and methods for performing RNA interference. Basic concepts known to those skilled in the art have previously been set forth in detail.

The present invention is directed to compositions and methods for inhibiting RNA interference, including siRNA, piRNA, and miRNA-induced gene silencing. Through the use of the present invention, modified polynucleotides, and derivatives thereof, one may improve the efficacy of RNA interference applications.

Unless stated otherwise, the following terms and phrases have the meanings provided below:

As used herein, "alkyl" refers to a hydrocarbyl moiety that can be saturated or unsaturated, and substituted or unsubstituted. It may comprise moieties that are linear, branched, cyclic and/or heterocyclic, and contain functional groups such as ethers, ketones, aldehydes, carboxylates, *etc.* Unless otherwise specified, alkyl groups are not cyclic, heterocyclic, or comprise functional groups. Exemplary alkyl groups include but are not limited to substituted and unsubstituted groups of methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl and alkyl groups of higher number of carbons, as well as 2-methylpropyl, 2-ethyl-4-ethylbutyl, 2,4-diethylpropyl, 3-propylbutyl, 2,8-dibutyldecyl, 6,6-dimethyloctyl, 6-propyl-6-butyloctyl, 2-methylbutyl, 2-methylpentyl, 3-methylpentyl, and 2-ethylhexyl. The term alkyl also encompasses alkenyl groups, such as vinyl, allyl, aralkyl and alkynyl groups. Unless otherwise specified, alkyl groups are not substituted.

Substitutions within alkyl groups, when specified as present, can include any atom or group that can be tolerated in the alkyl moiety, including but not limited to halogens, sulfurs, thiols, thioethers, thioesters, amines (primary, secondary, or tertiary), amides, ethers, esters, alcohols and oxygen. The alkyl groups can by way of example also comprise modifications such as azo groups, keto groups, aldehyde groups, carboxyl groups, nitro, nitroso or nitrile groups, heterocycles such as imidazole, hydrazino or hydroxylamino groups, isocyanate or cyanate groups, and sulfur containing groups such as sulfoxide, sulfone, sulfide, and disulfide. Unless otherwise specified, alkyl groups do not comprise halogens, sulfurs, thiols, thioethers, thioesters, amines, amides, ethers, esters, alcohols, oxygen, or the modifications listed above.

Further, alkyl groups may also contain hetero substitutions, which are substitutions of carbon atoms, by for example, nitrogen, oxygen or sulfur. Heterocyclic substitutions refer to alkyl rings having one or more heteroatoms. Examples of heterocyclic moieties include but are not limited to morpholino, imidazole, and pyrrolidino. Unless otherwise specified, alkyl groups do not contain hetero substitutions or alkyl rings with one or more heteroatoms (*i.e.,* heterocyclic substitutions). The preferred alkyl group for a 2' modification is a methyl group with an O-linkage to the 2' carbon of a ribosyl moiety (*i.e.,* a 2'-O-alkyl that comprises a 2'-O-methyl group).

As used herein, "2'-O-alkyl modified nucleotide" refers to a nucleotide unit having a sugar moiety, for example a deoxyribosyl moiety that is modified at the 2' position such that an oxygen atom is attached both to the carbon atom located at the 2' position of the sugar and to an alkyl group. In various embodiments, the alkyl moiety consists essentially of carbons and hydrogens. A particularly preferred embodiment is one wherein the alkyl moiety is methyl moiety.

As used herein, "antisense strand" as used herein, refers to a polynucleotide or region of a polynucleotide that is substantially (*e.g*., 80% or more) or completely (100%) complementary to a target nucleic acid of interest. An antisense strand may be comprised of a polynucleotide region that is RNA, DNA or chimeric RNA/DNA. For example, an antisense strand may be complementary, in whole or in part, to a molecule of messenger RNA, an RNA sequence that is not mRNA (*e.g*., tRNA, rRNA and hnRNA) or a sequence of DNA that is either coding, non-coding, transcribed, or untranscribed. The phrase "antisense strand" includes the antisense region of the polynucleotides that are formed from two separate strands, as well as unimolecular siRNAs that are capable of forming hairpin structures. The phrases "antisense strand" and "antisense region" are intended to be equivalent and are used interchangeably. The antisense strand can be modified with a diverse group of small molecules and/or conjugates.

As used herein, "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (*e.g*., A to T, A to U, C to G). Perfect complementarity or 100% complementarity refers to the situation in which each nucleotide unit of one polynucleotide strand or region can hydrogen bond with each nucleotide unit of a second polynucleotide strand or region. Less than perfect complementarity refers to the situation in which some, but not all, nucleotide units of two strands or two regions can hydrogen bond with each other. Substantial complementarity refers to polynucleotide strands or regions exhibiting 80% or greater complementarity.

As used herein, "central region" refers to the area or region of an inhibitor molecule of the invention that is the reverse complement (RC) of mature miRNA, a piRNA, or an siRNA. When prescribed, the central region can also refer to the area or region of an inhibitor molecule of the invention that is the reverse complement of a mature miRNA and regions that border the mature miRNA in e.g. the primary miRNA. Preferably, the central region of inhibitors are substantially complementary to the mature miRNA or mature miRNA and regions that border the mature miRNA in the primary miRNA, or piRNA. More preferably, the central region of inhibitors are 100% complementary to the mature miRNA or mature miRNA and regions that border the mature miRNA in the primary miRNA.

As used herein, "duplex" and "duplex region" are interchangeable and refer to structures that are formed when two regions of one or more oligonucleotides, modified oligonucleotides, or modified and conjugated oligonucleotides anneal together.

As used herein, "enhancer sequence" and "enhancer oligonucleotide" are interchangeable and refer to oligonucleotides that can anneal to the 5' and/or 3' flanking regions of the first oligonucleotide.

As used herein, "flanking region" refers to one or more regions of the first oligonucleotide of the invention that borders the central region which is the reverse complement to a mature miRNA, a mature miRNA and regions that border the mature miRNA in the primary miRNA, or the RISC entering strand of a piRNA.

As used herein, "hairpin" refers a stem-loop structure. The stem results from two sequences of nucleic acid or modified nucleic acid annealing together to generate a duplex. The loop is a single stranded region that lies between the two strands comprising the stem.

As used herein, "mature strand" refers to the strand of a fully processed miRNA, a piRNA, or an siRNA that enters RISC. In some cases, miRNAs have a single mature strand that can vary in length between about 17-28 nucleotides in length. In other instances, miRNAs can have two mature strands, and again, the length of the strands can vary between about 17 and 28 nucleotides.

As used herein, "microRNA", "miRNA", and "MiR" are interchangeable and refer to endogenous or synthetic non-coding RNAs that are capable of entering the RNAi pathway and regulating gene expression. "Primary miRNAs" or "pri-miRNA" represent the non-coding transcript prior to Drosha processing and include the hairpin(s) structure as well as 5' and 3' sequences. "Pre-miRNA" represent the noun-coding transcript after Drosha processing of the pri-miRNA. The term "mature miRNA" can refer to the double stranded product resulting from Dicer processing of pre-miRNA or the single stranded product that is introduced into RISC following Dicer processing. In some cases, only a single strand of an pre-miRNA enters the RNAi pathway. In other cases, each strand of a pre-miRNA are capable of entering the RNAi pathway. In addition, piRNAs are a recently discovered small ribonucleotides that also play a role in regulating genes. The inhibitor designed described in this application are expected to work equally well to inhibit the function of these molecules.

As used herein, "microRNA inhibitor", "miR inhibitor", and "inhibitor" are interchangeable and refer to polynucleotides or modified polynucleotides that interfere with the ability of specific miRNAs, piRNAs, or siRNAs to silence their intended targets. The mechanism(s) of action of an inhibitor are not limited and may include acting as an artificial substrate, inhibition of RISC action, inhibition of Drosha action, and/or inhibition of one or more additional steps associated with the RNAi pathway.

As used herein, "micro RNA reporter", "miR reporter", and "reporter" are interchangeable and refer to a vector or plasmid construct that encodes one or more reporter genes including but not limited to firefly luciferase, Renilla luciferase, secreted alkaline phosphatase, green fluorescent protein, yellow fluorescent protein, or others, and has miRNA target sites (also referred to as "miRNA recognition elements (MREs), piRNA recognition sites (PREs), or siRNA recognition elements (SREs) inserted into the 5' UTR, ORF, and/or 3'UTR of one or more of the reporter genes.

As used herein, "nucleotide" refers to a ribonucleotide or a deoxyribonucleotide or modified form thereof, as well as an analog thereof. Nucleotides include species that comprise purines, e.g., adenine, hypoxanthine, guanine, and their derivatives and analogs, as well as pyrimidines, e.g., cytosine, uracil, thymine, and their derivatives and analogs. Preferably, a "nucleotide" comprises a cytosine, uracil, thymine, adenine, or guanine moiety.

Nucleotide analogs include nucleotides having modifications in the chemical structure of the base, sugar and/or phosphate, including, but not limited to, 5-position pyrimidine modifications, 8-position purine modifications, modifications at cytosine exocyclic amines, and substitution of 5-bromo-uracil; and 2'-position sugar modifications, including but not limited to, sugar-modified ribonucleotides in which the 2'-OH or -H is replaced by a group such as an OR, R, halo, SH, SR, NH₂, NHR, NR₂, or CN, wherein R is an alkyl moiety as defined herein. Nucleotide analogs are also meant to include nucleotides with bases such as inosine, queuosine, xanthine, sugars such as 2'-methyl ribose, non-natural phosphodiester linkages such as methylphosphonates, phosphorothioates and peptides.

Modified bases refer to nucleotide bases such as, for example, adenine, guanine, cytosine, thymine, and uracil, xanthine, inosine, and queuosine that have been modified by the replacement or addition of one or more atoms or groups. Some examples of types of modifications that can comprise nucleotides that are modified with respect to the base moieties, include but are not limited to, alkylated, halogenated, thiolated, aminated, amidated, or acetylated bases, in various combinations. More specific modified bases include, for example, 5-propynyluridine, 5-propynylcytidine, 6-methyladenine, 6-methylguanine, N,N,-dimethyladenine, 2-propyladenine, 2-propylguanine, 2-aminoadenine, 1-methylinosine, 3-methyluridine, 5-methylcytidine, 5-methyluridine and other nucleotides having a modification at the 5 position, 5-(2-amino)propyl uridine, 5-halocytidine, 5-halouridine, 4-acetylcytidine, 1-methyladenosine, 2-methyladenosine, 3-methylcytidine, 6-methyluridine, 2-methylguanosine, 7-methylguanosine, 2,2-dimethylguanosine, 5-methylaminoethyluridine, 5-methyloxyuridine, deazanucleotides such as 7-deaza-adenosine, 6-azouridine, 6-azocytidine, 6-azothymidine, 5-methyl-2-thiouridine, other thio bases such as 2-thiouridine and 4-thiouridine and 2-thiocytidine, dihydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides. Modified nucleotides also include those nucleotides that are modified with respect to the sugar moiety, as well as nucleotides having sugars or analogs thereof that are not ribosyl. For example, the sugar moieties may be, or be based on, mannoses, arabinoses, glucopyranoses, galactopyranoses, 4'-thioribose, and other sugars, heterocycles, or carbocycles. The term nucleotide is also meant to include what are known in the art as universal bases. By way of example, universal bases include but are not limited to 3-nitropyrrole, 5-nitroindole, or nebularine. Further, the term nucleotide also includes those species that have a detectable label, such as for example a radioactive or fluorescent moiety, or mass label attached to the nucleotide.

As used herein, "polynucleotide" refers to polymers of nucleotides, and includes but is not limited to DNA, RNA, DNA/RNA hybrids and modifications of these kinds of polynucleotides wherein the attachment of various entities or moieties to the nucleotide units at any position are included. Unless otherwise specified, or clear from context, the term "polynucleotide" includes both unimolecular siRNAs, piRNAs, and miRNAs and siRNAs, miRNAs, and piRNAs comprised of two separate strands.

As used herein, "piRNAs" refers to Piwi-interacting RNAs, a class of small RNAs that are believed to be involved in transcriptional silencing (see Lau, N.C. et al (2006) Science, 313:305-306).

As used herein, the "reverse complement" of an oligonucleotide sequence is a sequence that will anneal/basepair or substantially anneal/basepair to a second oligonucleotide according to the rules defined by Watson-Crick base pairing and the antiparallel nature of the DNA-DNA, RNA-RNA, and RNA-DNA double helices. Thus, as an example, the reverse complement of the RNA sequence 5'-AAUUUGC would be 5'-GCAAAUU. Alternative base pairing schemes including but not limited to G-U pairings can also be included in reverse complements.

As used herein, "RISC" refers to the set of proteins that complex with single-stranded polynucleotides such as mature miRNA, piRNA, or siRNA, to target nucleic acid molecules (e.g. mRNA) for cleavage, translation attenuation, methylation, and/or other alterations. Known, non-limiting components of RISC include Dicer, R2D2 and the Argonaute family of proteins, as well as strands of siRNAs, piRNAs, and miRNAs.

As used herein, "RNA interference" and "RNAi" are interchangeable and refer to the process by which a polynucleotide (e.g., an miRNA, a piRNA, or siRNA) comprising at least one ribonucleotide unit exerts an effect on a biological process. The process includes, but is not limited to, gene silencing by degrading mRNA, attenuating translation, interactions with tRNA, rRNA, hnRNA, cDNA and genomic DNA, as well as methylation of DNA with ancillary proteins.

As used herein, "sense strand" refers to a polynucleotide or region that has the same nucleotide sequence, in whole or in part, as a target nucleic acid such as a messenger RNA or a sequence of DNA. The phrase "sense strand" includes the sense region of both polynucleotides that are formed from two separate strands, as well as unimolecular siRNAs that are capable of forming hairpin structures. When a sequence is provided, by convention, unless otherwise indicated, it is the sense strand (or region), and the presence of the complementary antisense strand (or region) is implicit. The phrases "sense strand" and "sense region" are intended to be equivalent and are used interchangeably.

As used herein, "siRNA" and "short interfering RNA" are interchangeable and refer to unimolecular nucleic acids and to nucleic acids comprised of two separate strands that are capable of performing RNAi and that have a duplex region that is between 18 and 30 base pairs in length. Additionally, the term siRNA and the phrase "short interfering RNA" include nucleic acids that also contain moieties other than ribonucleotide moieties, including, but not limited to, modified nucleotides, modified internucleotide linkages, non-nucleotides, deoxynucleotides and analogs of the aforementioned nucleotides. siRNAs can be duplexes, and can also comprise short hairpin RNAs, RNAs with loops as long as, for example, 4 to 23 or more nucleotides, RNAs with stem loop bulges, micro-RNAs, and short temporal RNAs. RNAs having loops or hairpin loops can include structures where the loops are connected to the variety of chemical structures, as long as they are of sufficient length and materials to enable effective intramolecular hybridization of the stem elements. Typically, the length to be spanned is at least about 10-24 atoms. When the siRNAs are hairpins, the sense strand and antisense strand are part of one longer molecule.

As used herein, "target" refers to a range of molecules including but not limited to an miRNA, an siRNA, a piRNA, an mRNA, rRNA, tRNA, hnRNA, cDNA and genomic DNA

### I. Single Stranded RNAi Inhibitors

Also described herein are compositions comprising single stranded RNAi inhibitors. As such, the RNAi inhibitor can be a modified or unmodified oligonucleotide that contains three domains (*e.g*., a 5' flanking domain, a central domain, and a 3' flanking domain) and ranges in length between about 41 and about 68 nucleotides. The three domains can be as described below.
1. The central region ranges in length between about 17 and about 32 nucleotides and is substantially similar to the reverse complement of the mature, RISC-entering strand of the miRNA and regions bordering the mature strand. More preferably, the central region ranges from about 22 to about 28 nucleotides. Most preferably, the central region ranges from about 25 to about 28 nucleotides.
2. The 5' flanking region is: (1) is about 12 to about 20 nucleotides in length; (2) is not rich in pyrimidines (*e.g*., preferably not more than about 70%, more preferably not more than about 60%, even more preferably not more than about 50%, still more preferably not more than about 40%, and most preferably less than about 30% pyrimidines); (3) is 5' of the central region; and (4) has minimal complementarity with the primary miRNA sequence that is 3' of the mature miRNA sequence. In one instance, the 5' flanking region does not interact with the miRNA.
3. The 3' flanking region is: (1) is about 12 to about 20 nucleotides in length; (2) is not rich in pyrimidines (*e.g*., preferably not more than about 70%, more preferably not more than about 60%, even more preferably not more than about 50%, still more preferably not more than about 40%, and most preferably less than about 30% pyrimidines); (3) is 3' of the central region; and (4) has minimal complementarity with the primary miRNA sequence that is 5' of the mature miRNA sequence. In one instance, the 3' flanking region does not interact with the miRNA. Furthermore, the preferred modifications of the oligonucleotide are: (a) 2' O-alkyl modification, and/or (b) 2'-ACE (*i.e.,* 2'-O- acetoxyethoxy) modifications of the ribose ring of all nucleotides.

### 1. The Central Region of Single Stranded Inhibitors

The sequences of the central region of miRNA inhibitors are the reverse complement of mature miRNA. Alternatively, the sequence of the central region of an miRNA inhibitor comprise those that are the reverse complement of the mature miRNA plus about 1 to about 5 additional nucleotides associated with the 5' and 3' ends of the sequence that are the reverse complement to the mature miRNA and are complementary to the 3' and 5' regions that border the mature miRNA sequence. Preferably, the central region of an miRNA inhibitor has substantial (*e.g*., about 80% or greater) complementarity to the mature strand and/or the mature strand and regions that border the mature strand in the pri-miRNA. More preferably, the central region of an miRNA inhibitor has greater than about 90% complementarity to the mature strand and/or the mature strand and regions that border the mature strand in the pri-miRNA. Most preferably, the central region of an miRNA inhibitor has about 100% complementarity to the mature strand and/or the mature strand and regions that border the mature strand in the pri-miRNA. Addition of sequences that are complementary to regions of the primary miRNA that border the mature sequence can disrupt Drosha processing, and further enhance the inhibitory properties of the molecules. Moreover, the authors recognize that cloning is an imperfect science and that on infrequently occasions; the true length and sequence of the mature miRNA is miscalculated and extends beyond the boundaries that are reported in one or more databases. Extending the central region to include sequences that anneal to regions that border the mature miRNA sequence compensates for possible errors in the mapping of the true boundaries of the mature miRNA (Figure 3).

Preferably, the length of the central region varies between about 17 to about 32 nucleotides and the region that is complementary to the mature miRNA sequence is centered or nearly centered in the middle of the central region sequence. More preferably, the length of the central region is between about 22 and about 28 nucleotides and the region that is complementary to the mature miRNA sequence is centered or nearly centered in the middle of the central region sequence. Even more preferably, the length of the central region is between about 25 and about 28 nucleotides and the region that is complementary to the mature miRNA sequence is centered or nearly centered in the middle of the central region sequence. Even more preferably, the length of the central region is between about 25 and about 28 nucleotides and the region that is complementary to the mature miRNA sequence is centered or nearly centered in the middle of the central region sequence. Most preferably, the length of the central region is about 28 nucleotides in length and the region that is complementary to the mature miRNA sequence being centered or nearly centered in the middle of the central region sequence.

A list of preferred central region nucleotide sequences of miRNA inhibitor molecules for human, mouse, and rat miRNA is presented in Table 1. In cases where two mature strands evolve from a single miRNA, two inhibitor molecules with different central region sequences can be designed and synthesized. The list of sequences provided in Table 1 is not intended to be limiting in any fashion and can include variants. In most cases, the region of the central sequence that is the reverse complement of the mature miRNA is centered between sequences that are complementary to regions that border the mature miRNA in the primary miRNA. While this is the preferred organization of the central region, the inventors envision cases where the region of the central sequence that is the reverse complement of the mature miRNA is not evenly centered between sequences that are complementary to regions that border the mature miRNA in the primary miRNA. Thus, for instance, in the case where the mature miR sequence is about 21 nucleotides in length, then four and three nucleotides could be added to the 5' and 3' ends respectively. Lastly, the list of sequences presented in Table I are not intended to be limiting as it is predicted to increase as the number of miRNA sequences in all species expands.

### 2. The 5' Flanking Region of Single Stranded Inhibitors

As mentioned above, the 5' flanking region has minimal complementarity to sequences bordering the 3' end of known mature miRNAs. Furthermore, the 5' flanking region is not rich in pyrimidines (*e.g*., preferably not more than about 70%, more preferably not more than about 60%, even more preferably not more than about 50%, still more preferably not more than about 40%, and most preferably less than about 30% pyrimidines), and/or mimics the composition of native mRNA. Preferably, the 5' flanking region is about 12 to about 20 nucleotides in length. More preferably, the 5' flanking region is about 12 to about 18 nucleotides in length. Even more preferably, the 5' flanking region is about 12 to about 16 nucleotides in length. Most preferably, the 5' flanking region is about 14 nucleotides in length. Preferably, the nucleotide content of the 5' flanking region is designed to match the overall content of mRNA coding sequences (*i.e*., about 25% G, C, A, and U), is designed to mimic a true mRNA substrate, and is sufficiently long that it, in conjunction with the central and 3' flanking regions, can be recognized by RISC and other proteins associated with the RNAi machinery. Most preferably, the sequence of the 5' flanking region is 5'-AGCUCUCAUCCAUG (SEQ ID NO: 4).

### 3. The 3' Flanking Region of Single Stranded Inhibitors

The 3' flanking region has minimal complementarity to sequences bordering the 5' end of known mature miRNAs, and is not rich in pyrimidines (e.g., preferably not more than about 70%, more preferably not more than about 60%, even more preferably not more than about 50%, still more preferably not more than about 40%, and most preferably less than about 30% pyrimidines). Preferably, the 3' flanking region is about 12 to about 20 nucleotides in length. More preferably, the 3' flanking region is about 12 to about 18 nucleotides in length. Even more preferably, the 3' flanking region is about 12 to about 16 nucleotides in length. Most preferably, the 3' flanking region is about 14 nucleotides in length. Preferably, the nucleotide content of the 3' flanking region is designed to match the overall content of mRNA coding sequences *(i.e.,* about 25% G, C, A, and U), is designed to mimic a true mRNA substrate, and is sufficiently long that it, in conjunction with the central and 5' flanking regions, can be recognized by RISC and other proteins associated with the RNAi machinery. Most preferably, the sequence of the 3' flanking region is 5'-GUACCUACUCUCGA (SEQ ID NO: 5).

One example of inhibitors containing the preferred 5' and 3' flanking regions is provided as follows: 5'-agcucucauccaugUAAAACUAUACAACCUACUACCUCAUCCguaccuacucucga (SEQ ID NO: 12), where the capital letters represent the central region for miR accession no. MIMAT0000062 (e.g, precursor accession no MI0000060) and the preferred sequence of 5' flanking region-central region-3' flanking region are in lower case letters.

### II. Double Stranded RNAi Inhibitors

In one embodiment, the present invention includes RNAi inhibitors that include at least one duplex region. As such, the RNAi inhibitors can include a composition comprising one or more of the following:
1. A first oligonucleotide comprising:
   a. A central region ranging in length between about 6 and about 37 nucleotides that is substantially similar to the reverse complement of a mature (*i.e.,* RISC-entering) strand of the miRNA or piRNA, or the mature strand plus regions bordering the mature strand of a pri-miRNA, or the RISC entering strand of an siRNA.
   b. A 5' flanking region that is about 10 to about 40 nucleotides in length, is 5' of the central region, and is capable of: (1) annealing to itself to create a duplex region, (2) annealing to the 3' flanking region to create a duplex region, (3) annealing to a second oligonucleotide *(i.e.,* first enhancer sequence or 5' enhancer) to create a double stranded region, or (4) has little or no secondary structure.
   c. A 3' flanking region that is about 10 to about 40 nucleotides in length, is 3' of the central region, and is capable of: (1) annealing to itself to create a hairpin structure, (2) annealing to the 5' flanking region, (3) annealing to a third oligonucleotide *(i.e.,* second enhancer sequence or 3' enhancer), or (4) has little or no secondary structure at all.
2. A second oligonucleotide (*i.e*., first enhancer sequence or 5' enhancer oligonucleotide) that is substantially complementary to, and capable of annealing with all or portions of the 5' flanking region.
3. A third oligonucleotide *(i.e.,* second enhancer sequence or 3' enhancer oligonucleotide) that is substantially complementary to and capable of annealing with all or portions of the 3' flanking region.

Referring to Figure 4A, examples of the RNAi inhibitors of the invention having double-stranded region(s) include the following designs: (A) an inhibitor molecule that has a central region and hairpin structures in both the 5' and 3' flanking regions, (B) an inhibitor molecule with a central region and a hairpin structure in the 5' flanking regions and an unstructured *(i.e.,* not duplexed) 3' flanking region, (C) an inhibitor molecule with a central region and a hairpin structure in the 3' flanking regions and an unstructured 5' flanking region, (D) an inhibitor molecule that has a central region and forms a double stranded region by having the 5' and 3' flanking regions anneal together, (E) an inhibitor molecule that has a central region and a hairpin structure in the 3' flanking region, but no 5' flanking region, (F) an inhibitor molecule that has a central region and a double stranded region in the 3' flanking region resulting from annealing to a complementary sequence, but no 5' flanking region, (G) an inhibitor molecule that has a central region, a hairpin structure in the 5' flanking region, but no 3' flanking region, (H) an inhibitor molecule that has a central region and double stranded regions in the 5' and 3' flanking regions resulting from addition of second and third oligonucleotides that anneal to the flanks, (I) an inhibitor molecule that has a central region, a 5' flanking region, and a double stranded 3' flanking region resulting from addition of a complementary oligonucleotide, (J) an inhibitor molecule that has a central region, a 3' flanking region, and a double stranded 5' flanking region resulting from addition of a complementary oligonucleotide, (K) an inhibitor with a central region, a double stranded 5' flanking region resulting from addition of a complementary oligonucleotide, and a 3' flanking region that contains a hairpin, (L) an inhibitor with a central region, a double stranded 3' flanking region resulting from addition of a complementary oligonucleotide, and a 5' flanking region that contains a hairpin, (M) an inhibitor molecule that contains a central region and a double stranded 5' flanking region resulting from addition of a complementary oligonucleotide, but no 3' flanking region. These and additional double stranded inhibitor designs are envisioned by the inventors and are diagramed in Figure 4A.

Preferably, one or more of the oligonucleotides of the invention are modified. The preferred modification is an O-alkyl modification of some or all of the 2' carbons of the ribose ring of some or all of the oligonucleotides. In addition, preferably one or more of the oligonucleotides or modified oligonucleotides of the invention are conjugated to a hydrophobic molecule (e.g. cholesterol).

The RNAi inhibitors of the invention exhibit multiple improvements over those previously known including: (1) longevity of inhibition, and (2) potency of silencing. In addition, while previous inhibitor designs exhibit some functionality when targeting miRNAs that are poorly expressed, these older designs fail to efficiently target highly expressed targets. The newer designs described below efficiently inhibit targets that are expressed at both high and low concentrations.

### 1. The Central Region of Double Stranded Inhibitors

The sequences of the central region of inhibitors are single stranded and the reverse complement of some or all of the mature miRNA, a piRNA, the RISC entering strand of siRNA, or any other regulating RNA that utilizes the RNAi pathway. Alternatively, the sequence of the central region of the invention comprise sequences that are the reverse complement of the mature miRNA plus about 1 to about 10 additional nucleotides (*e.g*., associated with the 5' and/or 3' end(s) of said sequence) that are complementary to the 3' and 5' regions, respectively, that border the mature miRNA sequence in the pri-miRNA. The motivation behind adding sequences other than those that are the reverse complement of the reported mature miRNA stems from an understanding of cloning. The inventors recognize that cloning is an imperfect science and that on occasion, the true length and sequence of the mature miRNA is misjudged and extends beyond the boundaries that are reported in one or more databases. For this reason, expanding the central region to include sequences that anneal to regions that border the mature miRNA sequence in the pri-miRNA compensates for possible errors in the mapping of the true boundaries of the mature miRNA. Furthermore, addition of sequences on the 5' and 3' end of the central region extends the overall length of the inhibitor molecules. As described in this document and in priority document USSN 60/774,350 the performance of longer inhibitors is superior to those of smaller (e.g. 21 nucleotide) inhibitors having the same chemical modifications (*e.g.*, 2'-O-methyl).)

For these reasons, the preferred length of the central region varies between about 6 to about 37 nucleotides. More preferably, the length of the central region is between about 22 to about 32 nucleotides. Even more preferably, the length of the central region is between about 26 to about 32 nucleotides. Most preferably, the length of the central region is between about 28 to about 32 nucleotides. Though it is not required, preferably in all of the instances, the region that is complementary to the mature miRNA sequence is centered or nearly centered in the middle of the central region sequence.

Preferably, the central region of an inhibitor of the invention has substantial *(e.g.,* 80% or greater) complementarity to the mature strand of an miRNA, a piRNA, the mature strand of an miRNA or piRNA and regions that border the mature strand in the pri-miRNA or pri-piRNA, or the RISC-entering strand of an siRNA. More preferably the central region of an RNAi inhibitor of the invention has greater than about 90% complementarity to the mature strand of an miRNA, or piRNA, the mature strand of an miRNA and regions that border the mature strand in the pri-miRNA or pri-piRNA, or the RISC-entering strand of an siRNA. Most preferably the central region of an inhibitor of the invention has about 100% complementarity to the mature strand of an miRNA, the mature strand miRNA and regions that border the mature strand in the pri-miRNA, or the RISC-entering strand of an siRNA.

Additionally, the central region nucleotide sequences of double stranded RNAi inhibitor molecules targeting human, mouse, and rat miRNA are presented in Table 1, and can be the same as sequences for single stranded RNAi inhibitor molecules.

### 2. The 5' Flanking Region

As mentioned above, the 5' flanking region is between about 10 to about 40 nts in length, is 5' of the central region, and is capable of: (1) annealing to itself to create a duplex region, (2) annealing to the 3' flanking region to create a double stranded region, (3) annealing to a second oligonucleotide *(i.e.,* first enhancer sequence or 5' enhancer oligonucleotide) to create a double stranded region, or (4) has little or no secondary structure. The first three of these alternatives (*e.g*., annealing back upon itself, annealing to the 3' flanking region, and annealing to a first enhancer oligonucleotide) create a region of double stranded RNA which, as demonstrated in Examples 6-19, greatly enhances the functional properties of the inhibitor in an unexpected way.

The inventors have observed that inhibitors that have duplex structures resulting from sequences in the 5' flanking region annealing with other sequences present in the 5' flanking region perform better than short single stranded inhibitors (see Example 6). Preferably, in these cases a hairpin structure is formed (see Figure 4). Hairpins comprise a loop structure and a stem region (*i.e*., a duplex region) that results from base pairing of two separate regions (*e.g*., having sufficient levels of complementarity) separated by a non-base pairing region (*e.g.*, the loop). Preferably, the length of the duplex region is between about 4 and about 20 base pairs in length and the level of complementarity is greater than about 80%. More preferably, the length of the duplex is about 6 to about 15 base pairs and the level of complementarity is greater than about 80%. Most preferably, the length of the duplex is between about 6 and about 10 base pairs in length and the level of complementarity is about 100%. Similarly, the loop can also vary in size (e.g., ranging from about 4 to about 15 nucleotides in length) and sequence.

Lastly, though the distance between the 5' terminus of the duplex region and the 5'-most boundary of the central region can vary greatly, preferably the duplex region of the 5' flanking sequence is adjacent to the boundary of the central region. Without wishing to be restricted to a particular theory or model, the inventors believe that in cases where segments of single stranded nucleotides flank the central region, the possibility of secondary structures resulting from base pairing between the central region and sequences of the single stranded 5' flanking region can occur, thus disrupting the overall functionality of the inhibitor. By having the double stranded region immediately adjacent to the border of the central region, this risk is minimized.

The inventors have also discovered that inhibitors that comprise regions of double stranded RNA resulting from base pairing between sequences in the 5' and 3' flanking regions perform better than short, single stranded inhibitors (see Example 7 and Figure 4). In the case where the 5' flanking region is capable of annealing with sequences of the 3' flanking region, preferably the length of the region that is duplexed is between about 10 to about 40 base pairs and the overall level of complementarity is greater than about 80%. In addition, the number of nucleotides that separate the borders of the central region and the duplexed region that results from 5' and 3' regions annealing, can vary in length and composition. The terminus can be blunt or contain 5' or 3' overhangs of about 1 to about 6 nucleotides.

In addition, the inventors have discovered that inhibitors that comprise regions of double stranded RNA resulting from base pairing between sequences of the 5' flanking region and a second oligonucleotide (*i.e*., first enhancer sequence or 5' enhancer oligonucleotide) can also perform better than short, single stranded inhibitors. In cases where a first enhancer sequence is used to generate a double stranded region, though the position and extent of the duplex region within the 5' flanking region can vary, the minimal length of the enhancer is important. Studies have been performed using enhancer sequences of both 8 and 16 nucleotides in length. In the case of the shorter enhancer sequence of 8 nts, no enhanced function (*e.g.*, characteristic of inhibitors with double stranded regions) was observed. In contrast, similar studies performed with enhancers that were 16 nucleotides in length can lead to improved performance (see Example 8). Though not wanting to be limited by any one theory, the inventors speculate that differences in the thermodynamic stability of duplexes formed between the 5' flanking region and the two enhancer duplexes resulting from the 8 nucleotide enhancer are thought to be unstable and therefore do not consistently generate a lasting duplex region with the first oligonucleotide. In contrast, increasing the length to 16 nucleotides allowed for stable duplexes to be formed, thus providing superior performance over the single stranded counterpart. For this reason, the first enhancer sequence must be greater than 8 nts in length, and can be long as 35 nucleotides. Preferably, the first enhancer sequence has at least greater than about 80% complementarity to the 5' flanking region or portions of the 5' flanking region to which it is designed to anneal to. More preferably the first enhancer sequence has at least greater than about 90% complementarity to the 5' flanking region or portions of the 5' flanking region to which it is designed to anneal to. Most preferably, the first enhancer sequence is about 100% complementary to the 5' flanking region or portions of the 5' flanking region to which it is designed to anneal to. In addition, as was the case in which the duplex region results from two regions in the 5' flanking region annealing with each other, the distance between the duplex region and the 5'-most boundary of the central region can vary greatly; preferably the duplex region of the 5' flanking sequence is adjacent to the boundaries of the central region.

Designs that incorporate enhancer sequences are also desirable from the perspective that they eliminate the effects that sequence or nucleotide content can have on inhibitor function. Studies disclosed in this document (and in priority document USSN 60/774,350) show that the sequence of the flanking regions of single stranded molecules can play a major role in determining functionality. Specifically, the inventors have previously observed that single stranded inhibitors that have unstructured (*e.g.*, pyrimidines rich) flanking regions are less functional than inhibitors that have flanking regions that more closely match mRNA nucleotide content (*i.e.,* referred to as "arbitrary sequences"). As shown in Example 11, such limitations do not apply to the current invention; inhibitors of the invention with either unstructured or mRNA-like flanking regions annealed to enhancer sequences perform equally.

### 3. The 3' Flanking Region

Many of the traits and/or properties associated with 3' flanking sequences are similar to those described for 5' flanking regions. As mentioned above, the 3' flanking region is between about 10 to about 40 nts in length, is 3' of the central region, and is capable of: (1) annealing to itself to create a duplex region, (2) annealing to the 5' flanking region to create a duplex region, (3) annealing to a third oligonucleotide *(i.e.,* second enhancer sequence or 3' enhancer oligonucleotide) to create a double stranded region, or (4) has little or no secondary structure. As was the case with the 5' flanking region, the first three alternatives (*e.g*., annealing to itself, annealing to the opposing (5') flanking region, and annealing to a second enhancer oligonucleotide) creates a region of double stranded RNA which, as demonstrated in Examples 6-19, greatly enhances the functional properties of the inhibitor in an unexpected way. Many of the properties associated with 5' flanking sequences and associated enhancers are similarly applicable to 3' flanking sequences.

In cases where duplex structures present in the 3' flanking region result from sequences present in the 3' flanking region annealing with other sequences present in the 3' flanking region, preferably a hairpin structure is formed. As described above, hairpins comprise a loop structure and a stem region (*i.e*., a duplex region) that results from base pairing of two separate regions (*e.g*., having sufficient levels of complementarity) separated by a non-base pairing region. Preferably, the length of the duplex region is between about 4 and about 20 base pairs in length and the level of complementarity is at least greater than about 80%. More preferably, the length of the duplex is about 6 to about 15 base pairs and the level of complementarity is at least greater than about 80%. More preferably, the length of the duplex is between about 6 and about 10 base pairs in length and the level of complementarity is about 100%. The loop can also vary in size (*e.g.*, ranging from about 4 to about 15 nucleotides in length) and sequence.

As was the case with similar structures in the 5' flanking region, the distance between the 3' terminus of the duplex region and the 3'-most boundary of the central region can vary greatly, preferably the duplex region of the 3' flanking sequence is adjacent to the boundary of the central region. By having the double stranded region immediately adjacent to the border of the central region, the possibility of secondary structures resulting from interactions between the central region and single stranded sequences of the 3' flanking region is minimized.

As stated above, the inventors have also discovered that RNAi inhibitors that comprise regions of double stranded RNA resulting from base pairing between sequences in the 3' and 5' flanking regions perform better than short single stranded inhibitors (see descriptions of 5' flanking region and Example 7).

In addition, the inventors have discovered that inhibitors that comprise double stranded regions resulting from base pairing between sequences of the 3' flanking region and a third oligonucleotide *(i.e.,* second enhancer sequence or 3' enhancer oligonucleotide) can also exhibit superior performance over single stranded inhibitors (see Example 9). Again, though the position and extent of the duplex region in the 3' flanking region can vary (as discussed previously), in cases where a second enhancer sequence is used to generate a region of double stranded RNA, the minimal length of the enhancer is critical. Thus, as was the case with the first enhancer sequence, the second enhancer sequence can be as long as about 35 nucleotides, yet must be longer than about 8 nucleotides. Preferably, the second enhancer sequence has at least greater than about 80% complementarity to the 3' flanking region or portions of the 3' flanking region to which it is designed to anneal to. More preferably, the second enhancer sequence has at least greater than about 90% complementarity to the 3' flanking region or portions of the 3' flanking region to which it is designed to anneal to. Most preferably, the second enhancer sequence has about 100% complementarity to the 3' flanking region or portions of the 3' flanking region to which it is designed to anneal to.

### 4. Double Stranded Inhibitor Designs

The inventors have discovered that the duplex region of this new generation of RNAi inhibitors can be presented in multiple ways and still give rise to enhanced functionality. As shown in multiple examples, inhibitors having duplexes have enhanced functionality compared to short, single stranded (21-32 nt) 2'-O-methyl inhibitor designs that are the reverse complement of the primary miRNA. Such double stranded inhibitors can include the following: (1) hairpins in the 5' flanking region, (2) hairpins in the 3' flanking regions, (3) hairpins in both the 5' and 3' flanking regions, (4) hairpins resulting from annealing the 5' and 3' flanking regions, (5) 5' flanking regions annealing to enhancer sequences, (6) 3' flanking regions annealing to enhancer sequences, (7) 3' and 5' flanking regions annealing to separate enhancer sequences, and (8) any combination of the above *(e.g.,* hairpins in the 5' flanking region plus 3' flanking regions annealing to enhancer sequences). In addition, truncated designs having a duplex region can also exhibit superior performance compared to short (*e.g.*, 21 to 32 nt) single stranded 2'-O-methyl modified inhibitors. Such truncated designs having a duplex region can include a central region and any of the following: (1) a 5' flanking region capable of annealing back upon itself to create a duplex region, (2) a 5' flanking region annealed to a second oligonucleotide (i.*e*., first enhancer sequence or 5' enhancer oligonucleotide) to create a double stranded region, (3) a 3' flanking region capable of annealing back upon itself to create a duplex region, or (4) a 3' flanking region annealed to a third oligonucleotide (*i.e*., second enhancer sequence or 3' enhancer oligonucleotide) to create a double stranded region. In another alternative, the sequence of the 5' and 3' flanking sequence can be the same, thus enabling a single enhancer sequence to be used so that both flanking regions embody a double stranded nature. In cases where an enhancer sequence is used to create the double stranded region, complexes can be blunt ended, or have 3' or 5' overhangs.

### 5. Short Double Stranded Inhibitors

In addition to the double stranded inhibitor designs described above, the inventors have also discovered short double stranded inhibitors. These molecules can include a first strand that includes a modified central region ranging in length between about 17 and about 37 nucleotides annealed to a second strand that includes an oligonucleotide having substantial complementarity to the first strand. Usually, neither the first strand nor the second strand includes a 3' or 5' flanking region. There are several variants of this design that can also exhibit enhanced functionality and delivery. These variants include any of the following: (1) inclusion of one or more bulges or mismatches in the duplex structure, (2) addition of a 2'-O-alkyl group at the C2 position of the ribose ring of the first and/or second 5' nucleotides (*e.g*., counting from the 5' terminus) of the second strand, or (3) mismatches between the first and second strands at the 5' end of the first strand. One or more of these variations can be combined to enhance inhibitor function. Also, the 2'-O-alkyl modification of the central region can be included on each nucleotide or on a portion of the nucleotides as described in other embodiments of inhibitors. While the second strand can include modifications, it can be beneficial for the second strand to be unmodified or otherwise susceptible to degradation. Additionally, the central region of the first strand includes a sequence that has at least partial complementarity to a functional strand of a target miRNA or siRNA. The complementarity of the central region can be substantially the same as described herein with regard to other embodiments of inhibitors. As such, the central region of the first strand can hybridize with the functional strand of the target miRNA or siRNA so as to inhibit the functional strand from regulating a gene through RNAi-mediated gene silencing.

For example, a short double stranded inhibitor can include a first oligonucleotide with a reverse complement region having 3' and 5' ends, and having a reverse complement sequence that is from about 17 to about 37 nucleotides and having at least about 80% complementarity with a target RNA sequence that is capable of silencing a target gene. Additionally, the short double stranded inhibitor can include a second oligonucleotide annealed to and having at least about 80% complementarity with the first oligonucleotide, wherein the second oligonucleotide has from about 17 to about 37 nucleotides.

In one embodiment, at least about 30% of nucleotides in the first oligonucleotide have a 2' modification. In another embodiment, about 100% of nucleotides in the first oligonucleotide have the 2' modification. In yet another embodiment, less than about 30% of nucleotides in the second oligonucleotide have a 2' modification. In still another embodiment, the second oligonucleotide is substantially devoid of having the 2' modification. In any of the embodiments, the 2' modification is a 2'-O-alkyl, 2' orthoester, or 2' ACE modification.

In one embodiment, one or more bulges are included between the first and second oligonucleotides.

In one embodiment, the short double stranded inhibitor can include a conjugate coupled to at least one oligonucleotide of the RNAi inhibitor. That is, a conjugate can be coupled to the first oligonucleotide and/or the second oligonucleotide. Such a conjugate can be at the 5' or 3' end of oligonucleotide. Optionally, the conjugate is conjugated to an end of the second oligonucleotide so as to inhibit the conjugated end from entering RISC. Also, the conjugate can be coupled to the oligonucleotide via a linker.

### III. Conjugates and Linkers

In addition, the inventors have identified inhibitor designs that are compatible with various conjugate chemistries that enhance delivery and/or performance of the inhibitor molecule. Such conjugates can be directly associated with the inhibitor molecules, or can be associated with the inhibitor through a linker molecule.

Previous studies by several groups (*e.g.*, Soutschek, J. et al (2004) Nature 432: 173) have used conventional linker chemistries (*e.g.*, cholesterol-aminocaproic acid-pyrrolidine linker) to attach conjugates to nucleic acids (*e.g.*, siRNAs). As such, the inventors have performed a detailed study of the importance of linker length, and have demonstrated that the overall functionality of nucleic acid-conjugate design is highly dependent on using linkers that have a narrow window of lengths. For this reason, one embodiment of this application is the use of linkers having specific numbers of atoms between the nucleic acid (*e.g.,* inhibitor molecule) and the conjugate *(e.g.,* cholesterol). Preferably, the number of atoms is between about 4 and about 8 in number. More preferably, the number of atoms is between about 4 and about 7 in number. More preferably, the number of atoms is between about 4 and about 6. Most preferably, the number of atoms between the cholesterol and the nucleic acid (*e.g*., inhibitor molecule) is about 5. It is important to note that in this application the length of the linker is described by counting the number atoms that represents the shortest distance between the nitrogen of the carbamate linkage of the conjugate and the terminal phosphate moiety associated with the oligonucleotide. In cases where ring structures are present, counting the atoms around the ring that represent the shortest path is preferred.

While preferred structures of the linker used in the invention include Chol-C5, Chol- PIP, and Chol-ABA, the inventors understand that alternative chemistries can be used and provide a similar length linker. Thus linkers/linker chemistries that are based on ω-amino-1,3-diols, ω-amino-1,2-diols, hydroxyprolinols, ω-amino-alkanols, diethanolamines, ω-hydroxy-1,3-diols, ω-hydroxy-1,2-diols, ω-thio-1,3-diols, ω-thio-1,2-diols, ω-carboxy-1,3-diols, ω-carboxy -1,2-diols, ω-hydroxy-alkanols, ω-thio-alkanols, ω-carboxy-alkanols, functionalized oligoethylene glycols, allyl amines, acrylic acids, allyl alcohols, propargyl amines, propargyl alcohols, and the like can be applied in this context to generate linkers of the appropriate length. Similarly, while the molecular structure of the chemical bond between the linker and the conjugate moiety is currently a carbamate linkage, alternative chemistries including those based on carbamates, ethers, esters, amides, disulfides, thioethers, phosphodiesters, phosphorothioates, phosphorodithioate, sulfonamides, sulfonates, fulfones, sulfoxides, ureas, hydrazides, oximes, photolabile linkages, C-C bond forming groups such as diels-alder cyclo-addition pairs or ring-closing metathesis pairs, and Michael reaction pairs. Lastly, linkers that have the same length, but are capable of associating with two or more conjugates, are also envisioned. Descriptions of the various cholesterol-linker-nucleic acid structures that have been tested for the ability to enhance delivery and functionality and the chemistries used to link conjugates with nucleic acids (*e.g.*, inhibitors) can be found in U.S. Provisional Application No. 60/826,702, from which priority is claimed.

The position of the linker-conjugate moiety on the inhibitor can vary with respect to the following: the strand or strands that are conjugated (*e.g*., first, second, and/or third oligonucleotides); the position or positions within the strand that are modified *(i.e.,* the nucleotide positions within the strand or strands); and the position on the nucleotide(s) that are modified (*e.g*., the sugar and/or the base). Linker-conjugates can be placed on the 5' and/or 3' terminus of one or more of the strands of the invention and/or can be conjugated to internal positions. In addition, multiple positions on the nucleotides including the 5-position of uridine, 5-position of cytidine, 4-position of cytidine, 7-position of guanosine, 7-position of adenosine, 8-position of guanosine, 8-position of adenosine, 6-position of adenosine, 2'-position of ribose, 5'-position of ribose, 3'-position of ribose, can be employed for attachment of the conjugate to the nucleic acid. Preferably, the position of the conjugate or linker-conjugate attachment point is not within the central region of the first oligonucleotide of the inhibitor because this position may disrupt the ability of this molecule to interact with siRNA or miRNA. In cases where the inhibitor comprises a 5' and/or 3' flanking region having a hairpin, the conjugate can be associated with one or more positions on the stem region, the loop region, and/or the terminal region. In cases where the 5' and/or 3' flanking regions are associated with an enhancer region, a conjugate and/or linker conjugate can be associated with the 5', 3' and/or internal positions of the flanking region of the first oligonucleotide, and/or the enhancer region of the second and/or third oligonucleotide. Each of these variants and any combination of the above, are envisioned by the inventors.

Conjugates of the invention can vary widely and target entry into the cell by a variety of means. For instance, conjugates can be lipid in nature and deliver their payload (*e.g.*, inhibitor), by inserting themselves into the membrane and being absorbed into the cell by one of several mechanisms including endocytosis. Accordingly, lipid-based conjugates can include cationic lipids, neutral lipids, sphingolipids, and fatty acids such as stearic, oleic, elaidic, linoleic, linoleaidic, linolenic, and myristic fatty acids. Alternatively, the conjugates can be proteinaceous in nature, such as peptides that are membrane translocating *(e.g.,* TAT, penetratin, or MAP) or cationic (*e.g.*, poly(lys), poly(arg), poly(his), poly (lys/arg/his), or protamine).

Alternatively, the conjugate can be a small molecule that, for instance, targets a particular receptor or is capable of inserting itself into the membrane and being absorbed by endocytic pathways. Thus, small molecules based on adamantanes, polyaromatic hydrocarbons (*e.g*., napthalenes, phenanthrenes, or pyrenes), macrocyles, steroidal, or other chemical backbones, are all potential conjugates for the invention.

In yet another alternative, conjugates can be based on cationic polymers. Numerous studies have demonstrated that cationic polymers such as cationic albumin can greatly enhance delivery to particular cell types and/or tissues (*e.g*. brain delivery; see Lu, W. et. al. (2005) J of Control Release 107:428-448). Given the benefits of these molecules, the conjugate can be a cationic polymers such as polyethyleneimine, dendrimers, poly(alkylpyridinium salts, or cationic albumin.

In some cases, the conjugates are ligands for receptors or can associate with molecules that in turn associate with receptors. Included in this class are conjugates that are steroidal in nature (*e.g.*, cholesterol, pregnolones, progesterones, corticosterones, aldosterones, testosterones, estradiols, ergosterols, and the like), bile acids, small molecule drug ligands, vitamins, aptamers, carbohydrates, peptides (*e.g*., hormones, proteins, protein fragments, antibodies or antibody fragments), viral proteins (*e.g.,* capsids), toxins (*e.g.,* bacterial toxins), and the like. In the case of cholesterol, the molecule can associate with one or more proteins or protein complexes in blood or other body fluid (*e.g*., albumin, LDLs), HDLs, IDLs, VLDLs, chylomicron remnants, and chylomicrons) and can be delivered to the cell through association with the appropriate receptor for that complex (*e.g.*, low density lipoprotein receptor (LDLR)). The example of delivery via the cholesterol-LDL association is particularly attractive since the opportunity for dozens or hundreds of inhibitors to be delivered in a single LDL particle is feasible. For that reason, the inventors can envision packaging cholesterol conjugated inhibitors, or inhibitors conjugated to derivatives of cholesterol, in one or more natural carriers (*e.g*., LDLs) *in vitro,* and using this as *an in vivo* delivery system.

In one embodiment, the conjugates that target a particular receptor are modified to eliminate the possible loss of the conjugated nucleic acid (*e.g*., the inhibitor) to other sources. For instance, when cholesterol-conjugated nucleic acids are placed in the presence of normal serum, a significant fraction of this material will associate with the albumin and/or other proteins in the serum, thus making the inhibitor unavailable for interactions with LDLs. For this reason, it is conceivable that the conjugates of the invention can be modified in such a way that they continue to bind or associate with their intended target *(e.g.,* LDLs) but have lesser affinities with unintended binding partners (*e.g.*, serum albumin).

### IV. Mode of Action

In one embodiment, a target miRNA can silence their target gene by inducing either transcript cleavage (*e.g*., in cases where the mature miRNA and target sequence are 100% complementary) or translation attenuation (*e*.g., in cases where the mature miRNA and target sequence are less than 100% complementary). As shown in multiple examples, the inhibitors of the invention exhibit potent activity irrespective of the mode of action. Thus, when the reverse complement region of the inhibitor is 100% complementary to the target miRNA, inhibitors of the invention are capable of preventing transcript cleavage. In cases where the reverse complement region of the inhibitor has less than 100% complementarity to the miRNA, inhibitors of the invention are potent inhibitors of translation attenuation.

Without wishing to be tied to any one theory as to why the double stranded inhibitors of the invention perform with enhanced potency, the inventors have noted that many of the proteins that mediate RNAi contain double stranded RNA binding domains (*e.g.,* Dicer). Therefore, the inventors speculate that inclusion of double stranded regions in the inhibitor designs facilitates assembly of the RNAi machinery around the inhibitor and thus enhances overall functionality of the inhibitor.

### V. Modifications and Conjugates

The composition of the oligonucleotides of the invention can vary greatly and can include homogeneous nucleic acids (*e.g*., all RNA), heterogeneous nucleic acids *(e.g.,* RNA and DNA), modified nucleic acids, and unmodified nucleic acids (*e.g*., see Example 13). In some instances, the oligonucleotides of the invention include a mixture of modified and unmodified RNA and/or DNA. More preferably, the oligonucleotides of the invention include modified RNA. Even more preferably, the oligonucleotides of the invention comprise 2'-O-alkyl modified ribonucleotides. Most preferably, the oligonucleotides of the invention comprise 2'-O-methyl modified ribonucleotides. In other embodiments, the compositions of the present invention can comprise at least one 2'-orthoester modification, wherein the 2'-orthoester modification is preferably a 2'-bis(hydroxy ethyl) orthoester modification. Alternatively, modifications of the invention can include 2' halogen modifications, or locked nucleic acids (LNAs).

In one embodiment, any of the inhibitor oligonucleotides can include a conjugate. While the conjugate can be selected from a large group consisting of amino acids, peptides, polypeptides, proteins, sugars, carbohydrates, lipids (*e.g.,* cholesterol, see Example 16), polymers (e.g. PEG), nucleotides, polynucleotides, targeted small molecules, and combinations thereof.

One preferred inhibitor design includes a first oligonucleotide containing a central region, a 5' flanking region, and a 3' flanking region. Optionally, the inhibitor can include a truncated first oligonucleotide containing a central region and a 5' flanking region or a central region and a 3' flanking region.

In one embodiment, the inhibitor can include a second oligonucleotide (*i.e*., first enhancer sequence or 5' enhancer oligonucleotide) that can anneal to the 5' flanking region of the first oligonucleotide. The second oligonucleotide can be modified on the 5' terminus, the 3' terminus, and/or at one or more internal regions with a nucleotide modification described herein. Also, the second oligonucleotide can be conjugated at the 5' terminus, 3' terminus, and/or at one or more internal regions with a conjugate, such as a hydrophobic group (*e.g*., a cholesterol, a hydrophobic alkyl chain, such as C3 or longer, or a hydrophobic dye).

In one embodiment, the inhibitor can include a third oligonucleotide *(i.e.,* second enhancer sequence or 3' enhancer oligonucleotide) that can anneal to the 3' flanking region of the first oligonucleotide. The third oligonucleotide can be modified on the 5' terminus, the 3' terminus, and/or at one or more internal regions with a nucleotide modification described herein. Also, the third oligonucleotide can be conjugated at the 5' terminus, 3' terminus, and/or at one or more internal regions with a conjugate, such as a hydrophobic group (*e.g.,* a cholesterol, a hydrophobic alkyl chain, such as C3 or longer, or a hydrophobic dye).

As shown in Examples 10, 16 and 17, several of the preferred inhibitor designs exhibit enhanced functionality due to the inclusion of double stranded regions within the design and minimizes the hurdles associated with manufacturing a large number of individual single stranded inhibitors (*e.g.*, a library of inhibitors) with conjugates (*e.g*., antagomirs). Instead, by designing all the first oligonucleotides with the same 5' and/or 3' flanking regions, the complexities and costs associated with conjugating hydrophobic molecules to a large number of modified oligonucleotide strands can be limited by having only one or two strands (*e.g.*, first enhancer and/or second enhancer) containing the conjugate. Such conjugates can be linked to the appropriate strand using any one of a number art proven chemistries and linkers. Preferably, the linker is similar to the structure shown in Figure 4B.

The conjugate can further comprise a label, such as, for example, a fluorescent label, a radioactive label, or a mass label. In cases where the label is a fluorescent label, the label can be selected from the group consisting of TAMRA, BODIPY, Cy3, Cy5, fluoroscein, and Dabsyl. Alternatively, the fluorescent label can be any fluorescent label known in the art.

In other embodiments, any of the compositions of the present invention can further comprise a 5' and/or 3' overhang, stabilized 5' and/or 3' overhangs, 3' or 5' cap (*e.g*., inverted deoxythymidine) as well as additional modifications that stabilize the oligonucleotides against RNase degradation (*e*.g., internucleotide linkage modifications such as phosphorothioates and methylphosphonates).

### VI. Synthesis

The inhibitor oligonucleotides of the invention can be synthesized by any method that is now known or that comes to be known and that from reading this disclosure a person of ordinary skill in the art would appreciate would be useful to synthesize the molecules of the present invention. For example, oligonucleotides of the invention containing the specified modifications may be chemically synthesized using compositions of matter and methods described in Scaringe, S.A. (2000) "Advanced 5'-silyl-2'-orthoester approach to RNA oligonucleotide synthesis," Methods Enzymol. 317, 3-18; Scaringe, S.A. (2001) "RNA oligonucleotide synthesis via 5'-silyl-2'-orthoester chemistry," Methods 23, 206-217; U.S. Patent No. 5,889,136; U.S. Patent No. 6,008,400; U.S. Patent No. 6111086; and U.S. Patent No. 6,590,093, at -20°C until they are ready for use.

The synthesis method utilizes nucleoside base-protected 5'-O-silyl-2'-O-orthoester-3'-O-phosphoramidites to assemble the desired unmodified oligonucleotide sequences on a solid support in the 3' to 5' direction. Briefly, synthesis of the required phosphoramidites begins from standard base-protected ribonucleosides (*e.g*., uridine, N4-acetylcytidine, N2-isobutyrylguanosine, and N6-isobutyryladenosine). Introduction of the 5'-O-silyl and 2'-O-orthoester protecting groups, as well as the reactive 3'-O-phosphoramidite moiety is then accomplished in five steps, including: (1) Simultaneous transient blocking of the 5'- and 3'-hydroxyl groups of the nucleoside sugar with Markiewicz reagent (1,3-dichloro-1,1,3,3,-tetraisopropyldisiloxane [TIPS-C12]) in pyridine solution (see, Markiewicz, W.T. (1979) "Tetraisopropyldisiloxane-1,3-diyl, a Group for Simultaneous Protection of 3'-and 5'-Hydroxy Functions of Nucleosides," J. Chem. Research(S), 24-25), followed by chromatographic purification; (2) Regiospecific conversion of the 2'-hydroxyl of the TIPS-nucleoside sugar to the bis(acetoxyethyl)orthoester [ACE derivative] using tris(acetoxyethyl)-orthoformate in dichloromethane with pyridinium p-toluenesulfonate as catalyst, followed by chromatographic purification; (3) Liberation of the 5'- and 3'-hydroxyl groups of the nucleoside sugar by specific removal of the TIPS-protecting group using hydrogen fluoride and N,N,N"N'-tetramethylethylene diamine in acetonitrile, followed chromatographic purification; (4) Protection of the 5'-hydroxyl as a 5'-O-silyl ether using benzhydroxy-bis(trimethylsilyloxy)silyl chloride [BzH-Cl] in dichloromethane, followed by chromatographic purification; and (5) Conversion to the 3'-O-phosphoramidite derivative using bis(N,N-diisopropylamino)methoxyphosphine and 5-ethylthio-1H-tetrazole in dichloromethane/acetonitrile, followed by chromatographic purification.

The phosphoramidite derivatives are typically thick, colorless to pale yellow syrups. For compatibility with automated RNA synthesis instrumentation, each of the products is dissolved in a pre-determined volume of anhydrous acetonitrile, and this solution is aliquoted into the appropriate number of serum vials to yield a 1.0 mmole quantity of phosphoramidite in each vial. The vials are then placed in a suitable vacuum desiccator and the solvent removed under high vacuum overnight. The atmosphere is then replaced with dry argon, the vials are capped with rubber septa, and the packaged phosphoramidites are stored at -20°C until needed. Each phosphoramidite is dissolved in sufficient anhydrous acetonitrile to give the desired concentration prior to installation on the synthesis instrument.

The synthesis of the desired oligoribonucleotide is carried out using automated synthesis instrumentation. It begins with the 3'-terminal nucleoside covalently bound via its 3'-hydroxyl to a solid beaded polystyrene support through a cleavable linkage. The appropriate quantity of support for the desired synthesis scale is measured into a reaction cartridge, which is then affixed to synthesis instrument. The bound nucleoside is protected with a 5'-O-dimethoxytrityl moiety, which is removed with anhydrous acid (3% [v/v] dichloroacetic acid in dichloromethane) in order to free the 5'-hydroxyl for chain assembly.

Subsequent nucleosides in the sequence to be assembled are sequentially added to the growing chain on the solid support using a four-step cycle, consisting of the following general reactions:
1. Coupling: the appropriate phosphoramidite is activated with 5-ethylthio-1H-tetrazole and allowed to react with the free 5'-hydroxyl of the support bound nucleoside or oligonucleotide. Optimization of the concentrations and molar excesses of these two reagents, as well as of the reaction time, results in coupling yields generally in excess of98% per cycle.
2. Oxidation: the internucleotide linkage formed in the coupling step leaves the phosphorous atom in its P(III) [phosphite] oxidation state. The biologically-relevant oxidation state is P(V) [phosphate]. The phosphorous is therefore oxidized from P(III) to P(V) using a solution of tert-butylhydroperoxide in toluene.
3. Capping: the small quantity of residual un-reacted 5'-hydroxyl groups must be blocked from participation in subsequent coupling cycles in order to prevent the formation of deletion-containing sequences. This is accomplished by treating the support with a large excess of acetic anhydride and 1-methylimidazole in acetonitrile, which efficiently blocks residual 5'-hydroxyl groups as acetate esters.
4. De-silylation: the silyl-protected 5'-hydroxyl must be deprotected prior to the next coupling reaction. This is accomplished through treatment with triethylamine trihydrogen fluoride in N,N-dimethylformamide, which rapidly and specifically liberates the 5'-hydroxyl without concomitant removal of other protecting groups (2'-O-ACE, N-acyl base-protecting groups, or phosphate methyl).

It should be noted that in between the above four reaction steps are several washes with acetonitrile, which are employed to remove the excess of reagents and solvents prior to the next reaction step. The above cycle is repeated the necessary number of times until the unmodified portion of the oligoribonucleotide has been assembled. The above synthesis method is only exemplary and should not be construed as limited the means by which the molecules may be made. Any method that is now known or that comes to be known for synthesizing siRNA and that from reading this disclosure one skilled in the art would conclude would be useful in connection with the present invention may be employed.

The oligonucleotides of certain embodiments include modified nucleosides (e.g., 2'-O-methyl derivatives). The 5'-O-silyl-2'-O-methyl-3'-O-phosphoramidite derivatives required for the introduction of these modified nucleosides are prepared using procedures similar to those described previously (*e.g*., steps 4 and 5 above), starting from base-protected 2'-O-methyl nucleosides (*e.g.*, 2'-O-methyl-uridine, 2'-O-methyl- N4-acetylcytidine, 2'-O-methyl-N2-isobutyrylguanosine and 2'-O-methyl-N6-isobutyryladenosine). The absence of the 2'-hydroxyl in these modified nucleosides eliminates the need for ACE protection of these compounds. As such, introduction of the 5'-O-silyl and the reactive 3'-O-phosphoramidite moiety is accomplished in two steps, including: (1) Protection of the 5'-hydroxyl as a 5'-O-silyl ether using benzhydroxy-bis(trimethylsilyloxy)silyl chloride (BzH-C1) in N,N-dimethylformamide, followed by chromatographic purification; and (2) Conversion to the 3'-O-phosphoramidite derivative using bis(N,N-diisopropylamino)methoxyphosphine and 5-ethylthio-1H-tetrazole in dichloromethane/acetonitrile, followed by chromatographic purification.

Post-purification packaging of the phosphoramidites is carried out using the procedures described previously for the standard nucleoside phosphoramidites. Similarly, the incorporation of the two 5'-O-silyl-2'-O-methyl nucleosides via their phosphoramidite derivatives is accomplished by twice applying the same four-step cycle described previously for the standard nucleoside phosphoramidites.

The oligonucleotides of certain embodiments can, but need not, include a phosphate moiety at the 5'-end of the strand. If desired, this phosphate is introduced chemically as the final coupling to the sequence. The required phosphoramidite derivative *(e.g.,* bis(cyanoethyl)-N,N-diisopropylamino phosphoramidite) is synthesized as follows. Briefly, phosphorous trichloride is treated one equivalent of N,N-diisopropylamine in anhydrous tetrahydrofuran in the presence of excess triethylamine. Then, two equivalents of 3-hydroxypropionitrile are added and allowed to react completely. Finally, the product is purified by chromatography. Post-purification packaging of the phosphoramidite is carried out using the procedures described previously for the standard nucleoside phosphoramidites. Similarly, the incorporation of the phosphoramidite at the 5'-end of the strand is accomplished by applying the same four-step cycle described previously for the standard nucleoside phosphoramidites.

The modified, protected oligoribonucleotide remains linked to the solid support at the finish of chain assembly. A two-step rapid cleavage/deprotection procedure is used to remove the phosphate methyl protecting groups, cleave the oligoribonucleotide from the solid support, and remove the N-acyl base-protecting groups. It should be noted that this procedure also removes the cyanoethyl protecting groups from the 5'-phosphate on the strand. Additionally, the procedure removes the acetyl functionalities from the ACE orthoester, converting the 2'-O-ACE protecting group into the bis(2-hydroxyethyl)orthoester. This new orthoester is significantly more labile to mild acid as well as more hydrophilic than the parent ACE group. The two-step procedure is briefly as follows:
1. The support-bound oligoribonucleotide is treated with a solution of disodium 2-carbamoyl-2-cyanoethylene-1,1-dithiolate trihydrate in N,N-dimethylformamide. This reagent rapidly and efficiently removes the methyl protecting groups from the internucleotide phosphate linkages without cleaving the oligoribonucleotide from the solid support. The support is then washed with water to remove excess dithiolate.
2. The oligoribonucleotide is cleaved from the solid support with 40% (w/v) aqueous methylamine at room temperature. The methylamine solution containing the crude oligoribonucleotide is then heated to 55°C to remove the protecting groups from the nucleoside bases. The crude orthoester-protected oligoribonucleotide is obtained following solvent removal in vacuo.

When desired, removal of the 2'-orthoesters is the final step in the synthesis process. This is accomplished by treating the crude oligoribonucleotide with an aqueous solution of acetic acid and N,N,N',N'-tetramethyl ethylene diamine, pH 3.8, at 55°C for 35 minutes. The completely deprotected oligoribonucleotide is then desalted by ethanol precipitation and isolated by centrifugation. In cases where retention of this group is preferred, this step is omitted.

While the preferred composition of the invention comprises a ribonucleotide where all of the nucleotides contain an alkyl modification (*e.g.,* preferably a 2'-O-in some cases, mixtures of 2'-O-alkyl and 2'-ACE modified nucleotides are desired. Such hybrid modified molecules are easily synthesized by introducing the appropriate precursors at the appropriate time during synthesis. In addition, supplementary modifications, including 2' halogen modifications (*e.g*., F, Cl, Br, I,), internucleotide modifications such as methylphosphonates and phosphorothioates, and base analogs can be included in the design of the inhibitors of the invention. Examples of positions of the nucleotide which may be derivatized include the following: the 5 position, such as 5-(2-amino)propyl uridine, 5-bromo uridine, 5-propyne uridine, 5-propenyl uridine, etc.; the 6 position, such as 6-(2-amino)propyl uridine; and the 8-position for adenosine and/or guanosines, such as 8-bromo guanosine, 8-chloro guanosine, 8-fluoroguanosine, etc. Nucleotide analogs also include the following: deaza nucleotides, such as 7-deaza-adenosine; 0- and N-modified (*e.g*., alkylated, e.g., N6-methyl adenosine, or as otherwise known in the art) nucleotides; and other heterocyclically modified nucleotide analogs such as those described in Herdewijn, Antisense Nucleic Acid Drug Dev., 2000 Aug. 10(4):297-310.

In addition, inhibitor oligonucleotides of the invention can by synthesized with an array of conjugates that enhance delivery, or allow visualization of the molecule in a cell or organism. Preferred conjugates for delivery include cholesterol, PEG, peptides, proteins, sugars, carbohydrates, and moieties or combinations of moieties that enhance cellular uptake. Additional conjugates can include fluorescent labels, such as fluorescein, lissamine, phycoerythrin, rhodamine (Perkin Elmer Cetus™), Cy2, Cy3, Cy3.5, Cy5, Cy5.5, Cy7, FluorX (Amersham™) and others (see, Kricka (1992) Nonisotopic DNA Probe Techniques, Academic Press San Diego, Calif.). Other labels include radioactive labels or mass labels. All of the before mentioned conjugates or labels can be associated with the 5' or 3' end of the molecule or can be conjugated to internal regions using methods described in the US patent 8,026,349.

### VII. Methods for Using Inhibitors

The inhibitors of the present invention may be administered to a cell by any method that is now known or that comes to be known and that from reading this disclosure, one skilled in the art would conclude would be useful with the present invention. For example, the inhibitor molecules of the invention may be passively delivered to cells. Passive uptake of an inhibitor can be modulated, for example, by the presence of a conjugate such as a polyethylene glycol moiety or a cholesterol moiety, or any other hydrophobic moiety associated with the 5' terminus, the 3' terminus, or internal regions of the first oligonucleotide, and/or one or more of the enhancer oligonucleotides. Other methods for inhibitor delivery include, but are not limited to, transfection techniques (*e.g*., using forward or reverse transfection techniques) employing DEAE-Dextran, calcium phosphate, cationic lipids/liposomes, microinjection, electroporation, immunoporation, and coupling of the inhibitors to specific conjugates or ligands such as antibodies, peptides, antigens, or receptors.

### VIII. Quantifying Inhibitor Function

The method of assessing the level of inhibition is not limited. Thus, the effects of any inhibitor can be studied by one of any number of art tested procedures including but not limited to Northern analysis, RT PCR, expression profiling, and others. In one preferred method, a vector or plasmid encoding reporter whose protein product is easily assayed is modified to contain the target site *(e.g.,* reverse complement of the mature miRNA, piRNA, or siRNA) in the 5' UTR, ORF, or 3'UTR of the sequence. Such reporter genes include alkaline phosphatase (AP), beta galactosidase (LacZ), chloramphenicol acetyltransferase (CAT), green fluorescent protein (GFP), variants of luciferase (Luc), and derivatives thereof. In the absence of the inhibitor, endogenous (or exogenously added) miRNAs target the reporter mRNA for silencing *(e.g.,* either by transcript cleavage or translation attenuation) thus leading to an overall low level of reporter expression. In contrast, in the presence of the inhibitors of the invention, miRNA, piRNA, or siRNA mediated targeting is suppressed, thus giving rise to a heightened level of reporter expression. Preferred reporter constructs include the psiCHECK-2 dual luciferase reporter (Promega).

### IX. Applications

The inhibitors of the present invention may be used in a diverse set of applications, including basic research. For example, the inhibitors of the present invention may be used to validate whether one or more miRNAs or targets of miRNA may be involved in cell maintenance, cell differentiation, development, or a target for drug discovery or development. Inventive inhibitors that are specific for inhibiting a particular miRNA are introduced into a cell or organism and said cell or organism is maintained under conditions that allow for specific inhibition of the targeted molecule. The extent of any decreased expression or activity of the target is then measured, along with the effect of such decreased expression or activity, and a determination is made that if expression or activity is decreased, then the target is an agent for drug discovery or development. In this manner, phenotypically desirable effects can be associated with inhibition of particular target of interest, and in appropriate cases toxicity and pharmacokinetic studies can be undertaken and therapeutic preparations developed.

The inhibitors of the invention can be used to inhibit single or multiple targets simultaneously. The ability to inhibit multiple targets is one of the innovations of the invention (see Examples 4 and 19). The authors recognize that previous inhibitor designs lacked potency and as such, required high concentrations to partially inhibit a single miRNA. Introduction of pools of inhibitors using previous designs would require excessively high concentrations because there are limited amounts of RISC available in cells, high concentrations of inhibitors can be cytotoxic, and addition of high levels of inhibitors could lead to a global disruption of the RNAi pathway and non-specific effects. In contrast, the enhanced potency of the inhibitors of the invention enables users to inhibit one or more specific targets at concentrations that preserve the overall functionality of the RNAi pathway with minimal non-specific effects. Knockdown of multiple targets can take place by introducing pools of inhibitors targeting different molecules. Alternatively, inhibitors can be designed such that single inhibitor molecules can inhibit multiple targets. In one non-limiting example, inhibitors designs can include the following: a 5' flanking region; a central region targeting gene A; a central region targeting gene B, and a first enhancer sequence capable of binding the 5' flanking region.

Because the inhibitors of the invention act independent of the cell type or species into which they are introduced, the present invention is applicable across a broad range of organisms. For example, the inhibitors can be used in plants, animals, protozoa, bacteria, viruses, and fungi. The present invention is particularly advantageous for use in mammals such as cattle, horse, goats, pigs, sheep, canines, rodents such as hamsters, mice, and rats, and primates such as, gorillas, bush babies, chimpanzees, and humans.

The present invention may be used advantageously with diverse cell types, such as primary cells, germ cell lines, and somatic cells. For example, the cell types may be embryonic cells, oocytes, sperm cells, adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes and cells of the endocrine or exocrine glands.

Advantageously, the present invention can be used to inhibit a broad range of miRNA, piRNA, and siRNAs. For example, the inhibitors can be used to inhibit miRNA and/or piRNAs of the human genome implicated in diseases, such as diabetes, Alzheimer's, and cancer, and miRNA and/or piRNAs associated with the genomes of pathogens (*e.g*., pathogenic viruses).

Additionally, the inhibitors of the present invention may be used in RNA interference applications, such as diagnostics, prophylactics, and therapeutics. This can include using inhibitors in the manufacture of a medicament for prevention and/or treatment of animals, such as mammals (*e.g*., humans). In particular, the inhibitors of the invention can be used to reverse the action of siRNAs, miRNAs, or piRNAs that are being used as therapeutic agents.

In the case of therapeutic or prophylactic purposes, dosages of medicaments manufactured in accordance with the present invention may vary from micrograms per kilogram to hundreds of milligrams per kilogram of a subject. As is known in the art, dosage will vary according to the mass of the mammal receiving the dose, the nature of the mammal receiving the dose, the severity of the disease or disorder, and the stability of the medicament in the serum of the subject, among other factors well known to persons of ordinary skill in the art. For these applications, an organism suspected of having a disease or disorder that is amenable to modulation by manipulation of a particular target nucleic acid of interest is treated by administering inhibitors of the invention. Results of the treatment may be ameliorative, palliative, prophylactic, and/or diagnostic of a particular disease or disorder.

Therapeutic or prophylactic applications of the present invention can be performed with a variety of therapeutic inhibitor compositions and methods of inhibitor administration. Pharmaceutically acceptable carriers and diluents are known to persons skilled in the art. Methods of administration to cells and organisms are also known to persons skilled in the art. Dosing regimens, for example, are known to depend on the severity and degree of responsiveness of the disease or disorder to be treated, with a course of treatment spanning from days to months, or until the desired effect on the disorder or disease state is achieved. Chronic administration of inhibitors of the invention may be required for lasting desired effects with some diseases or disorders. Suitable dosing regimens can be determined by, for example, administering varying amounts of one or more inhibitors in a pharmaceutically acceptable carrier or diluent, by a pharmaceutically acceptable delivery route, and amount of drug accumulated in the body of the recipient organism can be determined at various times following administration. Similarly, the desired effect can be measured at various times following administration of the inhibitor, and this data can be correlated with other pharmacokinetic data, such as body or organ accumulation. Those of ordinary skill can determine optimum dosages, dosing regimens, and the like. Those of ordinary skill may employ EC₅₀ data from *in vivo* and *in vitro* animal models as guides for human studies.

The inhibitors of the invention can be administered in a cream or ointment topically, an oral preparation such as a capsule or tablet or suspension or solution, and the like. The route of administration may be intravenous, intramuscular, dermal, subdermal, cutaneous, subcutaneous, intranasal, oral, rectal, by eye drops, by tissue implantation of a device that releases the inhibitor at an advantageous location, such as near an organ or tissue or cell type harboring a target nucleic acid of interest.

Having described the invention with a degree of particularity, examples will now be provided. These examples are not intended to and should not be construed to limit the scope of the claims in any way. Although the invention may be more readily understood through reference to the following examples, they are provided by way of illustration and are not intended to limit the present invention unless specified.

**Table 1**

| **Sequences of Inhibitors Targeting Human, Mouse, and Rat miRNAs** | | |
|---|---|---|
| **Mature Accession** | **Precursor Accession** | **Sequence of Inhibitors Targeting Human miRNAs (SEQ ID NOS 20-413)** |
| MIMAT0000062 | MI0000060 | UAAAACUAUACAACCUACUACCUCAUCC |
| MIMAT0000062 | MI0000061 | CUAAACUAUACAACCUACUACCUCAACC |
| MIMAT0000062 | MI0000062 | CCAAACUAUACAACCUACUACCUCACCC |
| MIMAT0000063 | MI0000063 | UGAAACCACACAACCUACUACCUCACCC |
| MIMAT0000064 | MI0000064 | CUAAACCAUACAACCUACUACCUCAACC |
| MIMAT0000063 | MI0000065 | UAAAACUAUGCAACCUACUACCUCUUCC |
| MIMAT0000066 | MI0000066 | CUCAACUAUACAACCUCCUACCUCAGCC |
| MIMAT0000067 | MI0000067 | CACAACUAUACAAUCUACUACCUCACUC |
| MIMAT0000067 | MI0000068 | UAAAACUAUACAAUCUACUACCUCAUCC |
| MIMAT0000068 | MI0000069 | AUCCACAAACCAUUAUGUGCUGCUACUU |
| MIMAT0000069 | MI0000070 | UAACGCCAAUAUUUACGUGCUGCUAAGG |
| MIMAT0000070 | MI0000071 | UCACUACCUGCACUGUAAGCACUUUGAC |
| MIMAT0000071 | MI0000071 | UGCUACAAGUGCCUUCACUGCAGUAGAU |
| MIMAT0000072 | MI0000072 | CACUAUCUGCACUAGAUGCACCUUAGAA |
| MIMAT0002891 | MI0000072 | UGCCAGAAGGAGCACUUAGGGCAGUAGA |
| MIMAT0000073 | MI0000073 | CCAUCAGUUUUGCAUAGAUUUGCACAAC |
| MIMAT0000074 | MI0000074 | CAGUCAGUUUUGCAUGGAUUUGCACAGC |
| MIMAT0000074 | MI0000075 | CAAUCAGUUUUGCAUGGAUUUGCACAGC |
| MIMAT0000075 | MI0000076 | ACACUACCUGCACUAUAAGCACUUUAGU |
| MIMAT0000076 | MI0000077 | CAGUCAACAUCAGUCUGAUAAGCUACCC |
| MIMAT0000077 | MI0000078 | GCAACAGUUCUUCAACUGGCAGCUUUAG |
| MIMAT0000078 | MI0000079 | GGUUGGAAAUCCCUGGCAAUGUGAUUUG |
| MIMAT0000079 | MI0000080 | AGAACUGAUAUCAGCUCAGUAGGCACCG |
| MIMAT0000080 | MI0000080 | CUCCUGUUCCUGCUGAACUGAGCCAGUG |
| MIMAT0000080 | MI0000081 | CCCCUGUUCCUGCUGAACUGAGCCAGUG |
| MIMAT0000081 | MI0000082 | CUGUCAGACCGAGACAAGUGCAAUGCCC |
| MIMAT0000082 | MI0000083 | CACAGCCUAUCCUGGAUUACUUGAACGA |
| MIMAT0000083 | MI0000084 | CACAACCUAUCCUGAAUUACUUGAACUG |
| MIMAT0000084 | MI0000085 | GGGGGCGGAACUUAGCCACUGUGAACAC |
| MIMAT0000085 | MI0000086 | UAACUCAAUAGACUGUGAGCUCCUUGAG |
| MIMAT0000086 | MI0000087 | UUAUAACCGAUUUCAGAUGGUGCUAGAA |
| MIMAT0000087 | MI0000088 | CAGCUUCCAGUCGAGGAUGUUUACAGUC |
| MIMAT0000088 | MI0000088 | GCAGCUGCAAACAUCCGACUGAAAGCCC |
| MIMAT0000089 | MI0000089 | UCAACAGCUAUGCCAGCAUCUUGCCUCC |
| MIMAT0000090 | MI0000090 | ACAUGCAACUUAGUAAUGUGCAAUAUCU |
| MIMAT0000091 | MI0000091 | ACAUGCAAUGCAACUACAAUGCACCACA |
| MIMAT0000092 | MI0000093 | UCAACAGGCCGGGACAAGUGCAAUACCA |
| MIMAT0000092 | MI0000094 | UCCACAGGCCGGGACAAGUGCAAUACUU |
| MIMAT0000093 | MI0000095 | ACACUACCUGCACGAACAGCACUUUGGA |
| MIMAT0000094 | MI0000097 | GGGUGCUCAAUAAAUACCCGUUGAAUGU |
| MIMAT0000095 | MI0000098 | CAAGCAAAAAUGUGCUAGUGCCAAAAUC |
| MIMAT0000097 | MI0000101 | CACCACAAGAUCGGAUCUACGGGUUUAU |
| MIMAT0000098 | MI0000102 | UACCACAAGUUCGGAUCUACGGGUUUGU |
| MIMAT0000099 | MI0000103 | AUCCUUCAGUUAUCACAGUACUGUACCU |
| MIMAT0000100 | MI0000105 | AAGAACACUGAUUUCAAAUGGUGCUAGA |
| MIMAT0000100 | MI0000107 | UAAAACACUGAUUUCAAAUGGUGCUAGA |
| MIMAT0000101 | MI0000108 | CUUUCAUAGCCCUGUACAAUGCUGCUUG |
| MIMAT0000101 | MI0000109 | CCUUCAUAGCCCUGUACAAUGCUGCUUG |
| MIMAT0000102 | MI0000111 | CACCACAGGAGUCUGAGCAUUUGACCAC |
| MIMAT0000102 | MI0000112 | CACCACAGGAGUCUGAGCAUUUGACCAC |
| MIMAT0000103 | MI0000113 | AAGCUACCUGCACUGUAAGCACUUUUAC |
| MIMAT0000104 | MI0000114 | CUUUGAUAGCCCUGUACAAUGCUGCUUG |
| MIMAT0000069 | MI0000115 | CUACGCCAAUAUUUACGUGCUGCUAGAG |
| MIMAT0000222 | MI0000234 | CACUGGCUGUCAAUUCAUAGGUCAGAGC |
| MIMAT0000226 | MI0000238 | AGGCCCAACAACAUGAAACUACCUAAUU |
| MIMAT0000227 | MI0000239 | CAUGCUGGGUGGAGAAGGUGGUGAAGGG |
| MIMAT0000228 | MI0000240 | AGGAACCUAUCUCCCCUCUGGACCAAUG |
| MIMAT0000232 | MI0000242 | CCUAACCAAUGUGCAGACUACUGUACAC |
| MIMAT0000231 | MI0000242 | CCUGAACAGGUAGUCUGAACACUGGGUU |
| MIMAT0000241 | MI0000251 | CCAACAAGCUUUUUGCUCGUCUUAUACG |
| MIMAT0000242 | MI0000252 | AACAGCAAGCCCAGACCGCAAAAAGAUC |
| MIMAT0000243 | MI0000253 | GAGACAAAGUUCUGUAGUGCACUGACUU |
| MIMAT0000244 | MI0000254 | ACAGCUGAGAGUGUAGGAUGUUUACAGU |
| MIMAT0000245 | M10000255 | CAGCUUCCAGUCGGGGAUGUUUACAACA |
| MIMAT0000250 | MI0000261 | ACUGGAGACACGUGCACUGUAGAAUACA |
| MIMAT0000251 | MI0000262 | UCUAGCAGAAGCAUUUCCACACACUGGC |
| MIMAT0000252 | MI0000263 | AAACAACAAAAUCACUAGUCUUCCACAC |
| MIMAT0000252 | MI0000264 | CAACAACAAAAUCACUAGUCUUCCAGAU |
| MIMAT0000252 | MI0000265 | GAACAACAAAAUCACUAGUCUUCCACAC |
| MIMAT0000253 | MI0000266 | UUACACAAAUUCGGAUCUACAGGGUAUA |
| MIMAT0000254 | MI0000267 | CACACAAAUUCGGUUCUACAGGGUAUAU |
| MIMAT0000255 | MI0000268 | CACAACAACCAGCUAAGACACUGCCAAA |
| MIMAT0000256 | MI0000269 | CAAACUCACCGACAGCGUUGAAUGUUCC |
| MIMAT0000257 | MI0000270 | CAACCCACCGACAGCAAUGAAUGUUGAU |
| MIMAT0000258 | MI0000271 | CAAACUCACCGACAGGUUGAAUGUUCCC |
| MIMAT0000260 | MI0000272 | CCCAUAGUUGGCAAGUCUAGAACCACCG |
| MIMAT0000259 | MI0000272 | CAGUGUGAGUUCUACCAUUGCCAAAAAC |
| MIMAT0000261 | MI0000273 | UCACAGUGAAUUCUACCAGUGCCAUACA |
| MIMAT0000262 | MI0000274 | CCUCCGGCUGCAACACAAGACACGAGGG |
| MIMAT0000226 | MI0000279 | AAUCCCAACAACAUGAAACUACCUAAGC |
| MIMAT0000232 | MI0000281 | UCUAACCAAUGUGCAGACUACUGUACAA |
| MIMAT0000231 | MI0000281 | CCUGAACAGGUAGUCUGAACACUGGGGC |
| MIMAT0000263 | MI0000282 | CCUGAACAGAUAGUCUAAACACUGGGUA |
| MIMAT0000264 | MI0000283 | GGUCUAGUGGUCCUAAACAUUUCACAAU |
| MIMAT0000265 | MI0000284 | CUCAGGCAUAGGAUGACAAAGGGAAGUC |
| MIMAT0000266 | MI0000285 | AGACAGACUCCGGUGGAAUGAAGGACAA |
| MIMAT0000267 | MI0000286 | AGAUCAGCCGCUGUCACACGCACAGUGG |
| MIMAT0000268 | MI0000287 | CCUAGGCGAAGGAUGACAAAGGGAAGCC |
| MIMAT0000269 | MI0000288 | CGGUGGCCGUGACUGGAGACUGUUACUG |
| MIMAT0000270 | MI0000289 | UAGGGUACAAUCAACGGUCGAUGGUUUU |
| MIMAT0000256 | MI0000289 | CAAACUCACCGACAGCGUUGAAUGUUCA |
| MIMAT0000271 | MI0000290 | GUGACUGCCUGUCUGUGCCUGCUGUACA |
| MIMAT0000272 | MI0000291 | UAUUGUCUGUCAAUUCAUAGGUCAUUUU |
| MIMAT0000273 | MI0000292 | AUCUCACAGUUGCCAGCUGAGAUUAAGC |
| MIMAT0000274 | MI0000293 | UUAUCCAAUCAGUUCCUGAUGCAGUAUC |
| MIMAT0000275 | MI0000294 | AACCACAUGGUUAGAUCAAGCACAACAG |
| MIMAT0000275 | MI0000295 | CACCACAUGGUUAGAUCAAGCACAAAGG |
| MIMAT0000276 | MI0000296 | CUCGAGAAUUGCGUUUGGACAAUCAGGA |
| MIMAT0000277 | MI0000297 | GCCCAAAGUGUCAGAUACGGUGUGGAGC |
| MIMAT0000278 | MI0000298 | CCUGAAACCCAGCAGACAAUGUAGCUGU |
| MIMAT0000279 | MI0000299 | CAGAGACCCAGUAGCCAGAUGUAGCUGC |
| MIMAT0000280 | MI0000300 | ACUUGGGGUAUUUGACAAACUGACACUC |
| MIMAT0000281 | MI0000301 | UACUAAACGGAACCACUAGUGACUUGAA |
| MIMAT0000318 | MI0000342 | GCCGUCAUCAUUACCAGGCAGUAUUAGA |
| MIMAT0000414 | MI0000433 | UCAAACUGUACAAACUACUACCUCAGCC |
| MIMAT0000415 | MI0000434 | ACCAACAGCACAAACUACUACCUCAGCC |
| MIMAT0000416 | MI0000437 | AAAAUACAUACUUCUUUACAUUCCAUAG |
| MIMAT0000417 | MI0000438 | GCAUGUAAACCAUGAUGUGCUGCUACAG |
| MIMAT0000418 | MI0000439 | GCGUGGUAAUCCCUGGCAAUGUGAUUUU |
| MIMAT0000419 | MI0000440 | AGGUGCAGAACUUAGCCACUGUGAACAA |
| MIMAT0000420 | MI0000441 | UACAGCUGAGUGUAGGAUGUUUACAUGA |
| MIMAT0000421 | MI0000442 | GACACAAACACCAUUGUCACACUCCACA |
| MIMAT0000422 | MI0000443 | UCUUGGCAUUCACCGCGUGCCUUAAUUG |
| MIMAT0000422 | MI0000444 | UCUUGGCAUUCACCGCGUGCCUUAAUUG |
| MIMAT0000422 | MI0000445 | UCUUGGCAUUCACCGCGUGCCUUAAUUG |
| MIMAT0000423 | MI0000446 | ACAUCACAAGUUAGGGUCUCAGGGACUG |
| MIMAT0000424 | MI0000447 | UGAAAAAGAGACCGGUUCACUGUGAGAA |
| MIMAT0000425 | MI0000448 | CCAAUGCCCUUUUAACAUUGCACUGCUA |
| MIMAT0000426 | MI0000449 | GGGCGACCAUGGCUGUAGACUGUUACCU |
| MIMAT0000427 | M10000450 | GCUACAGCUGGUUGAAGGGGACCAAAUC |
| MIMAT0000427 | MI0000451 | GCUACAGCUGGUUGAAGGGGACCAAAUC |
| MIMAT0000428 | MI0000452 | GAAUCACAUAGGAAUAAAAAGCCAUAGA |
| MIMAT0000428 | MI0000453 | CUAUCACAUAGGAAUAAAAAGCCAUAAA |
| MIMAT0000429 | MI0000454 | CGACUACGCGUAUUCUUAAGCAAUAACA |
| MIMAT0000430 | MI0000455 | CGGCCUGAUUCACAACACCAGCUGCAGC |
| MIMAT0000431 | MI0000456 | UAACCUACCAUAGGGUAAAACCACUGGC |
| MIMAT0000432 | MI0000457 | GAGCCAUCUUUACCAGACAGUGUUAGGA |
| MIMAT0000434 | MI0000458 | UCAUCCAUAAAGUAGGAAACACUACACC |
| MIMAT0000433 | MI0000458 | GUUAGUAGUGCUUUCUACUUUAUGGGUG |
| MIMAT0000435 | MI0000459 | UCCUGAGCUACAGUGCUUCAUCUCAGAC |
| MIMAT0000436 | MI0000460 | GGACUAGUACAUCAUCUAUACUGUAGUG |
| MIMAT0000437 | MI0000461 | CUAAGGGAUUCCUGGGAAAACUGGACCG |
| MIMAT0000438 | MI0000462 | GGGCCCAAGUUCUGUCAUGCACUGACUG |
| MIMAT0000439 | MI0000463 | AUGAUCACUUUUGUGACUAUGCAACUGG |
| MIMAT0000439 | MI0000464 | AUGAUCACUUUUGUGACUAUGCAACUGG |
| MIMAT0000440 | MI0000465 | AACAGCUGCUUUUGGGAUUCCGUUGCCC |
| MIMAT0001618 | MI0000465 | GCAGGGGACGAAAUCCAAGCGCAGCUGG |
| MIMAT0000442 | MI0000466 | UUUUACUUUCGGUUAUCUAGCUUUAUGA |
| MIMAT0000441 | MI0000466 | CACUCAUACAGCUAGAUAACCAAAGAUA |
| MIMAT0000442 | MI0000467 | UUUUACUUUCGGUUAUCUAGCUUUAUGA |
| MIMAT0000441 | MI0000467 | CACUCAUACAGCUAGAUAACCAAAGAUA |
| MIMAT0000442 | MI0000468 | UUCUACUUUCGGUUAUCUAGCUUUAUGA |
| MIMAT0000441 | MI0000468 | CACUCAUACAGCUAGAUAACCAAAGAGA |
| MIMAT0000443 | MI0000469 | CCUCACAGGUUAAAGGGUCUCAGGGACC |
| MIMAT0000423 | MI0000470 | ACCUCACAAGUUAGGGUCUCAGGGACUA |
| MIMAT0000444 | MI0000471 | ACAGCGCGUACCAAAAGUAAUAAUGUCC |
| MIMAT0000445 | MI0000471 | CGGCGCAUUAUUACUCACGGUACGAGUU |
| MIMAT0000446 | MI0000472 | ACCAGCCAAGCUCAGACGGAUCCGAUGA |
| MIMAT0000242 | MI0000473 | UACAGCAAGCCCAGACCGCAAAAAGAUU |
| MIMAT0000447 | MI0000474 | AUGCCCCUCUGGUCAACCAGUCACACAC |
| MIMAT0000448 | MI0000475 | GAAUCCAUCAUCAAAACAAAUGGAGUCC |
| MIMAT0000430 | MI0000476 | CGGCCUGAUUCACAACACCAGCUGCCCC |
| MIMAT0000449 | MI0000477 | CACAACCCAUGGAAUUCAGUUCUCAAAG |
| MIMAT0000450 | MI0000478 | ACGGGAGUGAAGACACGGAGCCAGAGCU |
| M1MAT0000451 | MI0000479 | CAGCACUGGUACAAGGGUUGGGAGACAG |
| MIMAT0000452 | MI0000480 | AAGCGAAGGCAACACGGAUAACCUAUCU |
| MIMAT0000453 | MI0000480 | AAAAAUAGGUCAACCGUGUAUGAUUCGU |
| MIMAT0000454 | MI0000481 | CCUACCCUUAUCAGUUCUCCGUCCAACA |
| MIMAT0000455 | MI0000482 | AUCAGGAACUGCCUUUCUCUCCAAUCCC |
| MIMAT0000456 | MI0000483 | AGAAAGCCCAAAAGGAGAAUUCUUUGGA |
| MIMAT0000457 | MI0000484 | CUCACCCUCCACCAUGCAAGGGAUGUGA |
| MIMAT0000458 | MI0000486 | ACAACCUAAUAUAUCAAACAUAUCACAC |
| MIMAT0000459 | MI0000487 | AGAACUGGGACUUUGUAGGCCAGUUGAU |
| MIMAT0000460 | MI0000488 | CAGUCCACAUGGAGUUGCUGUUACACUU |
| MIMAT0000461 | MI0000489 | CUGUGCCAAUAUUUCUGUGCUGCUAGAG |
| MIMAT0000462 | MI0000490 | AAACCACACACUUCCUUACAUUCCAUAG |
| MIMAT0000510 | MI0000542 | UUUUUCGCCCUCUCAACCCAGCUUUUCC |
| MIMAT0000617 | MI0000650 | CCUCCAUCAUUACCCGGCAGUAUUAGAG |
| MIMAT0000416 | MI0000651 | GAGAUACAUACUUCUUUACAUUCCAUAG |
| MIMAT0000646 | MI0000681 | AAACCCCUAUCACGAUUAGCAUUAACAG |
| MIMAT0000257 | MI0000683 | AAACCCACCGACAGCAAUGAAUGUUGAG |
| MIMAT0000676 | MI0000727 | AGGGAAAGAGACCGGUUCACUGUGAGAC |
| MIMAT0000680 | MI0000734 | CACUAUCUGCACUGUCAGCACUUUAGCC |
| MIMAT0000681 | MI0000735 | CAUAACCGAUUUCAAAUGGUGCUAGACA |
| MIMAT0000244 | MI0000736 | ACAGCUGAGAGUGUAGGAUGUUUACACA |
| MIMAT0000682 | MI0000737 | UGAACAUCGUUACCAGACAGUGUUAGAG |
| MIMAT0001620 | MI0000737 | AAAUCCAGCACUGUCCGGUAAGAUGCUC |
| MIMAT0000683 | MI0000738 | UUCAAAGCAAGUACAUCCACGUUUAAGU |
| MIMAT0000684 | MI0000738 | CCAUCACCAAAACAUGGAAGCACUUACU |
| MIMAT0000099 | MI0000739 | AUUCUUCAGUUAUCACAGUACUGUACCU |
| MIMAT0000276 | MI0000740 | UACAAGAAUUGCGUUUGGACAAUCAGUG |
| MIMAT0000685 | MI0000742 | GUACAAUCAGCUAAUGACACUGCCUACA |
| MIMAT0000686 | MI0000743 | UUAGCAAUCAGCUAACUACACUGCCUAG |
| MIMAT0000687 | MI0000744 | AAGAAGCGGUUUACCAUCCCACAUACAU |
| MIMAT0002890 | MI0000744 | AAAAUGUAUGUGGGACGGUAAACCAUUU |
| MIMAT0000688 | MI0000745 | GAUGCUUUGACAAUACUAUUGCACUGCU |
| MIMAT0000689 | MI0000746 | CCCCGCAAGGUCGGUUCUACGGGUGGGU |
| MIMAT0000690 | MI0000747 | CACAACAGGAUUGAGGGGGGGCCCUCUG |
| MIMAT0000691 | MI0000748 | CCGAUGCCCUUUCAUCAUUGCACUGCUU |
| MIMAT0000692 | MI0000749 | AGCUUCCAGUCAAGGAUGUUUACAGUAG |
| MIMAT0000693 | MI0000749 | GCCGCUGUAAACAUCCGACUGAAAGCUC |
| MIMAT0000082 | MI0000750 | AACAGCCUAUCCUGGAUUACUUGAAUCC |
| MIMAT0000703 | MI0000760 | AAAGUACCCCUGGAGAUUCUGAUAAGCU |
| MIMAT0000715 | MI0000772 | CUCCUACUAAAACAUGGAAGCACUUACU |
| MIMAT0000714 | MI0000772 | CACAGAAAGCACUUCCAUGUUAAAGUUG |
| MIMAT0000717 | MI0000773 | CCUCCACUGAAACAUGGAAGCACUUACU |
| MIMAT0000716 | MI0000773 | ACACAGCAGGUACCCCCAUGUUAAAGCA |
| MIMAT0000718 | MI0000774 | ACCACACUCAAACAUGGAAGCACUUAUU |
| MIMAT0000719 | MI0000775 | CCAUCACCAUUGCUAAAGUGCAAUUCCA |
| MIMAT0000720 | MI0000776 | UGAAAACGUGGAAUUUCCUCUAUGUUUA |
| MIMAT0000721 | MI0000777 | GAGAAAAGAUCAACCAUGUAUUAUUCGA |
| MIMAT0001621 | MI0000777 | AAAGCGAAUAUAACACGGUCGAUCUCCC |
| MIMAT0000722 | MI0000778 | CAGACCAGGUUCCACCCCAGCAGGCACU |
| MIMAT0000723 | MI0000779 | GGUAACACUCAAAAGAUGGCGGCACUUU |
| MIMAT0000724 | MI0000780 | GUGACGCUCAAAUGUCGCAGCACUUUCC |
| MIMAT0000726 | MI0000781 | GGGACACCCCAAAAUCGAAGCACUUCCC |
| MIMAT0000725 | MI0000781 | AAAGGAAAGCGCCCCCAUUUUGAGUAUC |
| MIMAT0000727 | MI0000782 | UAACACUUAUCAGGUUGUAUUAUAAUGG |
| MIMAT0000728 | MI0000783 | GCCUCACGCGAGCCGAACGAACAAAACG |
| MIMAT0000729 | MI0000784 | GAAAACGUGGAUUUUCCUCUAUGAUUAA |
| MIMAT0003386 | MI0000784 | UGUACUCAUAGAAGGAGAAUCUACCUUU |
| MIMAT0000730 | MI0000785 | CAAACAAAAGUUGCCUUUGUGUGAUUCA |
| MIMAT0000732 | MI0000786 | UCAGGCCUUCUGACUCCAAGUCCAGUGC |
| MIMAT0000731 | M10000786 | AACACACAGGACCUGGAGUCAGGAGCCC |
| MIMAT0000733 | MI0000787 | ACGCCUACGUUCCAUAGUCUACCAUCUC |
| MIMAT0000735 | MI0000788 | GAGAAGAUGUGGACCAUAUUACAUACGA |
| MIMAT0000734 | MI0000788 | AUAGCGCAUGUUCUAUGGUCAACCAUCU |
| MIMAT0000736 | MI0000789 | CUCACAGAGAGCUUGCCCUUGUAUAUUC |
| MIMAT0000737 | MI0000790 | AAGCGAAUCCACCACGAACAACUUCUCU |
| MIMAT0000738 | MI0000791 | CAAAGCCACAAUCACCUUCUGAUCUGAG |
| MIMAT0000750 | MI0000802 | UAUGGCUAUAAAGUAACUGAGACGGAUC |
| MIMAT0000751 | MI0000803 | GCCUCUCUGCAGGCCGUGUGCUUUGCUC |
| MIMAT0000752 | MI0000804 | GGGACGGAAGGGCAGAGAGGGCCAGGGG |
| MIMAT0000753 | MI0000805 | GUGACGGGUGCGAUUUCUGUGUGAGACA |
| MIMAT0000754 | MI0000806 | AAGAAAGGCAUCAUAUAGGAGCUGGAUA |
| MIMAT0000755 | MI0000807 | CAAAGAGGUCGACCGUGUAAUGUGCGCC |
| MIMAT0000756 | MI0000808 | GGGGCUGGAGGAAGGGCCCAGAGGCGAU |
| MIMAT0000757 | MI0000809 | UGUCCUCAAGGAGCUUCAGUCUAGUAGG |
| M1MAT0000758 | MI0000810 | AAUCACAUAGGAAUGAAAAGCCAUAGGC |
| MIMAT0000759 | MI0000811 | GAGACAAAGUUCUGUGAUGCACUGACUU |
| MIMAT0000760 | MI0000812 | UUGGUUCUAGGAUAGGCCCAGGGGCCUG |
| MIMAT0000762 | MI0000813 | CCCCCAGCAGCACCUGGGGCAGUGGGUC |
| MIMAT0000761 | MI0000813 | UUUACACCAAUGCCCUAGGGGAUGCGGG |
| MIMAT0000763 | MI0000814 | UCUUCAACAAAAUCACUGAUGCUGGAGU |
| MIMAT0000764 | MI0000815 | CACGUGAGCUCCUGGAGGAGAGGGAGAG |
| MIMAT0000765 | MI0000816 | CAAACAUUUUUCGUUAUUGCUCUUGACC |
| MIMAT0000770 | MI0000822 | GCUGUAGCUGGUUGAAGGGGACCAAACC |
| MIMAT0000771 | MI0000824 | CAAACACUUACUGGACACCUACUAGGAA |
| MIMAT0000772 | MI0000825 | ACGAGCCCUGGACUAGGAGUCAGCAGAC |
| MIMAT0000773 | MI0000826 | CAGAGAGGCAGGCAUGCGGGCAGACAGA |
| MIMAT0001075 | MI0001145 | GCAUUAUGAACAAUUUCUAGGAAUGACU |
| MIMAT0001080 | MI0001150 | GAUCCCAACAACAGGAAACUACCUAAAU |
| MIMAT0001339 | MI0001444 | UCAGGCCUUCUGACCCUAAGUCCAGUGC |
| MIMAT0001340 | MI0001445 | AGACUGAGGGGCCUCAGACCGAGCUUUU |
| MIMAT0001341 | MI0001446 | CACUUCAAAACAUGAAUUGCUGCUGUAU |
| MIMAT0001343 | MI0001448 | ACUGGGCGGACACGACAUUCCCGAUGGC |
| MIMAT0000710 | MI0000767 | GCAAUAAGGAUUUUUAGGGGCAUUAUGA |
| MIMAT0000710 | MI0000769 | ACAAUAAGGAUUUUUAGGGGCAUUAUGA |
| MIMAT0001532 | MI0001637 | GAGAUGGGACAUCCUACAUAUGCAACCA |
| MIMAT0001536 | MI0001641 | UGGACGGUUUUACCAGACAGUAUUAGAC |
| MIMAT0001541 | MI0001648 | UCAACCAGCUAACAAUACACUGCCAGCU |
| MIMAT0001545 | MI0001652 | GCAUAUUAGGAACACAUCGCAAAAACAG |
| MIMAT0000096 | MI0000100 | CACAACAAUACAACUUACUACCUCACCC |
| MIMAT0000705 | MI0000762 | GCACUCACACCUAGGUUCCAAGGAUUCA |
| MIMAT0000707 | MI0000764 | GUUUACAGAUGGAUACCGUGCAAUUUUU |
| MIMAT0003385 | MI0000764 | UCAAAAUUGCAUCGUGAUCCACCCGACA |
| MIMAT0001412 | MI0001518 | CACUAACUGCACUAGAUGCACCUUAACA |
| MIMAT0001413 | MI0001519 | AAACUACCUGCACUAUGAGCACUUUGGU |
| MIMAT0001625 | MI0001721 | GGCCUGCAUGACGGCCUGCAAGACACCU |
| MIMAT0001627 | MI0001723 | AGAACACCGAGGAGCCCAUCAUGAUCCU |
| MIMAT0001629 | MI0001725 | GGAAAAGAGGUUAACCAGGUGUGUUUCG |
| MIMAT0001629 | MI0001726 | GGAAAAGAGGUUAACCAGGUGUGUUUCG |
| MIMAT0001630 | MI0001727 | AUGCGAACUCACCACGGACAACCUCCCU |
| MIMAT0001635 | MI0001733 | AAAGUCUCAGUUUCCUCUGCAAACAGUU |
| MIMAT0001636 | M10001733 | CUUACUUCUUUGCAGAUGAGACUGAGAC |
| MIMAT0001638 | M10001735 | AGAUGCAAAGUUGCUCGGGUAACCUCUC |
| MIMAT0001639 | MI0001735 | AAAAGGGGUUCACCGAGCAACAUUCGUC |
| MIMAT0002170 | MI0002464 | CGGACGGCUAGUGGACCAGGUGAAGUAC |
| MIMAT0002171 | MI0002465 | GAAAACAGGCCAUCUGUGUUAUAUUCGU |
| MIMAT0002172 | MI0002466 | GAAAACAUGGAUUUUCCUCUAUGAUUAA |
| MIMAT0002175 | MI0002469 | AUCGAAUUCAUCACGGCCAGCCUCUCUC |
| MIMAT0002176 | MI0002469 | AAAAGAGAGGAGAGCCGUGUAUGACUCG |
| MIMAT0002804 | MI0003123 | UUGUUUGAGAGUGCCAUUAUCUGGGAGA |
| MIMAT0002805 | MI0003124 | UUAGCUGCCGUAUAUGUGAUGUCACUCC |
| MIMAT0002806 | MI0003125 | CAACAGCAUGGAGUCCUCCAGGUUGGUG |
| MIMAT0002807 | MI0003126 | UACUCCUCAUGGAAGGGUUCCCCACUAC |
| MIMAT0002808 | MI0003127 | UUACUGACUGCAGAGCAAAAGACACGAU |
| MIMAT0002808 | MI0003128 | UUACUGACUGCAGAGCAAAAGACACGAU |
| MIMAT0002809 | MI0003129 | CACAGCCUAUGGAAUUCAGUUCUCAGUG |
| MIMAT0002811 | MI0003130 | CCGUUUUCCCAUGCCCUAUACCUCUUUA |
| MIMAT0002810 | MI0003130 | CUCAAAGAAGUAUAUGCAUAGGAAAAAG |
| MIMAT0002812 | MI0003131 | ACCAAGAAUCUUGUCCCGCAGGUCCUCG |
| MIMAT0002813 | MI0003132 | AUGAAUGAAAGCCUACCAUGUACAAAGC |
| MIMAT0003161 | MI0003132 | GGGCCUGGCACACAGUAGACCUUCACCG |
| MIMAT0002814 | MI0003133 | GAUCCACCCAAUGACCUACUCCAAGACC |
| MIMAT0002815 | MI0003133 | UCCAAGACAUGGAGGAGCCAUCCAGUGG |
| MIMAT0002816 | MI0003134 | AAAAGAGGUUUCCCGUGUAUGUUUCAUC |
| MIMAT0002817 | MI0003135 | GAAAAAGAAGUGCACCAUGUUUGUUUCG |
| MIMAT0002818 | MI0003136 | CGAAAGGAGAUUGGCCAUGUAAUACUCA |
| MIMAT0002819 | MI0003I37 | CAAAAGCGGGACUUUGAGGGCCAGUUGG |
| MIMAT0002820 | MI0003138 | CCGUACAAACCACAGUGUGCUGCUGGGG |
| MIMAT0002821 | MI0003139 | ACAACCCACCGACAACAAUGAAUGUUGA |
| MIMAT0002822 | MI0003140 | CCAGAAAGUGCCCUCAAGGCUGAGUGCC |
| MIMAT0002823 | MI0003140 | UUGGACCUCAGCUAUGACAGCACUUUCA |
| MIMAT0002822 | MI0003141 | CCAGAAAGUGCCCUCAAGGCUGAGUGCC |
| MIMAT0002823 | MI0003141 | UUGGACCUCAGCUAUGACAGCACUUUCA |
| MIMAT0002824 | MI0003142 | AUAGAAAAACGCCCCCUGGCUUGAAAUC |
| MIMAT0002825 | MI0003143 | GUAACCCUCAAAAAGGAAGCACUUUCUU |
| MIMAT0002826 | MI0003144 | AACAGAAAGUGCUUUCUUUUGGAGAAUG |
| MIMAT0002827 | MI0003144 | AGUAACGCUCCAAAAGAAGGCACUCUGC |
| MIMAT0002828 | MI0003145 | ACAGAAAGUGCUCCCUUUUGGAGAAUGA |
| MIMAT0002829 | MI0003145 | GUAACACUCUAAAAGGAGGCACUUUGUU |
| MIMAT0002830 | MI0003146 | GGUAACCCUCUAAAAGGAAGCACUUGCU |
| MIMAT0002826 | MI0003147 | AACAGAAAGUGCUUUCUUUUGGAGAAUG |
| MIMAT0002827 | MI0003147 | AGUAACGCUCCAAAAGAAGGCACUCUGC |
| MIMAT0002832 | MI0003148 | GUAAUCCUCUAAAAAGAUGCACUUUCUU |
| MIMAT0002831 | MI0003148 | ACAACAGAAAGCGCUUCCCUCUAGAGGG |
| MIMAT0002834 | MI0003149 | GAAACAGUCCAAAGGGAAGCACUUUCUU |
| MIMAT0002833 | MI0003149 | CAACAGAAAGUACUUCCCUCUGGAGGGU |
| MIMAT0002836 | MI0003150 | GUAAGCCUCUAAAAGGAAGCACUUUCUC |
| MIMAT0002835 | MI0003150 | ACAACAGAAAGUGCUUCCCUCAAGAGGG |
| MIMAT0002837 | MI0003151 | AGUAAACCUCUAAAAGGAUGCACUUUCU |
| MIMAT0002831 | MI0003151 | ACAACAGAAAGCGCUUCCCUCUAGAGGG |
| MIMAT0002838 | MI0003152 | UAAGAGAAAGUGCAUCCCUCUGGAGAGU |
| MIMAT0002839 | MI0003152 | UAACGCUCUAAAGGGAAGCGCCUUCUUU |
| MIMAT0002840 | MI0003153 | GUAACCCUCUAUAGGGAAGCGCGUUCUU |
| MIMAT0002831 | MI0003153 | ACAACAGAAAGCGCUUCCCUCUAGAGGG |
| MIMAT0002841 | MI0003154 | ACAAGAGAAAGUGCUUCCCUCUAGAGGG |
| MIMAT0002842 | MI0003154 | GUAAUCCUCUAAAGAGAAGCGCUUUCUU |
| MIMAT0002843 | MI0003155 | GUAACCCUCUAAAAGGAAGCACUUUCUU |
| MIMAT0002844 | MI0003156 | UAAACCUCUAAAGGGGAGCGCUUUGUUU |
| MIMAT0002845 | MI0003157 | CAACAGAAAGUGCUUCCCUCUAGAGGGU |
| MIMAT0002846 | MI0003158 | GGUAACCCUCUAAAAGGAAGCACUUUCU |
| MIMAT0002845 | MI0003158 | CAACAGAAAGUGCUUCCCUCUAGAGGAC |
| MIMAT0002847 | MI0003159 | CAACAGAAAGUGCUUCCCUCCAGAGAGU |
| MIMAT0002848 | MI0003159 | UAACACUCUAAAGAGAAGCGCUUUGUUU |
| MIMAT0002850 | MI0003160 | UAACACUCCAAAGGGAAGCGCCUUCUUU |
| MIMAT0002849 | MI0003160 | CAAGAGAAAGUGCUUCCCUUUGUAGGGU |
| MIMAT0002852 | MI0003161 | AGUAACACUCUAAAGGGAUGCACGAUCU |
| MIMAT0002851 | MI0003161 | AACAGACAGUGCUUCCAUCUAGAGGGUC |
| MIMAT0002853 | MI0003162 | GUAACACUCUAAAGGGAGGCACUUUGUU |
| MIMAT0002854 | MI0003163 | GUAACACUCUAAAGGGAAGUGCGUUCUU |
| MIMAT0002856 | MI0003164 | CGUAACCCACCAAAGAGAAGCACUUUCU |
| MIMAT0002855 | MI0003164 | CAACAGAAAGGGCUUCCCUUUGUAGACU |
| MIMAT0002857 | MI0003165 | AGUAACACUCUAAAGGGAUGCACGAUCU |
| MIMAT0002851 | MI0003165 | AACAGACAGUGCUUCCAUCUAGAGGGUC |
| MIMAT0002858 | MI0003166 | UAACACUCUAAAGGGAAGCACUUUGUUU |
| MIMAT0002860 | MI0003167 | GAGUAACCCUCUGAAAGGAAGCACUUUC |
| MIMAT0002859 | MI0003167 | CACAAAGUGCUUCUUACCUCCAGAUGGU |
| MIMAT0002845 | MI0003168 | CAACAGAAAGUGCUUCCCUCUAGAGGGU |
| MIMAT0002861 | MI0003169 | UUAACACUCUGAAGGGAAGCGCUUUCUU |
| MIMAT0002831 | MI0003169 | CCAACAGAAAGCGCUUCCCUCUAGAGGG |
| MIMAT0002862 | MI0003170 | CAACAGAAAGGGCUUCCCUUUGCAGUCA |
| MIMAT0002863 | MI0003170 | GUAAUCCAGCAAAGGGAAGCGCUUUCUC |
| MIMAT0002864 | MI0003171 | UAACGCUCCAAAGGGAAGCGCUUUGGUU |
| MIMAT0002845 | MI0003171 | CAACAGAAAGUGCUUCCCUCUAGAGGGU |
| MIMAT0002860 | MI0003172 | GAGUAACCCUCUGAAAGGAAGCACUUUC |
| MIMAT0002859 | MI0003172 | CAGAAAGUGCUUCUUACCUCCAGAUGGU |
| MIMAT0002865 | MI0003173 | ACAGAAAGGGCUUCCCUUUGCAGACCCA |
| MIMAT0002863 | MI0003173 | GUAAUCCAGCAAAGGGAAGCGCUUUCUC |
| MIMAT0002866 | MI0003174 | GUAACACUCUAAAAGGAUGCACGAUCUU |
| MIMAT0002851 | MI0003174 | AACAGACAGUGCUUCCAUCUAGAGGGUC |
| MIMAT0002867 | MI0003175 | GUAACUCUAAAGGGAAGCACUUUGUUUU |
| MIMAT0002854 | MI0003176 | GUAACACUCUAAAGGGAAGUGCGUUCUU |
| MIMAT0002868 | MI0003177 | CGUAACACUCUAAAGGGAACCAUUUUCU |
| MIMAT0002831 | MI0003177 | ACAACAGAAAGCGCUUCCCUCUAGAGGG |
| MIMAT0002869 | MI0003178 | CAGUAACACUCUAAAAGGAUGCACUUUC |
| MIMAT0002831 | MI0003178 | ACAACAGAAAGCGCUUCCCUCUAGAGUG |
| MIMAT0002862 | MI0003179 | CAACAGAAAGGGCUUCCCUUUGCAGUCA |
| MIMAT0002860 | MI0003180 | CCGUAACCCUCUGAAAGGAAGCACUUUC |
| MIMAT0002860 | MI0003181 | CCGUAACCCUCUGAAAGGAAGCACUUUC |
| MIMAT0002869 | MI0003182 | CAGUAACACUCUAAAAGGAUGCACUUUC |
| MIMAT0002870 | MI0003183 | GAGUUAAACAUCACUGCAAGUCUUAACA |
| MIMAT0002871 | MI0003184 | UCUCAGAAUCCUUGCCCAGGUGCAUUGC |
| MIMAT0002872 | MI0003185 | CACUCUCACCCAGGGACAAAGGAUUAGA |
| MIMAT0001545 | MI0003187 | ACAUAUUAGGAACACAUCGCAAAAAUAG |
| MIMAT0002874 | MI0003188 | UCACUGCAGAACUGUUCCCGCUGCUAGG |
| MIMAT0002875 | MI0003189 | ACAGAUAGAGUGCAGACCAGGGUCUCCC |
| MIMAT0002876 | MI0003190 | AGAGAGGAAACCAGCAAGUGUUGACGCU |
| MIMAT0002877 | MI0003191 | CACAUAAAUGACACCUCCCUGUGAAAGG |
| MIMAT0002877 | MI0003192 | CACAUAAAUGACACCUCCCUGUGAAAGG |
| MIMAT0002878 | MI0003193 | UUACUCUACUCAGAAGGGUGCCUUACAA |
| MIMAT0002879 | MI0003194 | UUAUUUCACUCCAAAAGGUGCAAAACAU |
| MIMAT0002880 | MI0003195 | UACUCUACUCCAAAAGGCUACAAUCAUG |
| MIMAT0002881 | MI0003196 | UACUCUACCCACAGACGUACCAAUCAUU |
| MIMAT0002882 | MI0003197 | ACAUGUGAUUGCCACUCUCCUGAGUAGG |
| MIMAT0002883 | MI0003198 | UACUCUACUCACAGAAGUGUCAAUCAAA |
| MIMAT0002883 | MI0003199 | UACUCUACUCACAGAAGUGUCAAUCAAA |
| MIMAT0002883 | MI0003200 | UACUCUACUCACAGAAGUGUCAAUCAAA |
| MIMAT0002173 | MI0002467 | AGGAGAAGACGGGAGGAGAGGAGUGAGG |
| MIMAT0002177 | MI0002470 | CAGCUCGGGGCAGCUCAGUACAGGAUAC |
| MIMAT0002178 | MI0002471 | AAAAACUGGAUGUCCCUGUAUGAUUCGU |
| MIMAT0003150 | MI0003513 | CCACGAUGUAGUCCAAAGGCACAUACCC |
| MIMAT0002174 | MI0002468 | UUUAUCGGGAGGGGACUGAGCCUGACGA |
| MIMAT0002873 | MI0003186 | GCACUAGCACCCAGAUAGCAAGGAUUAG |
| MIMAT0003163 | MI0003514 | CGAACACACCAAGGAUAAUUUCUCCUCA |
| MIMAT0003164 | M10003515 | GAGAACUUGCUAAAAAUGCAGAAUCUUG |
| MIMAT0003165 | MI0003516 | AGGCACACAAUAAAUGUUUGCUGAUGAG |
| MIMAT0000729 | MI0003529 | GAAAACGUGGAUUUUCCUCUAUGAUUAA |
| MIMAT0003180 | MI0003530 | AAAAAGUGGAUGACCCUGUACGAUUCGA |
| MIMAT0003389 | MI0003686 | ACCUUUCAGUUAUCAAUCUGUCACAAGU |
| MIMAT0003340 | MI0003686 | UAUCUCGUGACAUGAUGAUCCCCGAGAU |
| MIMAT0000460 | MI0000732 | ACUUCCACAUGGAGUUGCUGUUACAGGG |
| MIMAT0001631 | MI0001729 | ACUAAACUCAGUAAUGGUAACGGUUUCC |

| **Mature Accession** | **Precursor Accession** | **Sequences of Inhibitors Targeting Mouse miRNAs, (SEQ ID NOS 414-714)** |
|---|---|---|
| MIMAT0000513 | MI0000718 | CAGUCAGUUUUGCAUGGAUUUGCACAGC |
| MIMAT0000539 | MI0000719 | UCAACAGGCCGGGACAAGUGCAAUACCA |
| MIMAT0000150 | MI0000722 | CGGCCUGAUUCACAACACCAGCUGUCCC |
| MIMAT0000375 | MI0000395 | UGCACAUGCACAUGCACACAUACAUACA |
| MIMAT0003120 | MI0003484 | AGAACAAGACGGGAGGGGAGGAGUGAGG |
| MIMAT0003128 | MI0003492 | AUCGAAUUCAUCACGGCCAGCCUCUCUC |
| MIMAT0003129 | MI0003492 | GAAGAGAGGAGAGCCGUGUAUGACUCGU |
| MIMAT0000769 | MI0000821 | GCUGUAGCUGGUUGAAGGGGACCAAACC |
| MIMAT0000211 | MI0000224 | CGGUGUGAGUUCUACCAUUGCCAAAAAU |
| MIMAT0000210 | MI0000223 | CAAACUCACCGACAGCGUUGAAUGUUCC |
| MIMAT0000213 | MI0000226 | CCUACCCUUAUCAGUUCUCCGUCCAACA |
| MIMAT0000214 | MI0000227 | AUCAGGAACUGCCUUUCUCUCCAAUCCC |
| MIMAT0000215 | MI0000228 | AGAAAGCCCAAAAGGAGAAUUCUUUGGA |
| MIMAT0001537 | MI0001642 | UGGACGGCAUUACCAGACAGUAUUAGAC |
| MIMAT0000217 | MI0000230 | CUCACCCUCCACCAUGCAAGGGAUGUGA |
| MIMAT0001342 | MI0001447 | ACUGGGCGGACACGACAUUCCCGAUGGC |
| MIMAT0001081 | MI0001151 | GAUCCCAACAACAGGAAACUACCUAAAU |
| MIMAT0000133 | MI0000148 | AUCCUUCAGUUAUCACAGUACUGUACCU |
| MIMAT0000366 | MI0000388 | AAAAAAAAGUGCCCCCAUAGUUUGAGUA |
| MIMAT0000367 | MI0000389 | UCAAGAGAGGGCCUCCACUUUGAUGGCC |
| MIMAT0000368 | MI0000389 | AGUGGCACACAAAGUGGAAGCACUUUCU |
| MIMAT0000374 | MI0000394 | CACAACAGGAUUGAGGGGGGGCCCUCCA |
| MIMAT0000372 | MI0000392 | GCAACACACAAAAGGGAAGCACUUUCCA |
| MIMAT0000373 | MI0000393 | GAGAGACUCAAAAGUAGUAGCACUUUCU |
| MIMAT0000376 | MI0000398 | AAGGGAAGAACAGCCCUCCUCUGCCAAA |
| MIMAT0000377 | MI0000399 | AAAAUGUAUGUGGGACGGUAAACCAUUU |
| MIMAT0000382 | MI0000404 | CUACAAUCAGCUAAUUACACUGCCUACA |
| MIMAT0000381 | MI0000403 | UUAGCAAUCAGCUAACUACACUGCCUAG |
| MIMAT0000542 | MI0000584 | CACAACAACCAGCUAAGACACUGCCAAA |
| MIMAT0000246 | MI0000256 | GACACAAACACCAUUGUCACACUCCACA |
| MIMAT0000131 | MI0000146 | CACCACAAGAUCGGAUCUACGGGUUUAU |
| MIMAT0000524 | MI0000561 | CUCAACUAUACAACCUCCUACCUCAGCC |
| MIMAT0000384 | MI0000405 | CUAAGAAAGGCAGCAGGUCGUAUAGUUA |
| MIMAT0000383 | MI0000405 | UAAAACUAUGCAACCUACUACCUCUUCC |
| MIMAT0000121 | MI0000137 | UCAAACUGUACAAACUACUACCUCAGCC |
| MIMAT0001092 | MI0001162 | GAAAAAGUGGAUGUUCCUCUAUGAUUAU |
| MIMAT0003388 | MI0001162 | ACGUAACCAUAGAAGGAAUAUCCACCUU |
| MIMAT0000740 | MI0000793 | GAAAACGUGGAUUUUCCUGUACGAUUAG |
| MIMAT0003387 | MI0000793 | UGUACUGAUAGAAGGAGAAUCUACCUUU |
| MIMAT0000145 | MI0000159 | GCUACAGCUGGUUGAAGGGGACCAAAUC |
| MIMAT0000522 | MI0000558 | UGAAACCACACAACCUACUACCUCACCC |
| MIMAT0000122 | MI0000138 | ACCAACAGCACAAACUACUACCUCAGCC |
| MIMAT0000247 | MI0000257 | UCCUGAGCUACAGUGCUUCAUCUCAGAC |
| MIMAT0000747 | MI0000799 | AAGCGAAUCCACCACGAACAACUUCUCU |
| MIMAT0000748 | MI0000800 | CAAAGCCACAGUCACCUUCUGAUCUGAG |
| MIMAT0000744 | MI0000797 | GUAGCGCAUGUUCUAUGGUCAACCAUCU |
| MIMAT0000745 | MI0000797 | GAGAAGAUGUGGACCAUACUACAUACGA |
| MIMAT0000746 | MI0000798 | CUCACAGAGAGCUUGCCCUUGUAUAUUC |
| MIMAT0000141 | MI0000156 | CCGAUGCCCUUUUAACAUUGCACUGCUC |
| MIMAT0001076 | MI0001146 | GCAUUGUGAACAAUUUCUAGGAAUGACU |
| MIMAT0000513 | MI0000546 | CAAUCAGUUUUGCAUGGAUUUCCACAGC |
| MIMAT0000546 | MI0000587 | CCUUCAUAGCCCUGUACAAUGCUGCUUG |
| MIMAT0000123 | MI0000139 | GAAAUACAUACUUCUUUACAUUCCAUAG |
| MIMAT0000123 | MI0000652 | AAAAUACAUACUUCUUUACAUUCCAUAG |
| MIMAT0000660 | MI0000697 | UAGGGUACAAUCAACGGUCGAUGGUUUU |
| MIMAT0000210 | MI0000697 | CAAACUCACCGACAGCGUUGAAUGUUCA |
| MIMAT0000616 | MI0000649 | AUUCUUCAGCUAUCACAGUACUGUACCU |
| MIMAT0000663 | MI0000701 | CACCACAUGGUUAGAUCAAGCACAAAGG |
| MIMAT0000663 | MI0000700 | AAGCACAUGGUUAGAUCAAGCACAACAG |
| MIMAT0000677 | MI0000729 | CAACAACAAAAUCACUAGUCUUCCAAAC |
| MIMAT0000677 | MI0000728 | AAACAACAAAAUCACUAGUCUUCCACAC |
| MIMAT0000673 | MI0000823 | AAACCCACCGACAGCAAUGAAUGUUGAG |
| MIMAT0000664 | MI0000741 | UACAAGAAUUGCGUUUGGACAAUCAGUG |
| MIMAT0000147 | MI0000715 | CGAUCACAUAGGAAUAAAAAGCCAUAAA |
| MIMAT0000673 | MI0000723 | CAACCCACCGACAGCAAUGAAUGUUGAU |
| MIMAT0000664 | MI0000702 | CUCGAGAAUUGCGUUUGGACAAUCAGGA |
| MIMAT0000147 | MI0000161 | GAAUCACAUAGGAAUAAAAAGCCAUAGA |
| MIMAT0000528 | MI0000567 | CACUAUCUGCACUAGAUGCACCUUAGAA |
| MIMAT0000230 | MI0000241 | CCUAACCAAUGUGCAGACUACUGUACAU |
| MIMAT0000229 | MI0000241 | CCUGAACAGGUAGUCUGAACACUGGGAU |
| MIMAT0000230 | MI0000713 | UCUAACCAAUGUGCAGACUACUGUACAA |
| MIMAT0000229 | MI0000713 | CCUGAACAGGUAGUCUGAACACUGGGGC |
| MIMAT0000674 | MI0000724 | CAAACUCACCGACAGGUUGAAUGUUCCC |
| MIMAT0000649 | MI0000687 | UCACUACCUGCACUGUAAGCACUUUGAC |
| MIMAT0000650 | MI0000687 | AUGCUACAAGUGCCCUCACUGCAGUAGA |
| MIMAT0000657 | MI0000694 | CCUCCAUCAUUACCCGGCAGUAUUAGAG |
| MIMAT0000233 | MI0000243 | GCCGUCAUCAUUACCAGGCAGUAUUAGA |
| MIMAT0000519 | MI0000554 | UGAACAUCGUUACCAGACAGUGUUAGAG |
| MIMAT0000653 | MI0000690 | CAACUCAAUAGACUGUGAGCUCCUUGAA |
| MIMAT0000531 | MI0000570 | GCAACAGUUCUUCAACUGGCAGCUUUAG |
| MIMAT0000530 | MI0000569 | CAGUCAACAUCAGUCUGAUAAGCUAUCC |
| MIMAT0000529 | MI0000568 | ACACUACCUGCACUAUAAGCACUUUAGU |
| MIMAT0000145 | MI0000820 | GCUACAGCUGGUUGAAGGGGACCAAAUC |
| MIMAT0000652 | MI0000689 | CUGUCAGACCGAGACAAGUGCAAUGCCC |
| MIMAT0001546 | MI0001653 | ACAUAUUAGGAACACAUCGCAAAAACAG |
| MIMAT0000904 | MI0000974 | CACGGUCUGUCAAAUCAUAGGUCAUUCU |
| MIMAT0000558 | MI0000597 | CAAACACUUACUGAGCACCUACUAGGAA |
| MIMAT0000516 | MI0000550 | GAGACAAAGUUCUGUAGUGCACUGACUU |
| MIMAT0000532 | MI0000571 | AGUUGGAAAUCCCUGGCAAUGUGAUUUG |
| MIMAT0000766 | MI0000817 | CAAACAUUUUUCGUUAUUGCUCUUGACC |
| MIMAT0000518 | MI0000553 | AAUCCCAACAACAUGAAACUACCUAAGC |
| MIMAT0000518 | MI0000552 | AGGCCCAACAACAUGAAACUACCUACUU |
| MIMAT0000521 | MI0000556 | UAAAACUAUACAACCUACUACCUCAUCC |
| MIMAT0000521 | MI0000557 | CUAAACUAUACAACCUACUACCUCAACC |
| MIMAT0000654 | MI0000691 | ACAUGCAACUUAGUAAUGUGCAAUAUCU |
| MIMAT0000667 | MI0000707 | ACAUGCAAUGCAACUACAAUGCACCACA |
| MIMAT0000538 | MI0000579 | CAACAGCUAUGCCAGCAUCUUGCCUCCU |
| MIMAT0000711 | MI0000768 | GCAAUAAGGAUUUUUAGGGGCAUUAUGA |
| MIMAT0000711 | MI0001645 | ACAAUAAGGAUUUUUAGGGGCAUUAUGA |
| MIMAT0000665 | MI0000703 | ACUUGGGGUAUUUGACAAACUGACACUC |
| MIMAT0000670 | MI0000710 | CAGAGACCCAGUAGCCAGAUGUAGCUGC |
| MIMAT0000135 | MI0000151 | CCUCACAGGUUAAAGGGUCUCAGGGACC |
| MIMAT0000565 | MI0000603 | GGGACGGAAGGGCAGAGAGGGCCAGGGG |
| MIMAT0001632 | MI0001730 | CUAAACUCAGUAAUGGUAACGGUUUCCU |
| MIMAT0001637 | MI0001734 | AAAGUCUCAGUUUCCUCUGCAAACAGUU |
| MIMAT0000136 | MI0000725 | ACAUCACAAGUUAGGGUCUCAGGGACUG |
| MIMAT0000136 | MI0000152 | ACCUCACAAGUUAGGGUCUCAGGGACUA |
| MIMAT0000666 | MI0000704 | UUUUUCGCCCUCUCAACCCAGCUUUUCC |
| MIMAT0000584 | MI0000621 | UACGUGAGCUCCUGGAGGACAGGGAUAG |
| MIMAT0000582 | MI0000619 | UCUUCAACAAAAUCACUGAUGCUGGAGU |
| MIMAT0000555 | MIOO00595 | UUACACCAAUGCCCUAGGGGAUGCGAGG |
| MIMAT0000556 | MI0000595 | CCCCCAGCAGCACCUGGGGCAGUGGGUC |
| MIMAT0000165 | MI0000177 | AAACCCCUAUCACAAUUAGCAUUAACAG |
| MIMAT0000223 | MI0000235 | AGGACUGGGACUUUGUAGGCCAGUUGAA |
| MIMAT0000571 | MI0000609 | UUGGUUCUAGGAUAGGCCCAGGGGCCUG |
| MIMAT0000569 | MI0000607 | ACCUCUCUGCAGGCCCUGUGCUUUGCUC |
| MIMAT0000655 | MI0000692 | CAGCACAAGUUCGGAUCUACGGGUUUGU |
| MIMAT0000578 | MI0000615 | AAGAAAGGCAUCAUAUAGGAGCUGAAUG |
| MIMAT0000647 | MI0000684 | CUUUGAUAGCCCUGUACAAUGCUGCUUG |
| MIMAT0000539 | MI0000580 | UCCUCAGGCCGGGACAAGUGCAAUACUU |
| MIMAT0000142 | MI0000720 | ACUCAUACAGCUAGAUAACCAAAGAUAA |
| MIMAT0000143 | MI0000720 | UUUUACUUUCGGUUAUCUAGCUUUAUGA |
| MIMAT0000659 | MI0000696 | CGGUGGCCGUGACUGGAGACUGUUACUG |
| MIMAT0000142 | MI0000721 | ACUCAUACAGCUAGAUAACCAAAGAGAG |
| MIMAT0000143 | MI0000721 | UUCUACUUUCGGUUAUCUAGCUUUAUGA |
| MIMAT0000658 | MI0000695 | AGAUCAGCCGCUGUCACACGCACAGUGG |
| MIMAT0000668 | MI0000708 | CCUAGGCAAAGGAUGACAAAGGGAAGCC |
| MIMAT0000662 | MI0000699 | AUCUCACAGUUGCCAGCUGAGAUUAAAC |
| MIMAT0000679 | MI0000731 | UUAUCCAGUCAGUUCCUGAUGCAGUAUC |
| MIMAT0000661 | MI0000698 | GUGACUGCCUGUCUGUGCCUGCUGUACA |
| MIMAT0000567 | MI0000605 | GAAAAAAAGGUUAGCUGGGUGUGUUUCA |
| MIMAT0000370 | MI0000390 | GUGACACUCAAAACCUGGCGGCACUUUU |
| MIMAT0000369 | MI0000390 | AUCCAAAAGAGCCCCCAGUUUGAGUAUC |
| MIMAT0000546 | MI0000588 | CUUUCAUAGCCCUGUACAAUGCUGCUUG |
| MIMAT0000157 | MI0000169 | CCAAGGGAUUCCUGGGAAAACUGGACCG |
| MIMAT0000656 | MI0000693 | ACUGGAGACACGUGCACUGUAGAAUACA |
| MIMAT0000151 | MI0000165 | AACCUACCAUAGGGUAAAACCACUGGCA |
| MIMAT0000152 | MI0000165 | CUGUCCGUGGUUCUACCCUGUGGUAGAA |
| MIMAT0000549 | MI0000590 | GGGGUGUUGCAGCGCUUCAUGUUUUGAA |
| MIMAT0000548 | MI0000590 | UACUCCAAAACAUGAAUUGCUGCUGCAG |
| MIMAT0000551 | MI0000592 | CAAAGAGGUCGACCGUGUAAUGUGCGCC |
| MIMAT0000125 | MI0000141 | GCGUGGUAAUCCCUGGCAAUGUGAUUUU |
| MIMAT0000144 | MI0000158 | GGGCGACCAUGGCUGUAGACUGUUACCU |
| MIMAT0000559 | MI0000598 | CGGGACUGGAGGAAGGGCCCAGAGGCGA |
| MIMAT0000149 | MI0000163 | CGACUACGCGUAUUCUUAAGCAAUAACA |
| MIMAT0000148 | MI0000162 | GAAUCCAUCAUCAAAACAAAUGGAGUCC |
| MIMAT0000236 | MI0000246 | GGGUCUAGUGGUCCUAAACAUUUCACAA |
| MIMAT0000146 | MI0000160 | ACGCCCCUCUGGUCAACCAGUCACACAC |
| MIMAT0000234 | MI0000244 | UAGAAGAACAAUGCCUUACUGAGUAAGG |
| MIMAT0002111 | MI0002405 | UAACUCACCAGUGCCAGUCCAAGAAGAG |
| MIMAT0002112 | MI0002406 | UAAUGUGAAAAGCACUAUACUACGUAAA |
| MIMAT0000669 | MI0000709 | CUGAAACCCAGCAGACAAUGUAGCUGUU |
| MIMAT0000134 | MI0000717 | UCUUGGCAUUCACCGCGUGCCUUAAUUG |
| MIMAT0000134 | MI0000150 | UCUUGGCAUUCACCGCGUGCCUUAAUUG |
| MIMAT0000127 | MI0000143 | AAGAACACUGAUUUCAAAUGGUGCUAGA |
| MIMAT0000134 | MI0000716 | UCUUGGCAUUCACCGCGUGCCUUAAUUG |
| MIMAT0000671 | MI0000711 | UACUAAACGGAACCACUAGUGACUUAAA |
| MIMAT0000127 | MI0000712 | UAAAACACUGAUUUCAAAUGGUGCUAGA |
| MIMAT0000523 | MI0000560 | CAAAACCAUACAACCUACUACCUCACCC |
| MIMAT0000523 | MI0000559 | CUAAACCAUACAACCUACUACCUCAACC |
| MIMAT0000612 | MI0000646 | AAUCACAUAGGAAUGAAAAGCCAUAGGC |
| MIMAT0000130 | MI0000145 | GACAGCUGAGUGUAGGAUGUUUACAUGA |
| MIMAT0000533 | MI0000573 | CACAGCCUAUCCUGGAUUACUUGAACGA |
| MIMAT0000534 | MI0000575 | CACAACCUAUCCUGAAUUACUUGAACUG |
| MIMAT0000533 | MI0000706 | CACAGCCUAUCCUGGAUUACUUGAAUCC |
| MIMAT0001542 | MI0001649 | UCAACCAGCUAACAAUACACUGCCAAGC |
| MIMAT0001533 | MI0001638 | GAGAUGGGACAUCCUACAUAUGCAACCA |
| MIMAT0000137 | MI0000153 | ACAGCGCGUACCAAAAGUAAUAAUGUGC |
| MIMAT0000138 | MI0000153 | CCGCGCAUUAUUACUCACGGUACGAGUU |
| MIMAT0000139 | MI0000154 | ACCAGCCAAGCUCAGACGGAUCCGAUGA |
| MIMAT0000651 | MI0000688 | CCAUCAGUUUUGCAUAGAUUUGCACAAC |
| MIMAT0000590 | MI0000627 | GUGACGGGUGCGAUUUCUGUGUGAGACA |
| MIMAT0000648 | MI0000685 | UUACACAAAUUCGGAUCUACAGGGUAUA |
| MIMAT0000208 | MI0000221 | ACCACACAAAUUCGGUUCUACAGGGUAU |
| MIMAT0000219 | MI0000572 | CUCCUGUUCCUGCUGAACUGAGCCAGUG |
| MIMAT0000708 | MI0000765 | GGUUUACAGAUGGAUACCGUGCAAUUUU |
| MIMAT0000132 | MI0000147 | CCCCGCAAGGUCGGUUCUACGGGUGGGU |
| MIMAT0000380 | MI0000402 | CCAUCACCAAAACAUGGAAGCACUUACU |
| MIMAT0000378 | MI0000400 | CUCGAAGAGAGCUUGCCCUUGCAUAUUC |
| MIMAT0000379 | MI0000401 | GAUGCUUUGACAAUACUAUUGCACUGCU |
| MIMAT0001091 | MI0001161 | GAAAACAGGCCAUCUGUGUUAUAUUCGU |
| MIMAT0000164 | MI0000176 | AAGCGAAGGCAACACGGAUAACCUAUCU |
| MIMAT0000161 | MI0000173 | CUGUCCUCAAGGAGCCUCAGUCUAGUAG |
| MIMAT0000160 | MI0000172 | CAGCACUGGUACAAGGGUUGGGAGACAG |
| MIMAT0000163 | MI0000175 | AAUGAUCACUUUUGUGACUAUGCAACUG |
| MIMAT0000162 | MI0000174 | GGGCCCAAGUUCUGUCAUGCACUGACUG |
| MIMAT0001093 | MI0001163 | GAUACUGAGGGUUAGUGGACCGUGUUAC |
| MIMAT0000520 | MI0000555 | CCAACAAGCUUUUUGCUCGUCUUAUACG |
| MIMAT0000371 | MI0000391 | GCAACACUACAAACUCUGCGGCACUUCU |
| MIMAT0000386 | MI0000407 | CACUAUCUGCACUGUCAGCACUUUAGUC |
| MIMAT0002110 | MI0002404 | AGGACACCAAGAUCAAUGAAAGAGGCAC |
| MIMAT0000220 | MI0000232 | ACAACCUAAUAUAUCAAACAUAUCACAC |
| MIMAT0000224 | MI0000236 | CAGUCCACAUGGAGUUGCUGUUACACCC |
| MIMAT0000235 | MI0000245 | CCAUCUUCCCAUGCGCUAUACCUCUUUA |
| MIMAT0001094 | MI0001164 | CCGACGGCUAGUGGACCAGGUGAAGUAC |
| MIMAT0000240 | MI0000250 | AGAGAGGGAGGAGAGCCAGGAGAAGCGC |
| MIMAT0000239 | MI0000249 | AAACCACACACUUCCUUACAUUCCAUAG |
| MIMAT0000238 | MI0000248 | AGACAGACUCCGGUGGAAUGAAGGACAA |
| MIMAT0000237 | MI0000247 | UCUCAGGCAUAGGAUGACAAAGGGAAGU |
| MIMAT0000743 | MI0000796 | AACGCCUACGUUCCAUAGUCUACCAUCU |
| MIMAT0000742 | MI0000795 | AACACACAGGACCUGGAGUCAGGAGCCC |
| MIMAT0003151 | MI0000795 | UCAGGCCUUCUGACUCCAAGUCCAGUGC |
| MIMAT0000739 | MI0000792 | GCCUCACGCGAGCCGAACGAACAAAACG |
| MIMAT0000159 | MI0000171 | ACGGGAGUGAAGACACGGAGCCAGAGCC |
| MIMAT0000741 | MI0000794 | CAAACAAAAGUUGCCUUUGUGUGAUUCA |
| MIMAT0002109 | MI0002403 | AGACAGACACACGCACAUCAGUCAUAUC |
| MIMAT0000156 | MI0000168 | AGACUAGUACAUCAUCUAUACUGUAGUG |
| MIMAT0001095 | MI0001165 | ACAAACCAGGUUCCACCCCAGCAGGCAC |
| MIMAT0000158 | MI0000170 | UAUAACCCAUGGAAUUCAGUUCUCAGAG |
| MIMAT0000675 | MI0000726 | AGGGAAAGAGACCGGUUCACUGUGAGAC |
| MIMAT0000153 | MI0000166 | GGGCCAUCUUUACCAGACAGUGUUAGGA |
| MIMAT0000154 | MI0000167 | GUUAGUAGUGCUUUCUACUUUAUGGGUG |
| MIMAT0000155 | MI0000167 | CAUCCAUAAAGUAGGAAACACUACACCC |
| MIMAT0000140 | MI0000155 | UGAAAAAGAGACCGGUUCACUGUGAGAA |
| MIMAT0002108 | MI0002402 | UGUGUGUAGGUGUGUGUAUGUAUAUGCA |
| MIMAT0000128 | MI0000144 | CAGCUUCCAGUCGAGGAUGUUUACAGUC |
| MIMAT0000129 | MI0000144 | GCAGCUGCAAACAUCCGACUGAAAGCCC |
| MIMAT0002104 | MI0002398 | UACAUGAUGGACAACAAAUUAGGUAAAG |
| MIMAT0000525 | MI0000563 | UAAAACUAUACAAUCUACUACCUCAUCC |
| MIMAT0000525 | MI0000562 | CACAACUAUACAAUCUACUACCUCACUC |
| MIMAT0002107 | MI0002401 | UGUGUCUUAUGUGUGCGUGUAUGUAUAU |
| MIMAT0002106 | MI0002400 | UUAUCACAUCAGUGCCAUUCUAAAUAGG |
| MIMAT0002105 | MI0002399 | GUCUAUCUCACAGAAUAAACUUGGUAGU |
| MIMAT0000678 | MI0000730 | GAACAACAAAAUCACAAGUCUUCCACAU |
| MIMAT0000216 | MI0000229 | CCUCCGGCUGCAACACAAGACACGAGGG |
| MIMAT0000142 | MI0000157 | ACUCAUACAGCUAGAUAACCAAAGAUAA |
| MIMAT0000143 | MI0000157 | UUUUACUUUCGGUUAUCUAGCUUUAUGA |
| MIMAT0000704 | MI0000761 | UAAGUACCCCUGGAGAUUCUGAUAAGCU |
| MIMAT0000540 | MI0000581 | ACACUACCUGCACGAACAGCACUUUGGA |
| MIMAT0000537 | MI0000578 | GGGGGCGGAACUUAGCCACUGUGAACAC |
| MIMAT0000126 | MI0000142 | AGGUGCAGAACUUAGCCACUGUGAACAA |
| MIMAT0000541 | MI0000583 | ACAAGCAAAAAUGUGCUAGUGCCAAAAU |
| MIMAT0000545 | MI0000586 | CACAACAAUACAACUUACUACCUCACCC |
| MIMAT0000514 | MI0000547 | ACAGCUGAGAGUGUAGGAUGUUUACACA |
| MIMAT0000514 | MI0000548 | ACAGCUGAGAGUGUAGGAUGUUUACAAU |
| MIMAT0001422 | MI0001526 | ACCAGGAGUCGAGUGAUGGUUCAAACCA |
| MIMAT0001421 | MI0001526 | AAUGGUUCAAACCAUGAGUCGAGCUUUG |
| MIMAT0001420 | MI0001525 | AGAACACCGAGGAGCCCAUCAUGAUCCU |
| MIMAT0001419 | MI0001525 | UCUGAAUAAUGACAGGCUCACCGUACUU |
| MIMAT0001418 | MI0001524 | UGGCCUGCAUGACGGCCUGCAAGACACC |
| MIMAT0000515 | MI0000549 | CAGCUUCCAGUCGGGGAUGUUUACAGAC |
| MIMAT0000248 | MI0000259 | AGCUUCCAGUCAAGGAUGUUUACAGUAG |
| MIMAT0000249 | MI0000259 | GCCGCUGUAAACAUCCGACUGAAAGCUC |
| MIMAT0000526 | MI0000564 | AUCCACAAACCAUUAUGUGCUGCUACUU |
| MIMAT0000124 | MI0000140 | GUAUGUAAACCAUGAUGUGCUGCUACAG |
| MIMAT0000375 | MI0000397 | UAUACAUGCACAUGCACACAUACAUGUA |
| MIMAT0000586 | MI0000623 | UAUGGCUAUAAAGUAACUGAGACGGAUC |
| MIMAT0000609 | MI0000643 | UCCAGGCUCAAAGGGCUCCUCAGGGAAA |
| MIMAT0000605 | MI0000640 | GGGUGAAAGUGUAUGGGCUUUGUGAACA |
| MIMAT0001090 | MI0001160 | GAAAAGGGGUUCACCGAGCAACAUUCGU |
| MIMAT0000387 | MI0000408 | CAGAUGCCCUUUCAUCAUUGCACUGCUU |
| MIMAT0000218 | MI0000231 | AGAACUGAUAUCAGCUCAGUAGGCACCG |
| MIMAT0000219 | MI0000231 | CUCCUGUUCCUGCUGAACUGAGCCAGUG |
| MIMAT0000150 | MI0000164 | CGGCCUGAUUCACAACACCAGCUGCAGC |
| MIMAT0000527 | MI0000566 | CUACGCCAAUAUUUACGUGCUGCUAGAG |
| MIMAT0000527 | MI0000565 | UAACGCCAAUAUUUACGUGCUGCUAAGG |
| MIMAT0000597 | MI0000634 | CAGAGAGGCAGGCACUCGGGCAGACAGA |
| MIMAT0000593 | MI0000630 | CUUACAGUCAGGCUUUGGCUAGAUCAGG |
| MIMAT0000595 | MI0000632 | ACAAGCACUGGACUAGGGGUCAGCAGGC |
| MIMAT0000536 | MI0000577 | CAUAACCGAUUUCAAAUGGUGCUAGACA |
| MIMAT0000221 | MI0000233 | AACAGCUGCUUUUGGGAUUCCGUUGCCC |
| MIMAT0000535 | MI0000576 | UUAUAACCGAUUUCAGAUGGUGCUAGAA |
| MIMAT0000588 | MI0000625 | GCCGACUGACCGACCGACCGAUCGACCG |
| MIMAT0000517 | MI0000551 | CUGGCUGUCAAUUCAUAGGUCAGAGCCC |
| MIMAT0000225 | MI0000237 | CCAUGCCAAUAUUUCUGUGCUGCUAGAG |
| MIMAT0000580 | MI0000617 | GAGACAAAGUUCUGUGAUGCACUGACUU |
| MIMAT0000544 | MI0000585 | CGAAUGCUUUUUGGGGUAAGGGCUUCCG |
| MIMAT0000209 | MI0000585 | UACAGCAAGCCCAGACCGCAAAAAGAUU |
| MIMAT0000209 | MI0000222 | AACAGCAAGCCCAGACCGCAAAAAGAUC |
| MIMAT0000385 | MI0000406 | AGCUACCUGCACUGUUAGCACUUUGACA |
| MIMAT0000672 | MI0000714 | CCUGAACAGGUAGUCUAAACACUGGGUA |
| MIMAT0000212 | MI0000225 | UCACAGUGAAUUCUACCAGUGCCAUACA |
| MIMAT0000706 | MI0000763 | GCAUUCACACCUAGGUUCCAAGGAUUCG |
| MIMAT0003112 | MI0003476 | UUAGCUGCCAUAUAUGUGGUGUCAUUCU |
| MIMAT0003127 | MI0003491 | UUUAUCGGGAGGGGACUGAGCCUGACGA |
| MIMAT0003130 | MI0003493 | CAGCUCGGGGCAGCUCAGUACAGGAUGC |
| MIMAT0003166 | MI0003517 | CUAGCUGACUCCGUGCCACCAUGAUAGA |
| MIMAT0003167 | MI0003518 | AGGCCCAGGAUCGACCUCUGACCUGUCU |
| MIMAT0003168 | MI0003519 | AAAAAGAAGUGCACCGCGAAUGUUUCGU |
| MIMAT0003169 | MI0003520 | CCAACACACCAAGGAUAAUUUCUCCUCA |
| MIMAT0003170 | MI0003521 | GAGUGUGACCAACAUCAGAAUCCCUUCU |
| MIMAT0003171 | MI0003522 | AUCUCGUGACAUGAUGAUCCCCGAGACG |
| MIMAT0003172 | MI0003522 | ACCUUUCAGUUAUCAAUCUGUCACAAGG |
| MIMAT0003173 | MI0003523 | UAUCUCACUCAAAGAUGUACCAAGCAUG |
| MIMAT0003181 | MI0003531 | CCAUCACCAUUGCUAAAGUGCAAUUCCA |
| MIMAT0003182 | MI0003532 | AAAGAGGUUUCCCGUGUAUGUUUCAUCA |
| MIMAT0003183 | MI0003533 | AAAACGUGAAAUUUCCUCUAUGUUUAAU |
| MIMAT0003184 | MI0003534 | AAAAAGUGGAUGACCCUGUACGAUUCGG |
| MIMAT0003186 | MI0003535 | AGAAAAGAl7CAACCAUGUAUUAUUCGAA |
| MIMAT0003185 | MI0003535 | CAAGCGAAUAUAACACGGUCGAUCUCCC |
| MIMAT0003187 | MI0003536 | AACUACCUGCACUAUGAGCACUUUGGCA |
| MIMAT0001546 | MI0003537 | ACAUAUUAGGAACACAUCGCAAAAAUAG |
| MIMAT0003188 | MI0003538 | CACUGCAGUACUGUUCCCGCUGCUAGGG |
| MIMAT0003190 | MI0003539 | AGAGACAAACAAAAUGGAUGCACUUUCC |
| MIMAT0003189 | MI0003539 | CGCGGAGAGGGCCUCCACUUUGAUCGAC |
| MIMAT0003374 | MI0003716 | CUUCUACUAAAACAUGGAAGCACUUACU |
| MIMAT0003373 | MI0003716 | GACAGAAAGCAUUCCCAUGUUAAAGUUG |
| MIMAT0003376 | MI0003717 | CCCCCACUGAAACAUGGAAGCACUUGCU |
| MIMAT0003375 | MI0003717 | ACACAGCAGGUAACCCCAUGUUAAAGCA |
| MIMAT0003377 | MI0003718 | ACCACACUCAAACAUGGAAGCACUUAUU |
| MIMAT0000224 | MI0000733 | ACUUCCACAUGGAGUUGCUGUUACAGAG |

| **Mature Accession** | **Precursor Accession** | **Sequence of Inhibitors Targeting Rat miRNAs (SEQ ID NOS 715-974)** |
|---|---|---|
| MIMAT0000547 | MI0000589 | GGGGUGUUGCAGCGCUUCAUGUUUUGAA |
| MIMAT0001619 | MI0000589 | UACUCCAAAACAUGAAUUGCUGCUGCAU |
| MIMAT0000550 | MI0000591 | CAAAGAGGUCGACCGUGUAAUGUGCGCC |
| MIMAT0000552 | MI0000593 | GGAUGCUUUGACAAUACUAUUGCACUGC |
| MIMAT0000553 | MI0000594 | UUUACACCAAUGCCCUAGGGGAUGCGAG |
| MIMAT0000554 | MI0000594 | CCCCCAGCAGCACCUGGGGCAGUGGGUC |
| MIMAT0000557 | MI0000596 | CAAACACUUACUGAGCACCUACUAGGAA |
| MIMAT0000560 | MI0000599 | CGGGACUGGAGGAAGGGCCCAGAGGCGA |
| MIMAT0000561 | MI0000600 | UGACUACCCUCAUGCCCCUCAAGGAUGA |
| MIMAT0000563 | MI0000601 | CUAAGAAAGGCAGCAGGUCGUAUAGUUA |
| MIMAT0000562 | MI0000601 | UAAAACUAUGCAACCUACUACCUCUUCC |
| MIMAT0000564 | MI0000602 | GGGACGGAAGGGCAGAGAGGGCCAGGGG |
| MIMAT0000566 | MI0000604 | GAAAAAAAGGUUAGCUGGGUGUGUUUCA |
| MIMAT0000568 | MI0000606 | ACCUCUCUGCAGGCCCUGUGCUUUGCUC |
| MIMAT0000570 | MI0000608 | UUGGUUCUAGGAUAGGCCCAGGGGCCUG |
| MIMAT0000572 | MI0000610 | GCCCAAAAGUAACUAGCACACCACGUGG |
| MIMAT0000573 | MI0000611 | UAACCUACCAUAGGGUAAAACCACUGGC |
| MIMAT0000574 | MI0000611 | CCUGUCCGUGGUUCUACCCUGUGGUAGA |
| MIMAT0000575 | MI0000612 | CAAACAUUUUUCGUUAUUGCUCUUGACC |
| MIMAT0000576 | MI0000613 | UCAGAGACUAGAUAUGGAAGGGUGAGAG |
| MIMAT0000577 | MI0000614 | AAGAAAGGCAUCAUAUAGGAGCUGAAUG |
| MIMAT0000579 | MI0000616 | GAGACAAAGUUCUGUGAUGCACUGACUU |
| MIMAT0000581 | MI0000618 | UCUUCAACAAAAUCACUGAUGCUGGAGU |
| MIMAT0000583 | MI0000620 | UACGUGAGCUCCUGGAGGACAGGGACGG |
| MIMAT0000585 | MI0000622 | UAUGGCUAUAAAGUAACUGAGACGGAUC |
| MIMAT0000587 | MI0000624 | GCCGACUGACCGACCGACCGAUCGACCG |
| MIMAT0000589 | MI0000626 | GUGACGGGUGCGAUUUCUGUGUGAGACA |
| MIMAT0000591 | MI0000628 | AGGAUCUGGGCACACGGAGGGAGAGGUU |
| MIMAT0000592 | MI0000629 | CUUACGGUCAGGCUUUGGCUAGAUCAGG |
| MIMAT0000594 | MI0000631 | ACAAGCACUGGACUAGGGGUCAGCAGGC |
| MIMAT0000596 | MI0000633 | CAGAGAGGCAGGCACUCAGGCAGACAGA |
| MIMAT0000598 | MI0000635 | CCAGCUGGOCGACCCAGAGGOACAGUCG |
| MIMAT0000600 | MI0000637 | UACAGCAAGCCCAGACCGCAAAAAGAUU |
| MIMAT0000601 | MI0000637 | CGAAUGCUUUUUGGGGUAAGGGCUUCCG |
| MIMAT0000603 | MI0000638 | GUACUGUAAGUGCUCGUAAUGCAGUAGA |
| MIMAT0000602 | MI0000638 | ACACUACCUGCACUAUAAGCACUUUAGU |
| MIMAT0000604 | MI0000639 | GGGUGAAAGUGUAUGGGCUUUGUGAACA |
| MIMAT0000606 | MI0000641 | AAACAACAAAAUCACUAGUCUUCCACAC |
| MIMAT0000607 | MI0000641 | CCAUAUGGCAGACUGUGAUUUGUUGUCG |
| MIMAT0000608 | MI0000642 | CUCAGGCUCAAAGGGCUCCUCAGGGAAA |
| MIMAT0000611 | MI0000645 | AAUCACAUAGGAAUGAAAAGCCAUAGGC |
| MIMAT0000614 | MI0000647 | UGUCCUCAAGGAGCCUCAGUCUAGUAGG |
| MIMAT0000613 | MI0000647 | ACAUACUAGACUGUGAGCUCCUCGAGGG |
| MIMAT0000615 | MI0000648 | AUUCUUCAGCUAUCACAGUACUGUACCU |
| MIMAT0000774 | MI0000827 | UAAAACUAUACAACCUACUACCUCAUCC |
| MIMAT0000774 | MI0000828 | CUAAACUAUACAACCUACUACCUCAGCC |
| MIMAT0000775 | MI0000829 | UGAAACCACACAACCUACUACCUCACCC |
| MIMAT0000776 | MI0000830 | CUAAACCAUACAACCUACUACCUCAACC |
| MIMAT0000776 | MI0000831 | CAAAACCAUACAACCUACUACCUCACCC |
| MIMAT0000777 | MI0000832 | CUCAACUAUACAACCUCCUACCUCAGCC |
| MIMAT0000778 | MI0000833 | CACAACUAUACAAUCUACUACCUCACUC |
| MIMAT0000778 | MI0000834 | UAAAACUAUACAAUCUACUACCUCAUCC |
| MIMAT0000779 | MI0000835 | ACCAACAGCACAAACUACUACCUCAGCC |
| MIMAT0000606 | MI0000836 | CAACAACAAAAUCACUAGUCUUCCAGAC |
| MIMAT0000780 | MI0000837 | GAACAACAAAAUCACAAGUCUUCCACAU |
| MIMAT0000781 | MI0000838 | CACUCAUACAGCUAGAUAACCAAAGAUA |
| MIMA1'0000781 | MI0000839 | CACUCAUACAGCUAGAUAACCAAAGAGA |
| MIMAT0000781 | MI0000840 | CACUCAUACAGCUAGAUAACCAAAGAUA |
| MIMAT0000782 | MI0000841 | UUACACAAAUUCGGAUCUACAGGGUAUA |
| MIMAT0000783 | MI0000842 | ACCACACAAAUUCGGUUCUACAGGGUAU |
| MIMAT0000784 | MI0000843 | GUAUGUAAACCAUGAUGUGCUGCUACAG |
| MIMAT0000785 | MI0000844 | CUACGCCAAUAUUUACGUGCUGCUAGAG |
| MIMAT0000786 | MI0000845 | CCACUACCUGCACUGUAAGCACUUUGAC |
| MIMAT0000787 | MI0000846 | CACUAUCUGCACUAGAUGCAGCUUAGAA |
| MIMAT0000788 | MI0000847 | CAGUCAGUUUUGCAUGGAUUUGCACAGC |
| MIMAT0000788 | MI0000848 | CAAUCAGUUUUGCAUGGAUUUGCACAGC |
| MIMAT0000789 | MI0000849 | CCAUCAGUUUUGCAUAGAUUUGCACAAC |
| MIMAT0000790 | MI0000850 | CAGUCAACAUCAGUCUGAUAAGCUACCC |
| MIMAT0000791 | MI0000851 | GCAACAGUUCUUCAACUGGCAGCUUUAG |
| MIMAT0003152 | MI0000851 | ACAUAAAGCUUGCCACUGAAGAACUACU |
| MIMAT0000792 | MI0000852 | AGUUGGAAAUCCCUGGCAAUGUGAUUUG |
| MIMAT0000793 | MI0000853 | GCGUGGUAAUCCCUGGCAAUGUGAUUUU |
| MIMAT0000794 | MI0000854 | CUCCUGUUCCUGCUGAACUGAGCCAGUG |
| MIMAT0003153 | MI0000854 | AGAACUGAUAUCAGCUCAGUAGGCACCG |
| MIMAT0000794 | MI0000855 | CUCCUGUUCCUGCUGAACUGAGCCAGUG |
| MIMAT0000795 | MI0000856 | CUGUCAGACCGAGACAAGUGCAAUGCCC |
| MIMAT0000796 | MI0000857 | CACAGCCUAUCCUGGAUUACUUGAACAA |
| MIMAT0000797 | MI0000858 | CACAACCUAUCCUGAAUUACUUGAACUG |
| MIMAT0000798 | MI0000859 | AGGUGCAGAACUUAGCCACUGUGAACAA |
| MIMAT0000799 | MI0000860 | GGGGGCGGAACUUAGCCACUGUGAACAC |
| MIMAT0000800 | MI0000861 | GAACUCAAUAGACUGUGAGCUCCUUGCG |
| MIMAT0000801 | MI0000862 | UAAAACACUGAUUUCAAAUGGUGCUAGA |
| MIMAT0000802 | MI0000863 | UUAUAACCGAUUUCAGAUGGUGCUAGAA |
| MIMAT0000801 | MI0000864 | AAGAACACUGAUUUCAAAUGGUGCUAGA |
| MIMAT0000803 | MI0000865 | CAUAACCGAUUUCAAAUGGUGCUAGACA |
| MIMAT0003154 | MI0000865 | UCUGAACACCAGGAGAAAUCGGUCAGCC |
| MIMAT0000804 | MI0000866 | ACAGCUGAGAGUGUAGGAUGUUUACACA |
| MIMAT0000806 | MI0000868 | GACAGCUGAGUGUAGGAUGUUUACAUGA |
| MIMAT0000807 | MI0000869 | CAGCUUCCAGUCGGGGAUGUUUACAGAC |
| MIMAT0000808 | MI0000870 | CAGCUUCCAGUCGAGGAUGUUUACAGUU |
| MIMAT0000809 | MI0000870 | GCAGCUGCAAACAUCCGACUGAAAGCCC |
| MIMAT0000804 | MI0000871 | ACAGCUGAGAGUGUAGGAUGUUUACAGU |
| MIMAT0000810 | MI0000872 | CAACAGCUAUGCCAGCAUCUUGCCUCCU |
| MIMAT0000811 | MI0000873 | ACAUGCAACUUAGUAAUGUGCAAUAUCC |
| MIMAT0000812 | MI0000874 | ACAUGCAAUGCAACUACAAUGCACCACG |
| MIMAT0000813 | MI0000875 | CUACAAUCAGCUAAUUACACUGCCUACA |
| MIMAT0000814 | MI0000876 | UUAGCAAUCAGCUAACUACACUGCCUAG |
| MIMAT0000815 | MI0000877 | CACAACAACCAGCUAAGACACUGCCAAA |
| MIMAT0000816 | MI0000878 | UCAACAGGCCGGGACAAGUGCAAUACUA |
| MIMAT0000816 | MI0000879 | UCCUCAGGCCGGGACAAGUGCAAUACUU |
| MIMAT0000817 | MI0000880 | GCACUACCUGCACGAACAGCACUUUGGA |
| MIMAT0000818 | MI0000881 | ACAAGCAAAAAUGUGCUAGUGCCAAAAU |
| MIMAT0000820 | MI0000883 | CACCACAAGAUCGGAUCUACGGGUUUAU |
| MIMAT0000821 | MI0000884 | CCCCGCAAGGUCGGUUCUACGGGUGGGU |
| MIMAT0000822 | MI0000885 | CAGCACAAGUUCGGAUCUACGGGUUUGU |
| MIMAT0000823 | MI0000886 | AUCCUUCAGUUAUCACAGUACUGUACCU |
| MIMAT0000824 | MI0000887 | CUUUCAUAGCCCUGUACAAUGCUGCUUG |
| MIMAT0000824 | MI0000888 | CCUUCAUAGCCCUGUACAAUGCUGCUUG |
| MIMAT0000825 | MI0000889 | CACUAUCUGCACUGUCAGCACUUUAGUC |
| MIMAT0000826 | MI0000890 | CUUUGAUAGCCCUGUACAAUGCUGCUUG |
| MIMAT0000827 | MI0000891 | GACACAAACACCAUUGUCACACUCCAGA |
| MIMAT0000828 | MI0000892 | UCUUGGCAUUCACCGCGUGCCUUAAUUG |
| MIMAT0000828 | MI0000893 | UCUUGGCAUUCACCGCGUGCCUUAAUUG |
| MIMAT0000828 | MI0000894 | UCUUGGCAUUCACCGCGUGCCUUAAUUG |
| MIMAT0000829 | MI0000895 | CCUCACAGGUUAAAGGGUCUCAGGGACC |
| MIMAT0000830 | MI0000896 | ACAUCACAAGUUAGGGUCUCAGGGACUG |
| MIMAT0000830 | MI0000897 | ACCUCACAAGUUAGGGUCUCAGGGACUA |
| MIMAT0000832 | MI0000898 | CCACGCAUUAUUACUCACGGUACGAGUU |
| MIMAT0000831 | MI0000898 | ACAGCGCGUACCAAAAGUAAUAAUGUGC |
| MIMAT0000833 | MI0000899 | ACCAGCCAAGCUCAGACGGAUCCGAUGA |
| MIMAT0000834 | MI0000900 | UGAAAAAGAGACCGGUUCACUGUGAGAA |
| MIMAT0000835 | MI0000901 | AGGGAAAGAGACCGGUUCACUGUGAGAC |
| MIMAT0000600 | MI0000902 | AACAGCAAGCCCAGACCGCAAAAAGACC |
| MIMAT0000836 | MI0000903 | CCGAUGCCCUUUUAACAUUGCACUGCUC |
| MIMAT0000837 | MI0000904 | CGGAUGCCCUUUCAUCAUUGCACUGCUU |
| MIMAT0000838 | MI0000905 | GGGCGACCAUGGCUGUAGACUGUUACCU |
| MIMAT0000839 | MI0000906 | GCUACAGCUGGUUGAAGGGGACCAAAUC |
| MIMAT0000840 | MI0000907 | ACGCCCCUCUGGUCAACCAGUCACACAC |
| MIMAT0000841 | MI0000908 | CGAUCACAUAGGAAUAAAAAGCCAUAAA |
| MIMAT0000842 | MI0000909 | GAAUCCAUCAUCAAAACAAAUGGAGUCC |
| MIMAT0000843 | MI0000910 | CGACUACGCGUAUUCUUAAGCAAUAACA |
| MIMAT0000844 | MI0000911 | CGGCCUGAUUCACAACACCAGCUGCAGC |
| MIMAT0000844 | MI0000912 | CGGCCUGAUUCACAACACCAGCUGUCCC |
| MIMAT0000845 | MI0000913 | ACUGGAGACACGUGCACUGUAGAAUACA |
| MIMAT0000846 | MI0000914 | GGGCCAUCUUUACCAGACAGUGUUAGGA |
| MIMAT0000847 | MI0000915 | GUUAGUAGUGCUUUCUACUUUAUGGGUG |
| MIMAT0000848 | MI0000915 | UCAUCCAUAAAGUAGGAAACACUACACC |
| MIMAT0000849 | MI0000916 | UCCUGAGCUACAGUGCUUCAUCUCAGAC |
| MIMAT0000850 | MI0000917 | AGACUAGUACAUCAUCUAUACUGUAGUG |
| MIMAT0000851 | MI0000918 | CCAAGGGAUUCCUGGGAAAACUGGACCG |
| MIMAT0000852 | MI0000919 | UAUAACCCAUGGAAUUCAGUUCUCAGAG |
| MIMAT0000853 | MI0000920 | CAGCACUGGUACAAGGGUUGGGAGACAG |
| MIMAT0000854 | MI0000921 | GGGCCCAAGUUCUGUCAUGCACUGACUG |
| MIMAT0000855 | MI0000922 | AUGAUCACUUUUGUGACUAUGCAACUGG |
| MIMAT0000856 | MI0000923 | AAGCGAAGGCAACACGGAUAACCUAUCU |
| MIMAT0000857 | MI0000924 | CAAACUCACCGACAGGUUGAAUGUUCCC |
| MIMAT0000858 | MI0000925 | CAAACUCACCGACAGCGUUGAAUGUUCC |
| MIMAT0000859 | MI0000926 | CAACCCACCGACAGCAAUGAAUGUUGAU |
| MIMAT0000859 | MI0000927 | AAACCCACCGACAGCAAUGAAUGUUGAG |
| MIMAT0000860 | MI0000928 | UCACAGUGAAUUCUACCAGUGCCAUACA |
| MIMAT0000861 | MI0000929 | CUUACCCUUAUCAGUUCUCCGUCCAACA |
| MIMAT0000862 | MI0000930 | AUCAGGAACUGCCUUUCUCUCCAAUCCC |
| MIMAT0000863 | MI0000931 | AGAAAGCCCAAAAGGAGAAUUCUUUGGA |
| MIMAT0000864 | MI0000932 | CCUCCGGCUGCAACACAAGACACGAGGG |
| MIMAT0000865 | MI0000933 | ACAACCUAAUAUAUCAAACAUAUCACAC |
| MIMAT0000866 | MI0000934 | AACAGCUGCUUUUGGGAUUCCGUUGCCC |
| MIMAT0000867 | MI0000935 | UACUGGCUGUCAAUUCAUAGGUCAGAGC |
| MIMAT0000868 | MI0000936 | AGGACUGGGACUUUGUAGGCCAGUUGAA |
| MIMAT0000869 | MI0000937 | CAGUCCACAUGGAGUUGCUGUUACACGU |
| MIMAT0000870 | MI0000939 | CCGUGCCAAUAUUUCUGUGCUGCUAGAG |
| MIMAT0000871 | MI0000940 | AAUCCCAACAACAUGAAACUACCUAAGC |
| MIMAT0000872 | MI0000941 | CCUGAACAGGUAGUCUGAACACUGGGGC |
| MIMAT0000873 | MI0000942 | CCUCCAUCAUUACCCGGCAGUAUUAGAG |
| MIMAT0000874 | MI0000943 | UGAACAUCGUUACCAGACAGUGUUAGAG |
| MIMAT0000875 | MI0000944 | GCCGUCAUCAUUACCAGGCAGUAUUAGA |
| MIMAT0000876 | MI0000945 | GGUCUAGUGGUCCUAAACAUUUCACAAU |
| MIMAT0000877 | MI0000946 | CUCAGGCAUAGGAUGACAAAGGGAAGUC |
| MIMAT0000878 | MI0000947 | AGACAGACUCCGGUGGAAUGAAGGACAG |
| MIMAT0000879 | MI0000948 | AAACCACACACUUCCUUACAUUCCAUAG |
| MIMAT0000880 | MI0000949 | CCAACAAGCUUUUUGCUCGUCUUAUACG |
| MIMAT0000881 | MI0000950 | AGAUCAGCCGCUGUCACACGCACAGUGG |
| MIMAT0000882 | MI0000951 | CCUAGGCAAAGGAUGACAAAGGGAAGCC |
| MIMAT0000883 | MI0000952 | CGGUGGCCGUGACUGGAGACUGUUACUG |
| MIMAT0000884 | MI0000953 | UAGGGUACAAUCAACGGUCGAUGGUUUU |
| MIMAT0000885 | MI0000954 | GUGACUGCCUGUCUGUGCCUGCUGUACA |
| MIMAT0000886 | MI0000955 | AUCUCACAGUUGCCAGCUGAGAUUAAAC |
| MIMAT0000887 | MI0000956 | UUAUCCAGUCAGUUCCUGAUGCAGUAUC |
| MIMAT0000888 | MI0000957 | CACCACAUGGUUAGAUCAAGCACAAAGG |
| MIMAT0000888 | MI0000958 | AAGCACAUGGUUAGAUCAAGCACAACAG |
| MIMAT0000889 | MI0000959 | CUCGAGAAUUGCGUUUGGACAAUCAGGA |
| MIMAT0000889 | MI0000960 | UACAAGAAUUGCGUUUGGACAAUCAGUG |
| MIMAT0000890 | MI0000961 | CCUGAAACCCAGCAGACAAUGUAGCUGU |
| MIMAT0000891 | MI0000962 | CAGAGACCCAGUAGCCAGAUGUAGCUGC |
| MIMAT0000892 | MI0000963 | ACUUGGGGUAUUUGACAAACUGACACUC |
| MIMAT0000893 | MI0000964 | AAAAAAAAGUGCCCCCAUAGUUUGAGAA |
| MIMAT0000894 | MI0000965 | CCAAGAGAGGGCCUCCACUUUGAUGGCU |
| MIMAT0000895 | MI0000965 | AGUGGCACACAAAGUGGAAGCACUUUCU |
| MIMAT0000896 | MI0000966 | ACCCAAAAGAGCCCCCAGUUUGAGUAUC |
| MIMAT0000897 | MI0000966 | GUAACACUCAAAACCUGGCGGCACUUUU |
| MIMAT0000898 | MI0000967 | CACAACAGGAUUGAGGGGGGGCCCUCCA |
| MIMAT0000900 | MI0000969 | AAGGGAAGAACAGCCCUCCUCUGCCGAA |
| MIMAT0000901 | MI0000970 | AAAAUGUAUGUGGGACGGUAAACCAUUU |
| MIMAT0000902 | MI0000971 | CUCGAAGAGAGCUUGCCCUUGCAUAUUC |
| MIMAT0000903 | MI0000972 | UUUUUCGCCCUCUCAACCCAGCUUUUCC |
| MIMAT0001082 | MI0001152 | GAUCCCAACAACAGGAAACUACCUAAAU |
| MIMAT0001320 | MI0001423 | CAUUCAACAAACAUUUAAUGAGGCCUAC |
| MIMAT0001534 | MI0001639 | GAGAUGGGACAUCCUACAUAUGCAACCA |
| MIMAT0001538 | MI0001643 | UGGACGGCAUUACCAGACAGUAUUAGAC |
| MIMAT0001543 | MI0001650 | UCAACCAGCUAACAAUACACUGCCAACC |
| MIMAT0001547 | MI0001654 | ACACAUUAGGAACACAUCGCAAAAACAG |
| MIMAT0001549 | MI0001656 | ACAAUAAGGAUUUUUAGGGGCAUUAUGA |
| MIMAT0000805 | MI0000867 | AGCUUCCAGUCAAGGAUGUUUACAGUAG |
| MIMAT0000819 | MI0000882 | CACAACAAUACAACUUACUACCUCACCC |
| MIMAT0001628 | MI0001724 | AGAACACCGAGGAGCCCAUCAUGAUCCU |
| MIMAT0001633 | MI0001731 | CUAAACUCAGUAAUGGUAACGGUUUCCU |
| MIMAT0003121 | MI0003485 | AGAACAAGACGGGAGGGGAGGAGUGAGG |
| MIMAT0003113 | MI0003477 | UUAGCUGCCAUAUAUGUGAUGUCAUUCU |
| MIMAT0003114 | MI0003478 | CAAAGCCACAGUCACCUUCUGAUCUGAG |
| MIMAT0003115 | MI0003479 | AGAAAGGGAGGAGAGCCAGGAGAAGCGC |
| MIMAT0003116 | MI0003480 | CAUUUUCACCCAGGGACAAAGGAUUAGA |
| MIMAT0003117 | MI0003481 | UAAGUACCCCUGGAGAUUCUGAUAAGCU |
| MIMAT0003118 | MI0003482 | CACUGUCUGUCAAAUCAUAGGUCAUUGU |
| MIMAT0003119 | MI0003483 | UACUAAACGGAACCACUAGUGACUUGAA |
| MIMAT0003122 | MI0003486 | ACAAACCAGGUUCCACCCCAGCAGGCAC |
| MIMAT0003123 | MI0003487 | CAACCAAAAGUUGCCUUUGUGUGAUUCA |
| MIMAT0003124 | MI0003488 | CCGACGGCUAGUGGACCAGGUGAAGUAC |
| MIMAT0003162 | MI0003489 | UAUGGGUACAUAAAGAAGUAUGUGCUCU |
| MIMAT0003125 | MI0003489 | AAAAUACACACUUCUUUACAUUCCAUAG |
| MIMAT0003126 | MI0003490 | GCUGUAGCUGGUUGAAGGGGACCAAACC |
| MIMAT0003174 | MI0003524 | AGGCCCAGGAUCGACCUCUGACCUGUCU |
| MIMAT0003175 | MI0003525 | AAAAAGAAGUGCACCGCGAAUGUUUCGU |
| MIMAT0003176 | MI0003526 | CAAACACACCAAGGAUAAUUUCUCCUCA |
| MIMAT0003177 | MI0003527 | GAGUGUGACCAACAUCAGAAUCCCUUCU |
| MIMAT0003178 | MI0003528 | AUCUCGUGACAUGAUGAUCCCCGAGACG |
| MIMAT0003179 | MI0003528 | ACCUUUCAGUUAUCAAUCUGUCACAAGU |
| MIMAT0003191 | MI0003540 | GGGCCUGGCACACAGUACACCUUCACCG |
| MIMAT0003192 | MI0003541 | ACGCCUACGUUCCAUAGUCUACCACCUC |
| MIMAT0003193 | MI0003542 | AAAGAGGUUUCCCGUGUAUGUUUCAUCA |
| MIMAT0003194 | MI0003543 | AAAACGUGAAAUUUCCUCUAUGUUUAAU |
| MIMAT0003195 | MI0003544 | ACGUAACCAUAGAAGGAAUAUCCACCUU |
| MIMAT0003196 | MI0003544 | AAAAAGUGGAUGUUCCUCUAUGAUUAUC |
| MIMAT0003197 | MI0003545 | UGUACUCAUAGAAGGAGAAUCUACCUUU |
| MIMAT0003198 | MI0003545 | AAAACGUGGAUUUUCCUCUACGAUUAGU |
| MIMAT0003199 | MI0003546 | UCACAGAGAGCUUGCCCUUGUAUAUCCC |
| MIMAT0003200 | MI0003547 | AAAAAGUGGAUGACCCUGUACGAUUCGG |
| MIMAT0003202 | MI0003548 | AAAAAAGUGUUGUCCGUGAAUGAUUCGU |
| MIMAT0003201 | MI0003548 | AAGCGAAUCCACCACGAACAACUUCUCU |
| MIMAT0003203 | MI0003549 | AUCGAAUUCAUCACGGCCAGCCUCUCUC |
| MIMAT0003205 | MI0003550 | AAAAGGGGUUCACCGAGCAACAUUCGUC |
| MIMAT0003204 | MI0003550 | GAUGCAAAGUUGCUCGGGUAACCUCUCU |
| MIMAT0003206 | MI0003551 | UAAGCGAAUAUAACACGGUCGAUCUCCC |
| MIMAT0003207 | MI0003551 | AGAAAAGAUCAACCAUGUAUUAUUCGAA |
| MIMAT0003208 | MI0003552 | GAACACUUAGCAGGUUGUAUUAUAUCCA |
| MIMAT0003210 | MI0003553 | GUUUACAGAUGGAUACCGUGCAAUUUCU |
| MIMAT0003209 | MI0003553 | UCAAAAUUGCAUCGUGAUCCACCCGAUA |
| MIMAT0003211 | MI0003554 | ACCUACCUGCACUAUGAGCACUUUGGCA |
| MIMAT0003212 | MI0003554 | GUACCAGAAGUGCUCACACUGCAGUAGA |
| MIMAT0003213 | MI0003555 | ACUGCAGUACUGUUCCCGCUGCUAGGGC |
| MIMAT0003378 | MI0003719 | AACACACAGGACCUGGAGUCAGGAGCCC |
| MIMAT0003379 | MI0003719 | CAGGCCUUCUGACUCCAAGUCCAGUGCU |
| MIMAT0003380 | MI0003720 | GAGAGGAAACCAGCAAGUGUUGACGCUA |
| MIMAT0003381 | MI0003721 | AGCUAAACAUCACUGCAAGUCUUAACAG |
| MIMAT0003382 | MI0003722 | UUGUAGGCUGGGGAGUAAAUGAAUAGAA |
| MIMAT0003382 | MI0003723 | UUGUAGGCUGGGGAGUAAAUGAAUAGAA |
| MIMAT0003383 | MI0003724 | CCGUACAAACCACAGUGUGCUGCUGGGG |
| MIMAT0000599 | MI0000636 | CCUAGAGGUUAAGACAGCAGGGCUGUGG |
| MIMAT0000610 | MI0000644 | AUCGUACUAUGCAACCUACUACUCUACA |
| MIMAT0000869 | MI0000938 | ACUUCCACAUGGAGUUGCUGUUACAGGG |
| MIMAT0000899 | MI0000968 | AUGCAUGCAUACAUGCACACAUACAUGC |
| MIMAT0001626 | MI0001722 | GGCCUGCAUGACGGCCUGCAAGACACCU |

### EXAMPLES

### General Techniques and Nomenclatures

For most of the experiments reported, quantitation of the level of inhibition was performed using the dual luciferase reporter system, psiCheck 2 (Promega). Briefly, the psiCheck plasmid encodes for two variants of luciferase, Renilla and Firefly. Target sequences were inserted into the multiple cloning site of the 3' UTR of the Renilla luciferase gene, thus allowing the Firefly sequence to be used as an internal control. To determine the practicality of different inhibitor designs, the oligonucleotide(s) of the invention and the modified psiCheck 2 plasmid were co-transfected into cells (100 ng of reporter DNA per well, 25-100 nM inhibitor, 0.3 microliters Lipofectamine 2000, Invitrogen). Twenty-four to ninety-six hours later cells were lysed and the relative amounts of each luciferase was determined using the Dual Glo Assay (Promega). For all experiments, unless otherwise specified, the transfection efficiency was ensured to be over 95%, and no significant levels of cellular toxicity were observed.

Firefly and *Renilla* luciferase activities were measured using the Dual-Glo™ Luciferase Assay System (Promega, Cat.# E2980) according to manufacturer's instructions with slight modification. When lysing cells, growth media was aspirated from the cells prior to adding 50 uL of firefly luciferase substrate and 50 uL *Renilla* luciferase substrate.

The Luciferase assays were all read with a Wallac Victor² 1420 multilabel counter (Perkin Elmer) using programs as recommended by the manufacturers.

All treatments were run in triplicate. In addition, each experimental treatment with a reporter plasmid was duplicated with the psiCHECK™-2 control plasmid (no insert). To account for non-specific effects on reporter plasmids, experimental results are expressed as a normalized ratio (Rluc/Fluc)ₙₒᵣₘ: the ratio of *Renilla* luciferase expression to firefly luciferase expression for a given miRNA reporter plasmid (Rluc/Fluc)_{miRNA} divided by the (Rluc/Fluc)_{control} ratio for the identically treated psiCHECK™ -2 reporter plasmid. The maximum values obtained from the reporter plasmid vary due to sequence; ideally values around 1 indicate low miRNA function, while values close to zero indicate high miRNA function. Data are reported as the average of the three wells and the error bars are the standard deviation of the three (Rluc/Fluc)_{miRNA} ratios from the experimental treatment, scaled by the normalizing factor (the average of (Rluc/Fluc)_{control}). We recognize that ratios do not follow a Normal distribution, but feel that the standard deviation values give a good sense of the variability of the data.

In cases where values between different miRNA reporter plasmids are compared, the maximum normalized (Rluc/Fluc)ₙₒᵣₘ ratio was used as an additional scaling factor so that all reporters have a maximum of approximately 1. The additional scaling was performed for ease of comparison and does not affect the results.

To study the effectiveness of 2'-ACE modified inhibitors, in vitro studies were performed to assess the ability of these molecules to prevent the cleavage of a labeled artificial substrate. Specifically reaction mixtures containing a radio labeled let-7 target molecule were incubated with HeLa cell extracts (3 micrograms of protein in 50 mM Tris buffer, pH 7.5, 0.1 % NP-40, 1 microgram tRNA, 5 mM ATP, 2 mM MgCl2, 37°C) in the presence of 2'-O-methylated or 2'-ACE modified 31 nucleotide inhibitor molecules. Following a 10-minute incubation, reactions were analyzed on a native polyacrylamide gel to determine the level of miRNA target cleavage.

Cells were grown under standard conditions and released from the solid support by trypsinization. For most assays, cells were diluted to 1 X 10⁵ cells/ml, followed by the addition of 100 µL of cells/well. Plates were then incubated overnight at 37° C, 5% CO₂.

Inhibitors were synthesized using modifications of 2' ACE chemistry described previously.

### Example 1

### Identification of Optimal Lengths for Inhibitors

To determine the optimal length of inhibitors, fully 2' O-methyl modified oligonucleotides targeting miR-21 and let-7c were synthesized with varying lengths (see Table II below). The additional sequences (underlined) were: 1) simultaneously added to both the 5' and 3' ends of the molecule, and 2) were the reverse complement of sequences bordering the mature sequence in the primary miRNA.

**Table 2**

| **Table of Inhibitors with varying lengths targeting Let-7c and miR-21** | | |
|---|---|---|
| MiR | Reverse Complement Sequence (SEQ ID NOS 975-992) | Added nts |
| Let-7C | AACCAUACAACCUACUACCUCA | 0 |
| | UAAACCAUACAACCUACUACCUCAAC | +2 |
| | UCUAAACCAUACAACCUACUACCUCAACCC | +4 |
| | ACUCUAAACCAUACAACCUACUACCUCAACCCGG | +6 |
| | UAACUCUAAACCAUACAACCUACUACCUCAACCCGGAU | +8 |
| | UGUAACUCUAAACCAUACAACCUACUACCUCAACCCGGAUGC | +10 |
| | GGUGUAACUCUAAACCAUACAACCUACUACCUCAACCCGGAUGCAC | +12 |
| | AGGGUGUAACUCUAAACCAUACAACCUACUACCUCAACCCGGAUGCACAC | +14 |
| | CCAGGGUGUAACUCUAAACCAUACAACCUACUACCUCAACCCGGAUGCACACAG | +16 |
| MiR-21 | UCAACAUCAGUCUGAUAAGCUA | |
| | AGUCAACAUCAGUCUGAUAAGCUACC | +2 |
| | ACAGUCAACAUCAGUCUGAUAAGCUACCCG | +4 |
| | CAACAGUCAACAUCAGUCUGAUAAGCUACCCGAC | +6 |
| | UUCAACAGUCAACAUCAGUCUGAUAAGCUACCCGACAA | +8 |
| | GAUUCAACAGUCAACAUCAGUCUGAUAAGCUACCCGACAAGG | +10 |
| | GAGAUUCAACAGUCAACAUCAGUCUGAUAAGCUACCCGACAAGGUG | +12 |
| | AUGAGAUUCAACAGUCAACAUCAGUCUGAUAAGCUACCCGACAAGGUG GU | +14 |
| | CCAUGAGAUUCAACAGUCAACAUCAGUCUGAUAAGCUACCCGACAAGG UGGUAC | +16 |

Subsequently, the sequences were co-transfected into cells at 100, 50 and 25 nM concentrations along with the appropriate psiCheck reporter construct (target sequence inserted into psiCheck multiple cloning site = let-7c target site: sense strand 5'-TCGAATGACCAACCATACAACCTACTACCTCACTCGAGCTGC (SEQ ID NO: 13); miR-21 target site: sense strand, 5'-TCGAATGACCTCAACATCAGTCTGATAAGCTAC TCGAGCTGC (SEQ ID NO: 14); sites inserted into the NotI-XhoI digest of psiCHECK2) and the level of expression of the reporter was assessed at 48 hours. Results of these studies identified that previous 21 nts and 31 nts designs were suboptimal and that longer molecules were far more potent (Figure 5A and 5B). Specifically, 21 and 31 nucleotide, 2'-O-methyl inhibitors provided minimal levels of silencing of endogenous miRNAs. At 100 nM, 21 and 31 nts modified oligos targeting Let-7c gave reporter expression levels of only 18 and 21% (respectively) relative to controls, suggesting that the endogenous miRNAs were only mildly inhibited by the two shorter constructs. Similarly, cells transfected with 21 and 31 nts modified oligos targeting miR-21 (e.g., 100 nM) exhibited 5 and 30% (respectively) relative to controls, again suggesting that the endogenous miRNAs were only mildly inhibited by the two shorter constructs. In contrast, the inventors have identified that a minimal sequence length for 2'-O-methyl inhibitor performance is 48 nucleotides (e.g., 22 nucleotides for the mature sequence plus 12 nucleotides on both the 5 and 3' ends of the molecule). At 50-100 nM concentrations, these molecules (and inhibitors with 14 and 16 nts 5' and 3' flanking sequences) provided 80-100% silencing of the respective miRNAs. At lower concentrations (e.g., 25 nM), where shorter molecules exhibited minimal levels of activity, inhibitors of the invention that had flanking regions of 12 nucleotides or greater in the 5' and 3' flanking regions induced 50-70% inhibition of the respective miRNAs. Lastly, longer inhibitors were found to silence for longer periods of time, thus the longer molecules exhibit and enhanced longevity of silencing.

### Example 2

### Identification of optimal positions

To assess whether the positioning of the flanking sequences was critical for the enhanced inhibitory effects, a second set of experiments was performed to determine whether performance was enhanced by preferentially adding the nucleotides to the 5' or 3' end of the sequence that was the reverse complement of the mature miRNA sequence. Specifically, inhibitor molecules containing the reverse complement (RC) of the mature let-7c or miR21 sequences were synthesized with 16 modified nucleotides associated with a) the 5' (16 + RC +0) end of the sequence, b) the 3' end of the molecule (0 + RC + 16), or c) both ends of the molecule (16 + C + 16). In all cases, the additional sequences were the reverse complement of the appropriate primary miRNA sequences that bordered the mature miRNA sequence. See Table 3 below.

**Table 3**

| MiR | Reverse Complement Sequence (SEQ ID NOS 993-998) | Added nts |
|---|---|---|
| Let-7C | | |
| | CCAGGGUGUAACUCUAAACCAUACAACCUACUACCUCAACCCGGAUGCACACAG | 16+ RC + 16 |
| | CCAGGGUGUAACUCUAAACCAUACAACCUACUACCUCA | 16 + RC + 0 |
| | AACCAUACAACCUACUACCUCAACCCGGAUGCACACAG | 0 + RC + 16 |
| MiR-21 | | |
| | CCAUGAGAUUCAACAGUCAACAUCAGUCUGAUAAGCUACCCGACAAGGUGGUAC | 16+ RC + 16 |
| | CCAUGAGAUUCAACAGUCAACAUCAGUCUGAUAAGCUA | 16 + RC + 0 |
| | UCAACAUCAGUCUGAUAAGCUACCCGACAAGGUGGUAC | 0 + RC + 16 |

The level of inhibition induced by these molecules was studied by co-transfecting each inhibitor into cells along with the appropriate psiCheck reporter construct. As shown in Figure 6A and 6B, at all concentrations, the most potent molecule was the 16 + RC + 16 inhibitor, followed by the 0 + RC + 16. The least potent inhibitor at all concentrations was the 16 + RC +0 variant. These results further demonstrate that longer 2'-O-methyl molecules are better inhibitors and that in some cases, sequences added to the 3' terminus of the central region of the inhibitor exhibit superior performance to inhibitor sequences where the sequences added to the 5' terminus of the central region of the inhibitor.

### Example 3

### Identifying Preferred and Non-preferred Flanking Sequences

An experiment was designed to test the importance of the following: (1) central region sequences of the inhibitor that anneal to sequences that flank the mature miRNA; and (2) 5' and 3' flanking regions of inhibitors that have nucleotide contents that mimic mRNA. Inhibitors were designed with a central region that was the reverse complement of miR21 or let-7C and contained the following: (1) 16 nucleotide flanking regions that were the reverse complement of sequences bordering each of the mature miRNA sequences (16 + RC +16), (2) 16 nucleotide flanking regions that were representative of mRNA (~ 25% A, T, G, and C, 16AR+RC+16AR), or (3) 16 nucleotide flanking regions that were not representative of mRNA (*i.e.,* polypyrimidine flanks, 16US+RC+16US). The flanking sequences that were representative of mRNA were based on cel-miR-51 sequences that have no human homolog. (See Table 4). The level of inhibition induced by these molecules was then studied by co-transfecting each inhibitor into cells along with the appropriate psi Check reporter construct.

**Table 4**

| MiR | Sequence (SEQ ID NOS 999-1004) | Content |
|---|---|---|
| Let-7c | CCAGGGUGUAACUCUAAACCAUACAACCUACUACCUCAAC CCGGAUGCACACAG | 16+RC+16 |
| | AGCUCUCAUCCAUGUUAACCAUACAACCUACUACCUCAUU GUACCUACUCUCGA | 16AR+RC+16AR |
| | CCUCUCCUCUCCCUCUAACCAUACAACCUACUACCUCACCU CUCCUCUCCCUCU | 16US+RC+16US |
| MiR-21 | CCAGGGUGUAACUCUAUCAACAUCAGUCUGAUAAGCUAAC CCGGAUGCACACAG | 16+RC+16 |
| | AGCUCUCAUCCAUGUUUCAACAUCAGUCUGAUAAGCUAUU GUACCUACUCUCGA | 16AR+RC+16AR |
| | CCUCUCCUCUCCCUCUUCAACAUCAGUCUGAUAAGCUAAC CCGGAUGCACACAG | 16US+RC+16US |

In most cases, inhibitors that had 16 nucleotide 5' and 3' flanking regions that were the reverse complement of the regions that bordered the mature miRNA sequence performed equally to those that had 16 nucleotide flanking regions that were representative of mRNA (see Figures 7A and 7B). Still, in a subset of cases, inhibitors that had flanking regions that were representative of mRNA (14AR-mature-14AR, miR-107, 5'-AGCUCUCAUCCAUGCUUUGAUAGCCCUGUACAAUGCUGCUUGGUACCUA CUCUCGA (SEQ ID NO: 15)) outperformed equivalent inhibitors that were the reverse complement of the regions that bordered the mature miRNA sequence (Figures 7A and 7B, 5'-AAGCUCUCUGUGCUUUGAUAGCCCUGUACAAUGCUGCUUGAACUCCAU GCCACA (SEQ ID NO: 13)).

In addition, it was observed that molecules that comprised polypyrimidine flanking regions performed more poorly than sequences that more closely match the nucleotide content of mRNAs. Overall, these findings suggest that the nucleotide content of flanking regions can play a role in overall inhibitor functionality.

### Example 4

### Multi-miRNA Targeting Using Inhibitors

Due to the heightened potency of inhibitor molecules of the invention, it was predicted that the new design would be capable of simultaneously targeting multiple miRs while previous designs could not. To test this, 21 nucleotide 2'-O-methyl modified, or 56 nts 2'-O-methyl modified inhibitors (28 nts central region, 14 nts 5' flanking region (5'-AGCUCUCAUCC AUG (SEQ ID NO: 4)) and 14 nts 3' flanking regions (5'-GUACCUACUCUCGA (SEQ ID NO: 5))) targeting miR-18, miR-22, and Let-7c were simultaneously transfected into cells (10K cells, Lipofectamine 2000, 100, 50, and 25 nM) along with one of the three reporter constructs (miR-18, miR-22, or Let-7c target sites) designed to detect function of each of the miRs. Subsequently, the level of luciferase activity was measured to determine the ability of each inhibitor to function in the presence of inhibitors targeting different miRNA.

The results of these studies are shown in Figure 8. With the 21 nucleotide, 2'-O-methyl modified design; variable levels of miR inhibition were observed in the multiplexing experiment. Twenty-one nucleotide inhibitors targeting let-7c performed poorly (<10% inhibition) while miR 22 and 18 inhibitors exhibited moderate levels of luc expression (40 and 60% of controls). Performance of the 21 nucleotide designs dropped at lower concentrations (at 25 nM: let-7c <10%, miR-22 = ~30%, and miR-18 = ~40% of controls). In contrast, longer, 58 nt inhibitors of the invention were observed to be more potent inhibitors at all of the concentrations studied and thus capable of efficient multi-miR inhibition. At 100 nM concentrations, all three inhibitors exhibited 65-100% of the luciferase levels observed in control experiments where vectors lacked the target sequence. While the degree of inhibition of let-7c dropped slightly at lower concentrations (~40% of control expression at 25 nM), the degree of inhibition by miR-18 and miR-22 inhibitors remained above 70% for both molecules. Lastly, the inhibition by these molecules was specific. As such, transfection of an inhibitor targeting miR-18 did not alter the expression of reporters designed to measure Let-7c or miR-22 (data not shown). These results demonstrate the heightened potency of molecules of the invention and display the ability of these agents to perform under circumstances where multiple miRNAs need to be simultaneously inhibited.

### Example 5

### Effectiveness of ACE-modified Inhibitors

To test the ability of ACE-modified nucleotides to function as inhibitors, an in vitro assay was performed to test the ability of these molecules to inhibit cleavage of a labeled, artificial target. Specifically a 41 nucleotide P32-labeled let-7 target molecule was incubated with HeLa cell extracts (3 micrograms of protein in 50 mM Tris buffer, pH 7.5, 0.1 % NP-40, 1 microgram tRNA, 5 mM ATP, 2 mM MgGl2, 37°C) in the presence of 25, 2.5, or 0.25 nanomolar 2'-O-methylated or 2'-ACE modified 31 nucleotide inhibitor molecules. Following a 10-minute incubation, reactions were analyzed on a native polyacrylamide gel to determine the level of miRNA target cleavage. As shown in Figure 9, 2'-O-methylated 31 nucleotide inhibitors prevented cleavage of the target at concentrations of 2.5 nM and above. In contrast, 2'-ACE modified inhibitors prevented the formation of cleavage product at all of the concentrations investigated, thus demonstrating the viability of using 2' ACE modifications in inhibitor designs of the invention.

### Example 6

### Duplex Structures of Hairpins Enhance Inhibitor Functionality

To test the effects of double stranded regions on inhibitor functionality, the 5' flanking region, 3' flanking region, or both 5' and 3' flanking regions of let7c and miR21 inhibitors were designed so that each respective flanking sequence would fold back upon itself to create a hairpin structure. All of the oligonucleotides were synthesized with a 2'-O-methyl modification at each position and sequences for each of the inhibitors tested are found in Table 5. Subsequently, the functionality of each inhibitor design was compared with short reverse complement (*e.g*., RC, 22 nts in length), and longer inhibitor designs consisting of the RC plus 5' and 3' flanking regions of equivalent length (*e.g.*, 16 nts) that do not form hairpin structures by transfecting each design into HeLa cells (*e.g.,* 50 and 25 nM) and assessing the degree of inhibition using the dual luciferase assay.

**Table 5**

| **Figure** | **Name of inhibitor molecule** | **Sequence (SEQ ID NOS 1005-1156)** |
|---|---|---|
| 10A | mir-21_struc1 (16hp_rc_16hp) | mAmGmCmUmCmUmGmAmAmAmAmGmAmGmCmUmUmCmAmAmCmAm UmCmAmGmUmCmUmGmAmUmAmAmGmCmUmAmUmCmGmAmGmAmU mUmCmGmUmCmUmCmGmA |
| 10A | mir-21_struc2 (16hp_rc_16AR) | mAmGmCmUmCmUmGmAmAmAmAmGmAmGmCmUmUmCmAmAmCmAm UmCmAmGmUmCmUmGmAmUmAmAmGmCmUmAmUmUmGmUmAmCmC mUmAmCmUmCmUmCmGmA |
| 10A | mir-21_struc3 (16AR-rc_16hp) | mAmGmCmUmCmUmCmAmUmCmCmAmUmGmUmUmUmCmAmAmCmAm UmCmAmGmUmCmUmGmAmUm AmAmGmCmUmAmUmCmGmAmGmAmU mUmCmGmUmCmUmCmGmA |
| 10A | mir21_16ARB+RC+ 16ARB | mAmGmCmUmCmUmCmAmUmCmCmAmUmGmUmUmUmCmAmAmCmAm UmCmAmGmUmCmUmGmAmUmAmAmGmCmUmAmUmUmGmUmAmCmC mUmAmCmUmCmUmCmGmA |
| 10A | mir-21_RC | mUmCmAmAmCmAmUmCmAmGmUmCmUmGmAmUmAmAmGmCmUmA |
| 10B | let-7c_struc1 (16hp_rc_16hp) | mAmGmCmUmCmUmGmAmAmAmAmGmAmGmCmUmAmAmCmCmAmUmA mCmAmAmCmCmUmAmCmUmAmCmCmUmCmAmUmCmGmAmGmAmUmU mCmGmUmCmUmCmGmA |
| 10B | let-7c_struc2 (16hp_rc_16AR) | mAmGmCmUmCmUmGmAmAmAmAmGmAmGmCmUmAmAmCmCmAmUm AmCmAmAmCmCmUmAmCmUmAmCmCmUmCmAmUmUmGmUmAmCmCm |
| | | UmAmCmUmCmUmCmGmA |
| 10B | let-7c_struc3 (16AR-rc_16hp) | mAmGmCmUmCmUmCmAmUmCmCmAmUmGmUmUmAmAmCmCmAmU mAmCmAmAmCmCmUmAmCmUmAmCmCmUmCmAmUmCmGmAmGmA mUmUmCmGmUmCmUmCmGmA |
| 10B | let-7c_16ARB+RC+16A RB | mAmGmCmUmCmUmCmAmUmCmCmAmUmGmUmUmAmAmCmCmAmU mAmCmAmAmCmCmUmAmCmUmAmCmCmUmCmAmUmUmGmUmAmC mCmUmAmCmUmCmUmCmGmA |
| 10B | let-7c_RC | mAmAmCmCmAmUmAmCmAmAmCmCmUmAmCmUmAmCmCmUmCmA |
| | | |
| 11A | let-7c_struc4 (16AR_rc_HP) | mAmGmCmUmCmUmCmAmUmCmCmAmUmGmUmUmAmAmCmCmAmU mAmCmAmAmCmCmUmAmCmUmAmCmCmUmCmAmAmAmCmAmUmG mGmAmUmGmAmGmAmGmCmU |
| 11A | let-7c_16ARB+RC+16A RB | mAmGmCmUmCmUmCmAmUmCmCmAmUmGmUmUmAmAmCmCmAmU mAmCmAmAmCmCmUmAmCmUmAmCmCmUmCmAmUmUmGmUmAmC mCmUmAmCmUmCmUmCmGmA |
| 11A | let-7c_RC | mAmAmCmCmAmUmAmCmAmAmCmCmUmAmCmUmAmCmCmUmCmA |
| 11B | mir-21_struc4 (16AR_rc_HP) | mAmGmCmUmCmUmCmAmUmCmCmAmUmGmUmUmUmCmAmAmCmA mUmCmAmGmUmCmUmGmAmUmAmAmGmCmUmAmAmAmCmAmUmG mGmAmUmGmAmGmAmGmCmU |
| 11B | mir-21_16ARB+RC+16A RB | mAmGmCmUmCmUmCmAmUmCmCmAmUmGmUmUmUmCmAmAmCmA mUmCmAmGmUmCmUmGmAmUmAmAmGmCmUmAmUmUmGmUmAmC mCmUmAmCmUmCmUmCmGmA |
| 11B | mir-21_RC | mUmCmAmAmCmAmUmCmAmGmUmCmUmGmAmUmAmAmGmCmUmA |
| | | |
| 12A | mir-21_5pArm (two sequences annealed) | mCmAmUmGmGmAmUmGmAmGmAmGmCmU + mAmGmCmUmCmUmCmAmUmCmCmAmUmGmCmAmGmUmCmAmAmCm AmUmCmAmGmUmCmUmGmAmUmAmAmGmCmUmAmCmCmCmGmUmA mCmCmUmAmCmUmCmUmCmGmA |
| 12A | mir-21_miRIDIAN | mAmGmCmUmCmUmCmAmUmCmCmAmUmGmCmAmGmUmCmAmAmC mAmUmCmAmGmUmCmUmGmAmUmAmAmGmCmUmAmCmCmCmGmU mAmCmCmUmAmCmUmCmUmCmGmA |
| 12A | mir-21_3pArm (two sequences annealed) | mAmGmCmUmCmUmCmAmUmCmCmAmUmGmCmAmGmUmCmAmAmC mAmUmCmAmGmUmCmUmGmAmUmAmAmGmCmUmAmCmCmCmGmU mAmCmCmUmAmCmUmCmUmCmGmA + mUmCmGmAmGmAmGmUmAmGmGmUmAmC |
| 12A | mir21_5pArm+3pAr m (three sequences annealed) | mCmAmUmGmGmAmUmGmAmGmAmGmCmU + mAmGmCmUmCmUmCmAmUmCmCmAmUmGmCmAmGmUmCmAmAmC mAmUmCmAmGmUmCmUmGmAmUmAmAmGmCmUmAmCmCmCmGmU mAmCmCmUmAmCmUmCmUmCmGmA + m UmCmGmAmGmAmGmUmAmGmGmUmAmC |
| 12B | let-7c_5pArm (two sequences annealed) | mCmAmUmGmGmAmUmGmAmGmAmGmCmU + mAmGmCmUmCmUmCmAmUmCmCmAmUmGmCmUmAmAmAmCmCmA mUmAmCmAmAmCmCmUmAmCmUmAmCmCmUmCmAmAmCmCmGmU mAmCmCmUmAmCmUmCmUmCmGmA |
| 12B | let-7c_miRIDIAN | mAmGmCmUmCmUmCmAmUmCmCmAmUmGmCmUmAmAmAmCmCmA mUmAmCmAmAmCmCmUmAmCmUmAmCmCmUmCmAmAmCmCmGmU mAmCmCmUmAmCmUmCmUmCmGmA |
| 12B | let-7c_3pArm (two sequences annealed) | mAmGmCmUmCmUmCmAmUmCmCmAmUmGmCmUmAmAmAmCmCmA mUmAmCmAmAmCmCmUmAmCmUmAmCmCmUmCmAmAmCmCmGmU |
| | | mAmCmCmUmAmCmUmCmUmCmGmA + mUmCmGmAmGmAmGmUmAmGmGmUmAmC |
| 12B | let7c_5pArm+3pArm (three sequences annealed) | mCmAmUmGmGmAmUmGmAmGmAmGmCmU + mAmGmCmUmCmUmCmAmUmCmCmAmUmGmCmUmAmAmAmCmCmA mUmAmCmAmAmCmCmUmAmCmUmAmCmCmUmCmAmAmCmCmGmU mAmCmCmUmAmCmUmCmUmCmGmA + mUmCmGmAmGmAmGmUmAmGmGmUmAmC |
| | | |
| 13 | mir21_ds16AR+RC (two sequences annealed) | mAmAmCmAmUmGmGmAmUmGmAmGmAmGmCmU + mAmGmCmUmCmUmCmAmUmCmCmAmUmGmUmUmUmCmAmAmCmA mUmCmAmGmUmCmUmGmAmUmAmAmGmCmUmA |
| 13 | mir21_RC+ds16AR (two sequences annealed) | mUmCmAmAmCmAmUmCmAmGmUmCmUmGmAmUmAmAmGmCmUmAmU mUmGmUmAmCmCmUmAmCmUmCmUmCmGmA + mUmCmGmAmGmAmGmUmAmGmGmUmAmCmAmA |
| 13 | mir21_ds16AR+RC+ ds16AR (three sequences annealed) | mAmAmCmAmUmGmGmAmUmGmAmGmAmGmCmU + mAmGmCmUmCmUmCmAmUmCmCmAmUmGmUmUmUmCmAmAmCmAmU mCmAmGmUmCmUmGmAmUmAmAmGmCmUmA + mUmCmGmAmGmAmGmUmAmGmGmUmAmCmAmA |
| 13 | mir-21_RC | mUmCmAmAmCmAmUmCmAmGmUmCmUmGmAmUmAmAmGmCmUmA |
| 13 | mir21_16ARB+RC+ 16ARB | mAmGmCmUmCmUmCmAmUmCmCmAmUmGmUmUmUmCmAmAmCmAmU mCmAmGmUmCmUmGmAmUmAmAmGmCmUmAmUmUmGmUmAmCmCmU mAmCmUmCmUmCmGmA |
| | | |
| 14A | let-7c_miRIDIAN | mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA |
| 14A | let-7c_5pArm (two sequences annealed) | mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA |
| 14A | let7c_5pArm_5pChl (two sequences annealed) | 5'Chl-mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA |
| 14A | let7c-5pArm-3pChl (two sequences annealed) | mCmAmUmGmGmAmUmGmAmGmAmGmCmU3'-Chl + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA |
| 14A | let7c_3pArm_5pChl (two sequences annealed) | mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + 5'Chl- mUmCmGmAmGmAmGmUmAmGmGmUmAmC |
| 14A | let7c_3pArm_3pChl (two sequences annealed) | mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + mUmCmGmAmGmAmGmUmAmGmGmU mAmC3'-Chl |
| 14A | let7c_5pArm+3pArm (three sequences annealed) | mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC |
| | | mCmUmAmCmUmCmUmCmGmA + mUmCmGmAmGmAmGmUmAmGmGmU mAmC |
| 14A | let7c_5pArm+3pArm 5pChl (three sequences annealed) | mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + 5'Chl- mUmCmGmAmGmAmGmUmAmGmGmUmAmC |
| 14A | let7c_5pArm5pChl+ 3pArm (three sequences annealed) | 5'Chl-mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + mUmCmGmAmGmAmGmUmAmGmGmU mAmC |
| 14A | let7c_5pArm5pChl+ 3pArm5pChl (three sequences annealed) | 5'Chl-mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + 5'Chl- mUmCmGmAmGmAmGmUmAmGmGmUmAmC |
| 14B | mir-21_miRIDIAN | mAmGmCmUmCmUmCmAmUmCmCmAmUmGmCm AmGmUmCmAmAmCmA mUmCmAmGmUmCmUmGmAmUmAmAmGmCmUmAmCmCmCmGmUmAmC mCmUmAmCmUmCmUmCmGmA |
| 14B | mir-21_5pArm (two sequences annealed) | mCmAmUmGmGmAmUmGmAmGmAmGmCmU + mAmGmCmUmCmUmCmAmUmCmCmAmUmGmCm AmGmUmCmAmAmCmA mUmCmAmGmUmCmUmGmAmUmAmAmGmCmUmAmCmCmCmGmUmAmC mCmUmAmCmUmCmUmCmGmA |
| 14B | mir21_5pArm_5pChl (two sequences annealed) | 5'Chl-mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA |
| 14B | mir21_5pArm_3pChl (two sequences annealed) | mCmAmUmGmGmAmUmGmAmGmAmGmCmU-3'Chl + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA |
| 14B | mir-21_3pArm (two sequences annealed) | mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + mUmCmGmAmGmAmGmUmAmGmGmU mAmC |
| 14B | mir21_5pArm+3pAr m (three sequences annealed) | mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + mUmCmGmAmGmAmGmUmAmGmGmU mAmC |
| 14B | mir21 _5pArm+3pAr m5pChl (three sequences annealed) | mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + 5'Chl- mUmCmGmAmGmAmGmUmAmGmGmUmAmC |
| 14B | mir21_5pArm5pChl+ 3pArm (three sequences annealed) | 5'Chl-mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + mUmCmGmAmGmAmGmUmAmGmGmU |
| | | mAmC |
| 14B | mir21_5pArm5pChl+ 3pArm5pChl (three sequences annealed) | 5'Chl-mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + 5'Chl- mUmCmGmAmGmAmGmUmAmGmGmUmAmC |
| 14C | let-7c_miRIDIAN | mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA |
| 14C | let7c_SpArm+3pArm (three sequences annealed) | mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + mUmCmGmAmGmAmGmUmAmGmGmU mAmC |
| 14C | let7c_5pArm5pCy3+ 3pArm (three sequences annealed) | 5'Cy3-mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + mUmCmGmAmGmAmGmUmAmGmGmU mAmC |
| 14C | let7c_5pArm+3pArm 5pCy3 (three sequences annealed) | mCmAmUmGmGmAmUmGmAmGmAmGmCmU + mAmGmCmUmCmUmCmAmUmCmCmAmUmGmCmUmAmAmAmCmCmAmU mAmCmAmAmCmCmUmAmCmUmAmCmCmUmCmAmAmCmCmGmUmAmC mCmUmAmCmUmCmUmCmGmA + 5'Cy3- mUmCmGmAmGmAmGmUmAmGmGmUmAmC |
| 14D | mir-21_miRIDIAN | mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA |
| 14D | mir21_5pArm+3pAr m (three sequences annealed) | mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + mUmCmGmAmGmAmGmUmAmGmGmU mAmC |
| 14D | mir21_SpArm5pCy3 +3pArm (three sequences annealed) | 5'Cy3-mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + mUmCmGmAmGmAmGmUmAmGmGmU mAmC |
| 14D | mir21_5pArm+3pAr m5pCy3 (three sequences annealed) | mCmAmUmGmGmAmUmGmAmGmAmGmCmU + mAmGmCmUmCmUmCmAmUmCmCmAmUmGmCmAmGmUmCmAmAmCmA mUmCmAmGmUmCmUmGmAmUmAmAmGmCmUmAmCmCmCmGmUmAmC mCmUmAmCmUmCmUmCmGmA + 5'Cy3- mUmCmGmAmGmAmGmUmAmGmGmUmAmC |
| | | |
| 15A | mir21_8Y+RC+8Y | mUmCmUmCmUmUmCmUmUmCmAmAmCmAmUmCmAmGmUmCmUmGmAm UmAmAmGmCmUmAmUmCmUmUmCmUmCmU |
| 15A | mir-21_8Y hp+RC+8Y hp | mAmGmAmAmGmAmGmAmGmAmAmAmUmCmUmCmUmUmCmUmUmCmA mAmCmAmUmCmAmGmUmCmUmGmAmUmAmAmGmCmUmAmUmCmUmU mCmUmCmUmUmUmCmGmAmGmAmGmAmAmGmA |
| 15A | mir-21_16Y+RC+16Y | mCmUmCmUmUmCmUmCmUmCmUmCmUmUmCmUmUmCmAmAmCmAmU |
| | | mCmAmGmUmCmUmGmAmUmAmAmGmCmUmAmUmCmUmUmCmUmCmU mCmUmCmUmUmCmUmC |
| 15A | mir-21_16Y ds+RC+16Y ds | mAmGmAmAmGmAmGmAmGmAmGmAmAmGmAmG + mCmUmCmUmUmCmUmCmUmCmUmCmUmUmCmUmUmCmAmAmCmAmU mCmAmGmUmCmUmGmAmUmAmAmGmCmUmAmUmCmUmUmCmUmCmU mCmUmCmUmUmCmUmC + mGmAmGmAmAmGmAmGmAmGmAmGmAmA mGmA |
| 15A | mir-21_RC | mUmCmAmAmCmAmUmCmAmGmUmCmUmGmAmUmAmAmGmCmUmA |
| 15B | mir21_8A+RC+8A | mUmCmCmAmUmGmUmUmUmCmAmAmCmAmUmCmAmGmUmCmUmGmA mUmAmAmGmCmUmAmUmUmGmUmAmCmCmU |
| 15B | mir-21_8A hp+RC+8A hp | mAmAmCmAmUmGmGmAmGmAmAmAmUmCmCmAmUmGmUmUmUmCmA mAmCmAmUmCmAmGmUmCmUmGmAmUmAmAmGmCmUmAmUmUmGmU mAmCmCmUmUmUmCmGmAmGmGmUmAmCmAmA |
| 15B | mir21_16A+RC+16 A | mAmGmCmUmCmUmCmAmUmCmCmAmUmGmUmUmUmCmAmAmCmAmU mCmAmGmUmCmUmGmAmUmAmAmGmCmUmAmUmUmGmUmAmCmCmU mAmCmUmCmUmCmGmA |
| 15B | mir-21_16A ds+RC+16A ds | mAmAmCmAmUmGmGmAmUmGmAmGmAmGmCmU + mAmGmCmUmCmUmCmAmUmCmCmAmUmGmUmUmUmCmAmAmCmAmU mCmAmGmUmCmUmGmAmUmAmAmGmCmUmAmUmUmGmUmAmCmCmU mAmCmUmCmUmCmGmA + mUmCmGmAmGmAmGmUmAmGmGmUmAmC mAmA |
| 15B | mir-21_RC | mUmCmAmAmCmAmUmCmAmGmUmCmUmGmAmUmAmAmGmCmUmA |
| | | |
| 16 - RC mixture of six seqs | mir-17-5p_RC | mAmCmUmAmCmCmUmGmCmAmCmUmGmUmAmAmGmCmAmCmUmUmUmG |
| | mir-18a-5p_RC | mUmAmUmCmUmGmCmAmCmUmAmGmAmUmGmCmAmCmCmUmUmA |
| | mir-19a_RC | mUmCmAmGmUmUmUmUmGmCmAmUmAmGmAmUmUmUmGmCmAmCmA |
| | mir-20a_RC | mCmUmAmCmCmUmGmCmAmCmUmAmUmAmAmGmCmAmCmUmUmUmA |
| | mir-19b-1_RC | mUmCmAmGmUmUmUmUmGmCmAmUmGmGmAmUmUmUmGmCmAmCmA |
| | mir-92-1_RC | mCmAmGmGmCmCmGmGmGmAmCmAmAmGmUmGmCmAmAmUmA |
| 16 - 16+RC+ 16 mixture of six seqs | mir-17-5p_16RC +RC+ 16RC | mAmGmUmAmGmAmUmGmCmAmCmAmUmAmUmCmAmCmUmAmCmCmUmG mCmAmCmUmGmUmAmAmGmCmAmCmUmUmUmGmAmCmAmUmUmAmUmU mCmUmGmAmCmUmGmG |
| | mir-18a-5p_ 16RC +RC+ 16RC | mUmGmCmUmAmAmUmCmUmAmCmUmUmCmAmCmUmAmUmCmUmGmCmA mCmUmAmGm AmUmGmCmAmCmCmUmUmAmGmAmAmCmAmAmAmAmAmG mCmAmCmUmCmA |
| | mir-19a_16RC +RC+ 16RC | mAmAmAmUmAmGmCmAmGmGmCmCmAmCmCmAmUmCmAmGmUmUmUmU mGmCmAmUmAmGmAmUmUmUmGmCmAmCmAmAmCmUmAmCmAmUmUmC mUmUmCmUmUmGmU |
| | mir-20a_16RC +RC+ 16RC | mAmGmUmAmGmAmUmAmAmCmUmAmAmAmCmAmCmUmAmCmCmUmGmC mAmCmUmAmUmAmAmGmCmAmCmUmUmUmAmGmUmGmCmUmAmCmAmG mAmAmGmCmUmGmU |
| | mir-19b-1_16RC +RC+ 16RC | mCmUmUmUmUmCmAmCmUmAmCmCmAmCmAmGmUmCmAmGmUmUmUmU mGmCmAmUmGmGmAmUmUmUmGmCmAmCmAmGmCmAmGmAmAmUmAmU |
| | | mCmAmCmAmCmAmG |
| | mir-92-1_16RC +RC+ 16RC | mUmCmCmCmCmAmCmCmAmAmAmCmUmCmAmAmCmAmGmGmCmCmGmG mGmAmCmAmAmGmUmGmCmAmAmUmAmCmCmAmUmAmCmAmGmAmAmA mCmAmCmAmG |
| 16 - hp mixture of six seqs | mir-17-5p_struc1 (16hp_rc_16hp) | mAmGmCmUmCmUmGmAmAmAmAmGmAmGmCmUmAmCmUmAmCmCmUmG mCmAmCmUmGmUmAmAmGmCmAmCmUmUmUmGmUmCmGmAmGmAmUmU mCmGmUmCmUmCmGmA |
| | mir-18a5p_struc1 (16hp_rc_16hp) | mAmGmCmUmCmUmGmAmAmAmAmGmAmGmCmUmUmAmUmCmUmGmCmA mCmUmAmGmAmUmGmCmAmCmCmUmUmAmUmCmGmAmGmAmUmUmCmG mUmCmUmCmGmA |
| | mir-19a_struc1 (16hp_rc_16hp) | mAmGmCmUmCmUmGmAmAmAmAmGmAmGmCmUmUmCmAmGmUmUmUmU mGmCmAmUmAmGmAmUmUmUmGmCmAmCmAmUmCmGmAmGmAmUmUmC mGmUmCmUmCmGmA |
| | mir-20a_struc1 (16hp_rc_16hp) | mAmGmCmUmCmUmGmAmAmAmAmGmAmGmCmUmCmUmAmCmCmUmGmC mAmCmUmAmUmAmAmGmCmAmCmUmUmUmAmUmCmGmAmGmAmUmUmC mGmUmCmUmCmGmA |
| | mir-19b-1_struc1 (16hp_rc_16hp) | mAmGmCmUmCmUmGmAmAmAmAmGmAmGmCmUmUmCmAmGmUmUmUmU mGmCmAmUmGmGmAmUmUmUmGmCmAmCmAmUmCmGmAmGmAmUmUmC mGmUmCmUmCmGmA |
| | mir-92-1_struc1 (16hp_rc_16hp) | mAmGmCmUmCmUmGmAmAmAmAmGmAmGmCmUmCmAmGmGmCmCmGmG mGmAmCmAmAmGmUmGmCmAmAmUmAmUmCmGmAmGmAmUmUmCmGmU mCmUmCmGmA |
| | | |
| 11 | mir21_struc4_HP (2'-O-met) | mAmGmCmUmCmUmCmAmUmCmCmAmUmGmUmUmUmCmAmAmCmAmUmC mAmGmUmCmUmGmAmUmAmAmGmCmUmAmAmAmCmAmUmGmGmAmUmG mAmGmAmGmCmU |
| 11 | mir21_struc4RNA _HP (RNA stem) | AGCUCUCAUCCAUGUUmUmCmAmAmCmAmUmCmAmGmUmCmUmGmAmUmA mAmGmCmUmAAACAUGGAUGAGAGCU |
| 11 | mir21_struc5_HP (2'-O-Me) | mUmCmGmAmGmAmGmUmAmGmGmUmAmCmAmAmUmCmAmAmCmAmUmC mAmGmUmCmUmGmAmUmAmAmGmCmUmAmUmUmGmUmAmCmCmUmAmC mUmCmUmCmGmA |
| 11 | mir21_struc5RNA_H P (RNA stem) | UCGAGAGUAGGUACAAmUmCmAmAmCmAmUmCmAmGmUmCmUmGmAmUmA mAmGmCmUmAUUGUACCUACUCUCGA |
| | | |

| **miRNA** | **Figures** | **Sequence Inserted into the 3' UTR of Luciferase Gene** |
|---|---|---|
| hsa-let-7C | 10,11,12,14 | TCGAATGACCAACCATACAACCTACTACCTCACTCGAGCTGC(GGCC) |
| hsa-mir-21 | 10,11,12,13 14, 15, 17, 18 | TCGAATGACCTCAACATCAGTCTGATAAGCTACTCGAGCTGC(GGCC) |
| hsa-mir-21 ('attenuat ion site') | 18 | TCGAATGACCTCAACATCAGTCTGCTCTATAAGCTACTCGAGCTGC(GGCC) † |
| hsa-miR-17-5p | 16 | TCGAATGACCTCACTACCTGCACTGTAAGCACTTTGACCTCGAGCTGC(GGCC) |
| hsa-miR-18a | 16 | TCGAATGACCACTATCTGCACTAGATGCACCTTAGACTCGAGCTGC(GGCC) |
| hsa-miR-19a | 16 | TCGAATGACCCATCAGTTTGCATAGATTTGCACAACCTCGAGCTGC(GGCC) |
| hsa-miR-20a | 16 | TCGAATGACCCACTACCTGCACTATAAGCACTTTAGTCTCGAGCTGC(GGCC) |
| hsa-mir-19b-1 | 16 | TCGAATGACCAGTCAGTTTTGCATGGATTTGCACAGCCTCGAGCTGC(GGCC) |
| hsa-mir-92-1 | 16 | TCGAATGACCAACAGGCCGGGACAAGTGCAATACCCTCGAGCTGC(GGCC) |
| hsa-mir-22 | 16 | TCGAATGACCACAGTTCTTCAACTGGCAGCTTCTCGAGCTGC(GGCC) |
| hsa-mir-320 | 16 | TCGAATGACCTTCGCCCTCTCAACCCAGCTTTTCTCGAGCTGC(GGCC) |

The underlined sequence on the left is the 5' overhang on the sense strand (shown) that is the compatible cohesive end for the XhoI site. The antisense strand (not shown) will be the reverse complement of the remainder of the sense strand and will have a 5' overhang that is the reverse complement of the sequence shown underlined in parentheses to make the compatible cohesive end for the NotI site. Note that the original XhoI site is disabled by the replacement of the final G with an A. A new XhoI site (in bold) is introduced after the miRNA target site before the final NotI site. † The four nucleotides inserted to create an 'attenuation site' are indicated in non-bold. For multiple attenuation sites, the XhoI and NotI sites in the new insert are cut, and the identical insert is put in, this can be repeated as many times as desired to insert any number of sites.

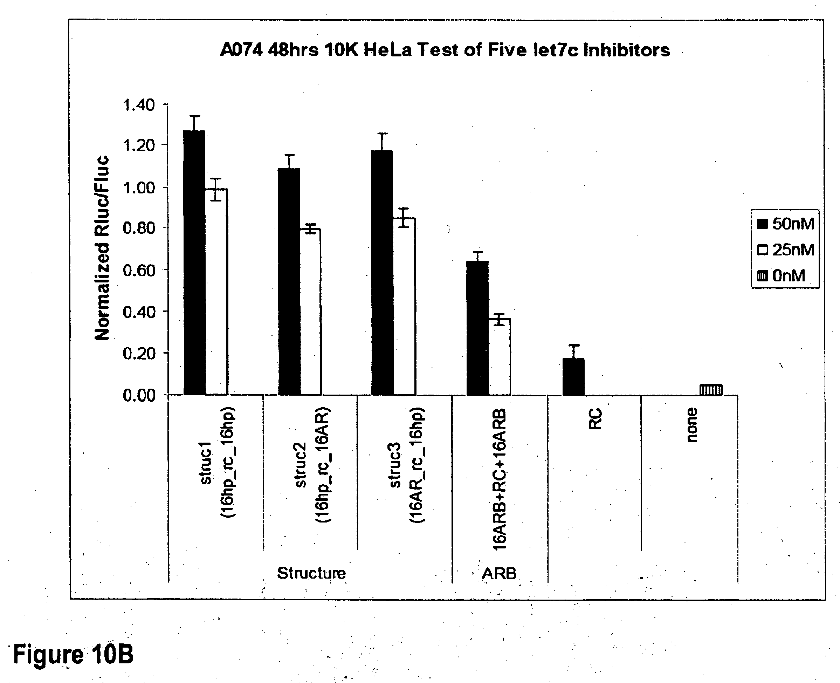

The results of the studies are found in Figures 10A and 10B. In both cases (e.g., miR21 and let7c), inhibitors designs in which 1) both the 5' and 3' flanking regions fold to form hairpins (*i.e*., structure 1), 2) the 5' flanking region folds to form hairpins (i.e., structure 2), and 3) the 3' flanking region folds to form a hairpin (i.e., structure 3), exhibited greater potency at 50 and 25 nM concentrations than inhibitors of equivalent length which did not form flanking region hairpins *(e.g.,* ARB), and short, reverse complement oligonucleotides (RC). Thus incorporation of duplexes into inhibitor molecules enhances overall functionality.

### Example 7

### Inhibitors Having Duplex Of Annealed 5' and 3' Flanking Sequences Exhibit Enhanced Functionality

To further test the effects of double stranded regions on inhibitor functionality, the 5' flanking and 3' flanking regions for both let7c and miR21 inhibitors were designed so that the respective sequences could anneal to each other. This design, also known as a lollipop design, contains a large loop region that includes the central region associated with a stem having a duplex region. As was the case in Example 1, all of the oligonucleotides were synthesized with a 2'-O-methyl modification at each position. Sequences for each of the inhibitors tested are found in Table 5. The functionality of each inhibitor design was then compared with 1) short reverse complement (e.g., RC, 22 nts in length) 2'-O-methyl inhibitors, and 2) longer inhibitor designs (*e.g.,* 54 nts) comprising the RC *(e.g.,* 22 nts) plus 5' and 3' flanking regions of equivalent length *(e.g.,* 16 nts) that do not anneal with each other *(e.g.,* ARB), using the dual luciferase assay.

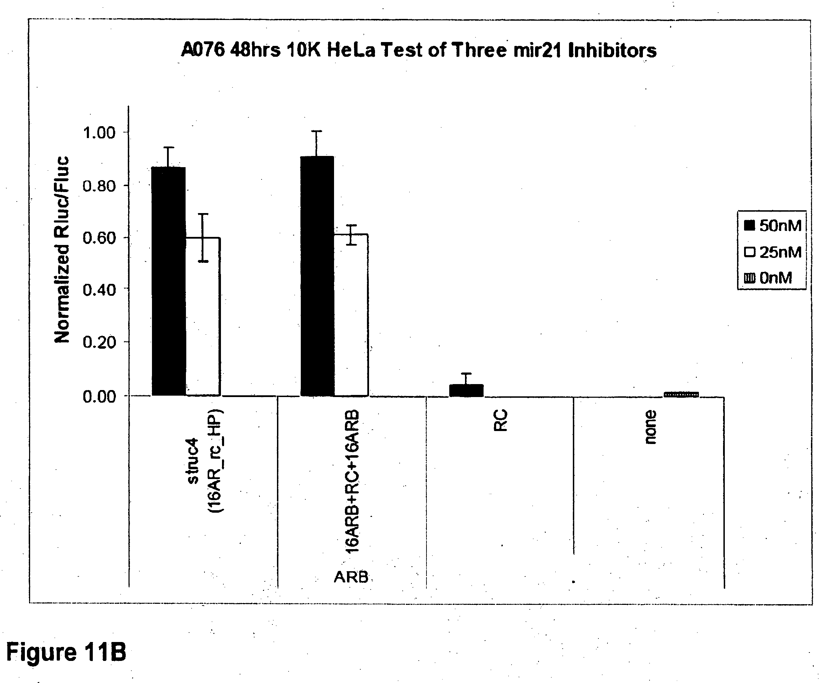

Let7c inhibitors having the lollipop design described above (e.g., structure 4) were more potent than non-annealing inhibitors of equivalent length (e.g., ARB) and short, reverse complement oligonucleotides (RC) at both of the concentrations tested (see Figure 11 A). In the case of miR21, both structure 4 and ARB designs were comparable, while shorter RC designs were considerably less potent (see Figure 11B). Subsequent folding studies using MFold (M. Zucker) revealed that while the Let7c inhibitor folded into a simple stem-loop structure having the central region unencumbered by secondary structure (Figure 11c), the miR21 inhibitor contained smaller (possibly dilatory) secondary structures within the larger loop (Figure 11D). Thus, while inhibitors having this design can in some cases exhibit enhanced functionality, the inventors believe this design is less than optimal due to secondary structures that can form in the key central region of the inhibitor.

### Example 8

### Duplex With Enhancer Sequences Boosts Inhibitor Functionality

To further test the effects of double stranded regions on inhibitor functionality, first oligonucleotide sequences containing central *(e.g.,* 22 nts), 5' flanking *(e.g.,* 14 nts), and 3' flanking (e.g., 14 nts) regions and targeting miR21 and let7c were designed and synthesized with complementary enhancer sequences capable of annealing to the 5' and/or 3' flanking regions. Subsequently, the functionality of inhibitors consisting of 1) the first oligonucleotide plus a first enhancer sequence, 2) first oligonucleotide plus a second enhancer sequence , or 3) the first oligonucleotide plus both a first and second enhancer sequences were compared with single stranded inhibitors of equivalent length. All of the oligonucleotides (*e.g*., first oligonucleotide, the first enhancer sequence, and the second enhancer sequence) were synthesized with a 2'-O-methyl modification at each position (see Table 5 for sequences) and the functionality of each design was compared using the dual luciferase assay. Smaller RC designs (e.g., 22 nt 2'-O-methylated molecules) were not considered due to the proven absence of functionality under the conditions of the assay (low concentrations, 48 hour time point).

The results for these studies are provided in Figures 12A and 12B. For miR21 studies, while the performance of the inhibitor containing the first oligonucleotide plus a second oligonucleotide capable of annealing to the 5' flanking sequence was equal to the long single stranded inhibitor (miRIDIAN), the inhibitors having 1) then first oligonucleotide plus an additional oligonucleotide capable of binding the 3' flanking sequence, and 2) the first oligonucleotide plus a second and third oligonucleotides capable of binding the 5' and 3' flanking sequences, out performed long single stranded inhibitor designs. Similar findings were observed for the let7c studies. Specifically, inhibitors having the first oligonucleotide (e.g., central region, 5' flanking region, and 3' flanking region) as well as enhancer sequences binding to both the 5' and 3' flanking sequences, out-performed the simpler, single stranded design. The findings of these studies further demonstrate that double stranded inhibitors exhibit superior performance over single stranded molecules.

### Example 9

### Introduction of Duplex Structures to Truncated Inhibitor Designs Boosts Inhibitor Functionality

To further test the effects of double stranded regions on inhibitor functionality, truncated first oligonucleotide sequences containing a central region targeting miR21 plus either a 5' or 3' flanking region targeting miR21 were synthesized along with complementary enhancer sequences to the appropriate region. Subsequently, truncated inhibitors annealed to the appropriate enhancer *(e.g.,* 5' flanking region-central region + the 5' enhancer sequence; or central region-3' flanking region + 3' flanking region) were compared to 1) full length first oligonucleotides (*e.g*., 5' flanking region- central region- 3' flanking region), 2) full length first oligonucleotides annealed to 5' and 3' enhancers, and 3) simple RC (central region) inhibitors. All of the oligonucleotides were synthesized with a 2'-O-Methyl modification at each position and are reported in Table 5.

The results for these studies are provided in Figure 13 and show that truncated inhibitors containing double stranded regions exhibit superior performance to short 2'-O-methyl modified inhibitors (RC) and long, modified, single stranded inhibitors with 5' and 3' flanking regions. Specifically, truncated double stranded inhibitors having either the 5' flank-central region orientation *(i.e.,* ds16AR+RC) or the central region-3' flank orientation (RC+ds16AR) both outperformed the short (RC) and long (16AR+RC+16AR) the single stranded inhibitors. These findings provide further demonstrate the enhanced functionality of double stranded inhibitor molecules.

### Example 10

### Addition of Conjugates to Double Stranded Inhibitors Boosts Functionality

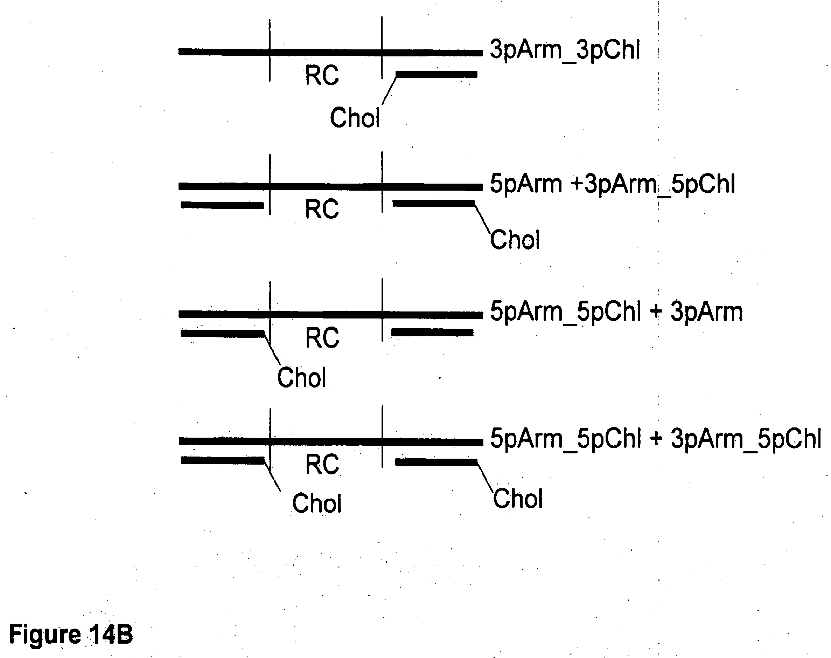

To test the efficacy of the double stranded inhibitor design in the context of hydrophobic conjugates, cholesterol or the fluorescent dye, Cy3, was conjugated to the 5' or 3' terminus of enhancer sequences that anneal to the flanking sequences of inhibitors targeting let-7c and miR21. As was the case in all previous experiments, all of the oligonucleotides were synthesized with a 2'-O-methyl modification at each position (see Table 5 for sequences) and overall functionality was assessed using the dual luciferase assay. All of the molecules were transfected into cells using standard lipid transfection protocols and schematic representations of each molecule are shown in Figures 14A and 14B.

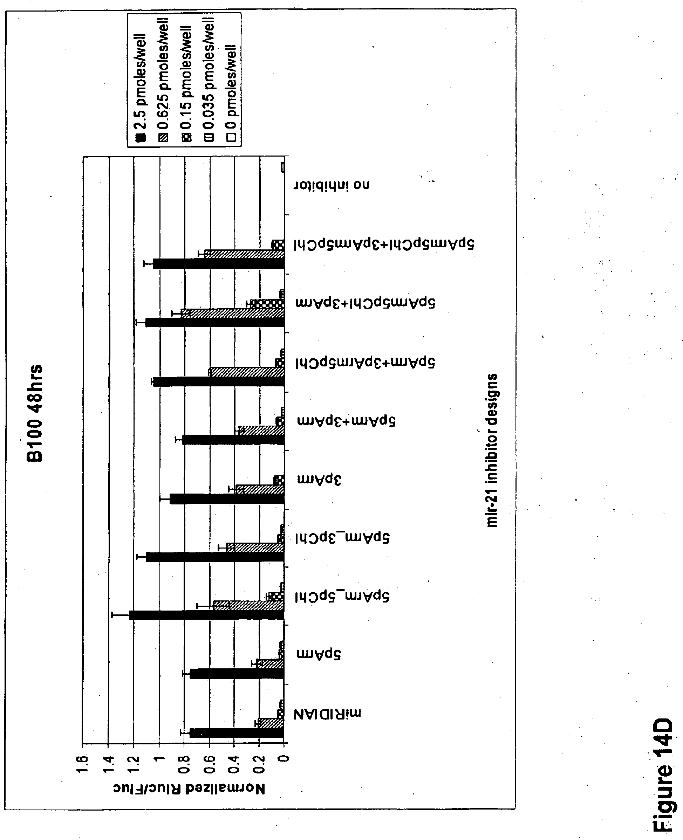

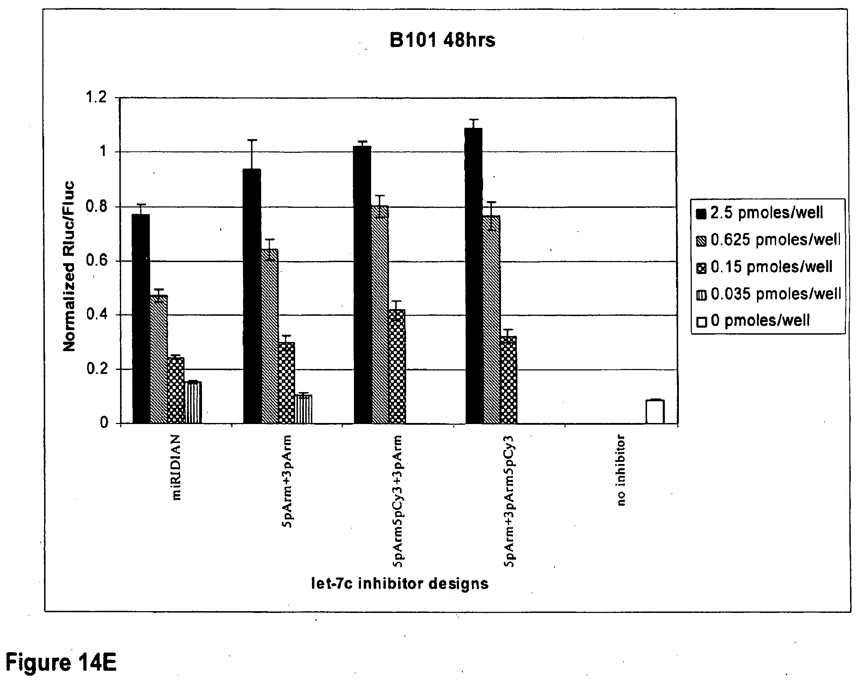

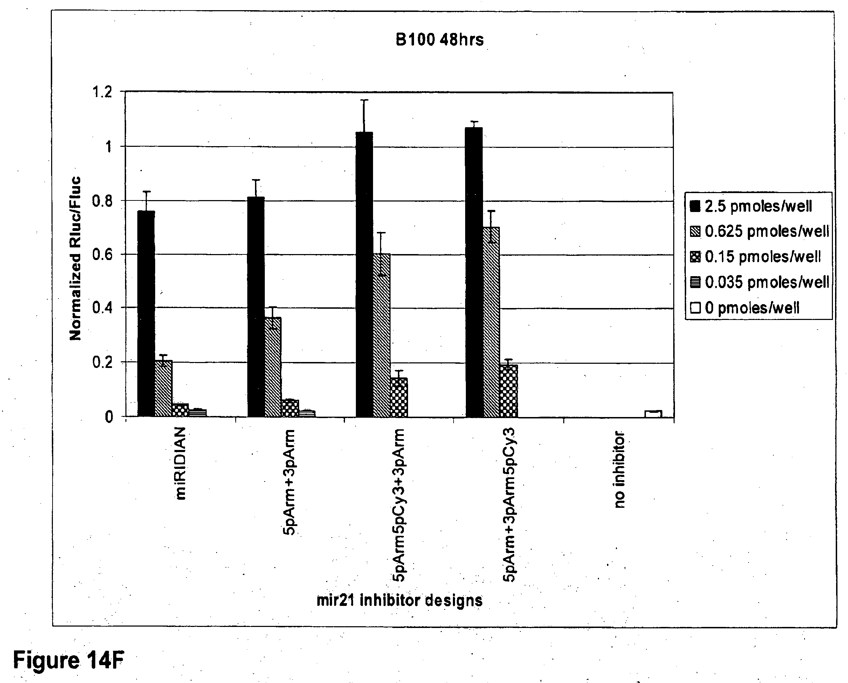

Figures 14C and 14D show the effects that conjugation of cholesterol to enhancer sequences has on overall inhibitor functionality. Compared to long single stranded inhibitors (miRIDIAN), double stranded inhibitors conjugated to cholesterol including those that contain the following: (1) a first oligonucleotide plus a 5' enhancer sequence with a 5' cholesterol modification (5pArm_5pChl), (2) a first oligonucleotide plus a 5' enhancer sequence with a 3' cholesterol modification (5pArm_3pChl), (3) a first oligonucleotide plus a 3' enhancer sequence with a 5' cholesterol modification (3pArm_5pChl), (4) a first oligonucleotide plus a 3' enhancer sequence with a 3' cholesterol modification (3pArm_3pChl), (5) a first oligonucleotide plus a 3' enhancer sequence with a 5' cholesterol modification plus a 5' enhancer sequence (5pArm+3pArm5pChl), (6) a first oligonucleotide plus a 5' enhancer sequence with a 5' cholesterol modification plus a 3' enhancer sequence (5pArm5pChl+3pArm), and (7) a first oligonucleotide plus both 3' and 5' enhancer sequences, both of which are modified with a 5' cholesterol modification, exhibit superior performance. Similar results were observed with Cy3 conjugates (Figure 14E and 14F).

### Example 11

### Incorporation of Double Stranded Regions Into Inhibitor Eliminates the Sequence-Dependence of Single Stranded Inhibitor Designs

Previous studies have shown that 1) the functionality of single stranded inhibitors is improved by incorporating flanking regions around the reverse complement of the target sequences (i.e., extending the length of the single stranded inhibitor), and 2) not all flanking sequences perform equally. Specifically, flanking sequences that are rich in polypyrimidine sequences were found to be less functional than sequences that more closely reflected mRNA (i.e., also referred to as "arbitrary sequences"). To determine whether these limitations were also a part of the double stranded design, the following designs were generated against miR21:
1. a single stranded inhibitor consisting of first oligonucleotide comprising an 8 nucleotide 5' flanking region consisting of a polypyrimidine sequence, a central region, and an 8 nucleotide 3' flanking region consisting of a polypyrimidine sequence.
2. a double stranded inhibitor consisting of first oligonucleotide comprising a polypyrimidine 5' flanking region that folds back upon itself to form a hairpin, a central region, a polypyrimidine 3' flanking region that folds back upon itself to form a hairpin.
3. a single stranded inhibitor consisting of first oligonucleotide comprising an 16 nucleotide 5' flanking region consisting of a polypyrimidine sequence, a central region, and an 16 nucleotide 3' flanking region consisting of a polypyrimidine sequence.
4. a double stranded inhibitor consisting of first oligonucleotide comprising an 16 nucleotide 5' flanking region consisting of a polypyrimidine sequence, a central region, and an 16 nucleotide 3' flanking region consisting of a polypyrimidine sequence, plus the appropriate first and second enhancer sequences that are capable of annealing to the 5' and 3' flanking sequences.

In addition, the four designs described above were also generated using "arbitrary sequences" in the flanking regions that mimic natural mRNA nucleotide content. As was the case in all previous experiments, all of the oligonucleotides were synthesized with a 2'-O-methyl modification at each position (see Table 5 for sequences) and overall functionality was assessed using the dual luciferase assay.

The results of these studies are presented in Figures 15A and 15B and demonstrate the following:
1. Single stranded inhibitors with polypyrimidine flanking regions exhibit poorer performance than those that have arbitrary sequences.
2. Double stranded inhibitors (generated by addition of enhancer sequences or by incorporation of hairpin designs in the flanking regions perform better than equivalent single stranded inhibitors.
3. Conversion of single stranded inhibitors to double stranded inhibitors eliminates the functional differences that result from flanking region sequence content.

These results demonstrate a novel attribute of the double stranded inhibitor design that is not present in single stranded designs.

### Example 12

### Double Stranded Inhibitors and Multi-miRNA Inhibition

To compare the functionality of single stranded inhibitors with double stranded inhibitors in the context of multigene targeting, three inhibitor designs (*e.g.*, simple single stranded RC designs, long single stranded designs, and inhibitors having 5' and 3' flanking hairpins) were synthesized to target six different miRNAs (*e.g*., miR17-5p, miR18a-5p, miR19a, miR20a, miR19b-1, and miR92-1). Subsequently, pools of each design were simultaneously co-transfected into HeLa cells (total concentration = 0.8 nM total) along with one of the six respective luciferase reporter constructs containing the appropriate target site in the 3' UTR. Results of these experiments (see Figure 16) show that while short and long single stranded inhibitors give highly variable results, double stranded inhibitors are consistently the most potent, highly functional design. For instance, at these concentrations, short (*e.g*., 21 nt RC) single stranded inhibitors provide adequate silencing of miR18a-5p and miR92-1. Long single stranded inhibitors silence an additional miRNA (miR17-5p), but fail to adequately silence miR19a, miR20a, and miR19b-1. In contrast, double stranded inhibitors simultaneously provide strong, specific, silencing of all the miRNAs tested. In addition, these results further demonstrate that the enhanced functionality related to this design is not restricted to e.g. miR21 or let7c, but in fact extends to a much broader population of miRNAs.

### Example 13

### Double Stranded Inhibitors That Contain A Mixture of Modified And Unmodified Nucleotides Exhibit Superior Performance

To test whether double stranded inhibitors containing mixtures of modified and unmodified nucleotides perform well, miR21-targeting inhibitors in which 1) the 3' flanking region was altered so as to promote annealing with the 5' flanking region (*e.g*., structure 4), or 2) the 5' flanking region was altered so as to promote annealing with the 3' flanking region (e.g., structure 5), were designed. In all cases the central region was modified with 2'-O-methyl groups, but designs deviated on the basis of whether the stem region was modified or unmodified (see Table 5 for sequences and modification patterns). In addition, unmodified single stranded inhibitors were not included in this study due to the lack of stability of these molecules.

The results of these experiments are presented in Figure 17 and demonstrate that double stranded inhibitors can consist of a heterogeneous population of modified and unmodified nucleotides. Specifically, partially modified structure 4 and structure 5 molecules performed far better than short, fully modified, single stranded inhibitors and nearly as well as their fully modified double stranded counterparts. These experiments demonstrate that the designs of the invention can accommodate mixtures of modified and unmodified nucleotides without dramatically altering the functionality of the molecule.

### Example 14

### Double Stranded Inhibitors Perform Equally Well In Both Translation Attenuation and Cleavage Assays

Reporter constructs were designed to determine whether double stranded inhibitors functioned to prevent both target cleavage and translation attenuation. To assess the inhibitor molecules ability to affect miRNA mediated cleavage, an exact complement to the miR21 target site was inserted into the 3' UTR of the Renilla luciferase gene of the psi-CHECK2 reporter. To determine the effectiveness of double stranded inhibitors on translation attenuation, one (or three) natural attenuation sites were cloned into the 3' UTR of the luciferase reporter gene (See Figure 18). Subsequently, simple, single stranded inhibitors (e.g., 21 nts) and double stranded inhibitors containing hairpin structures in the 5' and 3' flanking regions were synthesized and tested using assays designed to detect changes in protein (*e.g*., luciferase reporter assay) or mRNA (e.g., Branched DNA (BDNA) assay) expression (see Table 5 for sequences).

The results of these studies are presented in Figures 18A, 18B, and 18C and demonstrate the following:

In cases where a cleavage site was inserted into the 3' UTR of the reporter gene:
1. short single stranded inhibitors (InmiR21_RC) proved effective only at high concentrations in both B-DNA and luciferase assays.
2. double stranded inhibitors (miR21_struc1) exhibited strong performance over a range of concentrations as detected by both the BDNA-, and luciferase-based assays.

In cases where a single attenuation site was inserted into the 3' UTR of the reporter gene:
1. in the BDNA assay, neither short single stranded inhibitors or double stranded inhibitors exhibited significant levels of functionality as compared to controls.
2. in the protein assay, short, single stranded inhibitors functioned well at high concentrations, but exhibited significant losses in functionality at lower concentrations.
3. in the protein assay, double stranded inhibitors remained active at all concentrations tested.

In cases where three attenuation sites were inserted into the 3' UTR of the reporter genes:
1. in the BDNA assay, the short single stranded inhibitors exhibited only minor levels of activity at higher concentrations as compared to controls. In contrast, double stranded inhibitors exhibited higher levels of inhibition at all of the concentrations tested, suggesting that this novel inhibitor design is capable of preventing transcript degradation characteristic of this mode of action.
2. in the protein assay, short, single stranded inhibitors again functioned at higher concentrations, but exhibited a precipitous loss in functionality at lower concentrations. In contrast, double stranded inhibitors of the invention performed strongly at all of the concentrations tested.

These studies demonstrate the superior performance of double stranded inhibitors of the invention in both miRNA-based cleavage and translation attenuation mechanisms.

### Example 15

### Double Stranded Inhibitors Exhibit Greater Longevity Than Short Reverse Complement Inhibitor Designs

To examine the longevity of inhibition induced by 1) short 21 nt, 2'-O-methyl modified reverse complement (RC) inhibitors and 2) double stranded 2'-O-methyl inhibitors (e.g., hairpin design, 6 bp stems, 4 nt loops, 21 nt central region) molecules of both designs were synthesized (*e.g*., let7c target) and transfected into HeLa cells at 50 and 25 nM concentrations, respectively, with the appropriate reporter dual luciferase reporter construct. The higher concentrations used for RC designs were required due to the lower potency of these molecules.

As shown in Figure 19, inhibitors that utilize the hairpin design provide strong inhibition for periods up to 96 hrs at the lower (e.g., 25 nM) concentration. In contrast, simple RC designs are less potent, and lose all functionality at times between 48 and 72 hours. Therefore, an additional trait associated with the double stranded inhibitor design is improved longevity.

### Example 16

### Demonstration of C5-Chol Linker-Conjugate Technology to Inhibitor Designs

Double stranded inhibitors can be delivered to cells to inhibit the action of either an siRNA or miRNA. To test the efficacy of compositions of the invention in this context, an siRNA targeting PPIB and having a 3' C8 conjugated cholesterol on the sense strand (hPPIB #3 Sense: 5'-ACAGCAAAUUCCAUCGUGU (SEQ ID NO: 17)) was mixed with an inhibitor having the design shown in Figure 20A. HeLa cells (2,500 cells per well) were then transfected simultaneously with the inhibitor and the siRNA to measure the ability of the inhibitor to prevent knockdown of endogenous PPIB target by the siRNA (as measured by BDNA assays). Transfections were by passive delivery (*i.e.,* no lipid transfection reagent).

The results of these studies are shown in Figure 20B and demonstrate that in the absence of the inhibitor molecule, the siRNA knocks down its respective target by greater than 90% (see lane 1). Addition of the cholesterol conjugated inhibitor molecule (with or without the optional phosphorothioate modification) severely limits the ability of the siRNA to act (see lanes 2 and 3). Similar experiments with control, non-targeting inhibitors, or targeting inhibitors that are un-conjugated to cholesterol, fail to prevent the siRNA from knocking down its target. Thus, these experiments demonstrate the efficacy of forms of the inhibitor of present invention which contain a conjugate such as cholesterol.

### Example 17

### Passive Delivery of Inhibitor-Cholesterol Conjugates inhibits RNAi

To test whether cholesterol conjugated double stranded inhibitors could be passively delivered to inhibit RNAi, two different inhibitor molecules directed toward a DBI-targeting shRNA and having different patterns of cholesterol modification, were synthesized (see Figure 21 A). Subsequently, cells (HT1080 cells, 2,500 cells per well) that stably expressed an shRNA targeting DBI (NM_020548) were exposed to either inhibitor design (e.g., 1 micromolar) in the absence of any lipid delivery reagent and under low serum conditions ( Hy-Q Media, Figure 21B). Gene expression levels were measured at the 72 hr time point using branched DNA assays.

The results of these experiments are presented in Figure 21C and demonstrate that double stranded inhibitors having cholesterol conjugates can be passively delivered to cells to inhibit RNAi. While untreated cells exhibit less than 20% of the normal levels of DBI expression (due to the expressed hairpin), cells that have been treated with the conjugated inhibitor exhibit 40% of the normal levels, thus demonstrating that passively delivered inhibitor molecules effectively impede RNAi.

### Example 18

### Passive Delivery of Inhibitor-Cholesterol Conjugates Using Short Double Stranded Inhibitors

To test the effectiveness of short ds inhibitors in a passive delivery system 2.5K HT1080 cells that stably expressed an shRNA targeting DBI were plated in each well of a 96 well plate (DMEM + 10% serum). Twenty-four hours later, cells were exposed to a cholesterol conjugated inhibitor (e.g., 1 uM) directed against the DBI targeting shRNA construct in HyClone reduced serum media (sequence of modified strand: 5'-mG.*.mG.*.mA.mA.mU.mG.mA.mG.mC.mU.mG.mA.mA. mA.mG.mG.mG.mA.mC.mU.mU.mC.mC.mA.*.mA.*.mG.C5-Chol (SEQ ID NO: 18); sequence of unmodified strand: 5' CUUGGAAGUCCCUUUCAGCUCAUUCC (SEQ ID NO: 19); "m" = 2'-O-methyl modified nucleotide, "*" refers to phosphorothioate internucleotide linkage). Cells were then cultured for 72 hours, and then DBI expression was analyzed using the branched DNA assay.

The results of this work are shown in Figure 22. In untransfected cells, DBI expression is very low due to down regulation resulting from the DBI shRNA. In cells exposed to the cholesterol conjugated double stranded inhibitor, DBI expression is up-regulated by over three fold. These results demonstrate the effectiveness of passively delivered short double stranded inhibitors.

### Example 19

### Double Stranded miRNA Inhibitors Are Capable of Multi-miRNA Silencing.

To test the effectiveness of ds inhibitors to target multiple miRNA simultaneously, three inhibitor designs (e.g., reverse complement to the mature miRNA (RC), 54-nucleotide reverse complement (16+RC+16), and a hairpin-containing sequences) were tested for the ability to simultaneously silence all of the members of a polycistronic miRNA cluster encoding 6 separate miRNAs (Figure 23A, miR~17~18a~19a~20~19b~92, Lagos-Quintana et al., 2001; Lau et al., 2001). Both of the single-stranded inhibitor pools (RC and 16+RC+16) failed to inhibit all the miRNAs in the cluster (particularly in the cases of miR-19a and miR-19b-1). In contrast, the hairpin-containing inhibitor pool efficiently repressed the function of each of the targeted miRNAs (Figure 23B).

The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

We claim:
3 <110> APPLICANT: DHARMACON, INC.
   5 <120> TITLE OF INVENTION: COMPOSITIONS AND METHODS FOR INHIBITING GENE SILENCING 6 BY RNA INTERFERENCE
   8 <130> FILE REFERENCE: 16542.28.1.1a
   **C--> 10 <140> CURRENT APPLICATION NUMBER:** PCT/US07/04223
   **C--> 11 <141> CURRENT FILING DATE: 2007-02-27**
   13 <150> PRIOR APPLICATION NUMBER: 60/870,815
   14 <151> PRIOR FILING DATE: 2006-12-19
   16 <150> PRIOR APPLICATION NUMBER: 60/865,508
   17 <151> PRIOR FILING DATE: 2006-11-13
   19 <150> PRIOR APPLICATION NUMBER: 60/826,702
   20 <151> PRIOR FILING DATE: 2006-09-22
   22 <150> PRIOR APPLICATION NUMBER: 60/774,350
   23 <151> PRIOR FILING DATE: 2006-02-17
   25 <160> NUMBER OF SEQ ID NOS: 24
   27 <170> SOFTWARE: PatentIn version 3.3
29 <210> SEQ ID NO: 1
   30 <211> LENGTH: 22
   31 <212> TYPE: RNA
   32 <213> ORGANISM: Artificial Sequence
   34 <220> FEATURE:
   35 <223> OTHER INFORMATION: Description of Artificial Sequence: Synthetic 36 oligonucleotide
38 <400> SEQUENCE: 1
   39 aaccauacaa ccuacuaccu ca 22
42 <210> SEQ ID NO: 2
   43 <211> LENGTH: 22
   44 <212> TYPE: RNA
   45 <213> ORGANISM: Artificial Sequence
   47 <220> FEATURE:
   48 <223> OTHER INFORMATION: Description of Artificial Sequence: Synthetic 49 oligonucleotide
51 <400> SEQUENCE: 2
   52 ucaacaucag ucugauaagc ua 22
55 <210> SEQ ID NO: 3
   56 <211> LENGTH: 22
   57 <212> TYPE: RNA
   58 <213> ORGANISM: Artificial Sequence
   60 <220> FEATURE:
   61 <223> OTHER INFORMATION: Description of Artificial Sequence: Synthetic 62 oligonucleotide

64 <400> SEQUENCE: 3
   65 acaguucuuc aacuggcagc uu 22
68 <210> SEQ ID NO: 4
   69 <211> LENGTH: 14
   70 <212> TYPE: RNA
   71 <213> ORGANISM: Artificial Sequence
   73 <220> FEATURE:
   74 <223> OTHER INFORMATION: Description of Artificial Sequence: Synthetic 75 oligonucleotide
77 <400> SEQUENCE: 4
   78 agcucucauc caug 14
81 <210> SEQ ID NO: 5
   82 <211> LENGTH: 14
   83 <212> TYPE: RNA
   84 <213> ORGANISM: Artificial Sequence
   86 <220> FEATURE:
   87 <223> OTHER INFORMATION: Description of Artificial Sequence: Synthetic 88 oligonucleotide
90 <400> SEQUENCE: 5
   91 guaccuacuc ucga 14
94 <210> SEQ ID NO: 6
   95 <211> LENGTH: 53
   96 <212> TYPE: RNA
   97 <213> ORGANISM: Artificial Sequence
   99 <220> FEATURE:
   100 <223> OTHER INFORMATION: Description of Artificial Sequence: Synthetic 101 oligonucleotide
103 <400> SEQUENCE: 6
   104 ucgagaguag guacaaaacc aacaaccuac uaccucauug uaccuacucu cga 53
107 <210> SEQ ID NO: 7
   108 <211> LENGTH: 54
   109 <212> TYPE: RNA
   110 <213> ORGANISM: Artificial Sequence
   112 <220> FEATURE:
   113 <223> OTHER INFORMATION: Description of Artificial Sequence: Synthetic 114 oligonucleotide
116 <400> SEQUENCE: 7
   117 ucgagaguag guacaaucaa caucagucug auaagcuauu guaccuacuc ucga 54
120 <210> SEQ ID NO: 8
   121 <211> LENGTH: 56
   122 <212> TYPE: RNA
   123 <213> ORGANISM: Artificial Sequence
   125 <220> FEATURE:
   126 <223> OTHER INFORMATION: Description of Artificial Sequence: Synthetic
   127 oligonucleotide
129 <400> SEQUENCE: 8
   130 agcucucauc caugaaaaac agcaaauucc aucguguaau caguaccuac ucucga 56
133 <210> SEQ ID NO: 9
   134 <211> LENGTH: 14
   135 <212> TYPE: RNA
   136 <213> ORGANISM: Artificial Sequence
   138 <220> FEATURE:
   139 <223> OTHER INFORMATION: Description of Artificial Sequence: Synthetic
   140 oligonucleotide
142 <400> SEQUENCE: 9
   143 cauggaugag agcu 14
146 <210> SEQ ID NO: 10
   147 <211> LENGTH: 14
   148 <212> TYPE: RNA
   149 <213> ORGANISM: Artificial Sequence
   151 <220> FEATURE:
   152 <223> OTHER INFORMATION: Description of Artificial Sequence: Synthetic
   153 oligonucleotide
155 <400> SEQUENCE: 10
   156 ucgagaguag guac 14
159 <210> SEQ ID NO: 11
   160 <211> LENGTH: 56
   161 <212> TYPE: RNA
   162 <213> ORGANISM: Artificial Sequence
   164 <220> FEATURE:
   165 <223> OTHER INFORMATION: Description of Artificial Sequence: Synthetic
   166 oligonucleotide
168 <400> SEQUENCE: 11
   169 agcucucauc cauguggaau gagcugaaag ggacuuccaa guguaccuac ucucga 56
172 <210> SEQ ID NO: 12
   173 <211> LENGTH: 56
   174 <212> TYPE: RNA
   175 <213> ORGANISM: Artificial Sequence
   177 <220> FEATURE:
   178 <223> OTHER INFORMATION: Description of Artificial Sequence: Synthetic
   179 oligonucleotide
181 <400> SEQUENCE: 12
   182 agcucucauc cauguaaaac uauacaaccu acuaccucau ccguaccuac ucucga 56
185 <210> SEQ ID NO: 13
   186 <211> LENGTH: 42
   187 <212> TYPE: DNA
   188 <213> ORGANISM: Artificial Sequence
   190 <220> FEATURE:
   191 <223> OTHER INFORMATION: Description of Artificial Sequence: Synthetic
   192 oligonucleotide
194 <400> SEQUENCE: 13
   195 tcgaatgacc aaccatacaa cctactacct cactcgagct gc 42
198 <210> SEQ ID NO: 14
   199 <211> LENGTH: 42
   200 <212> TYPE: DNA
   201 <213> ORGANISM: Artificial Sequence
   203 <220> FEATURE:
   204 <223> OTHER INFORMATION: Description of Artificial Sequence: Synthetic
   205 oligonucleotide
207 <400> SEQUENCE: 14
   208 tcgaatgacc tcaacatcag tctgataagc tactcgagct gc 42
211 <210> SEQ ID NO: 15
   212 <211> LENGTH: 56
   213 <212> TYPE: RNA
   214 <213> ORGANISM: Artificial Sequence
   216 <220> FEATURE:
   217 <223> OTHER INFORMATION: Description of Artificial Sequence: Synthetic
   218 oligonucleotide
220 <400> SEQUENCE: 15
   221 agcucucauc caugcuuuga uagcccugua caaugcugcu ugguaccuac ucucga 56
224 <210> SEQ ID NO: 16
   225 <211> LENGTH: 54
   226 <212> TYPE: RNA
   227 <213> ORGANISM: Artificial Sequence
   229 <220> FEATURE:
   230 <223> OTHER INFORMATION: Description of Artificial Sequence: Synthetic
   231 oligonucleotide
233 <400> SEQUENCE: 16
   234 aagcucucug ugcuuugaua gcccuguaca augcugcuug aacuccaugc caca 54
237 <210> SEQ ID NO: 17
   238 <211> LENGTH: 19
   239 <212> TYPE: RNA
   240 <213> ORGANISM: Artificial Sequence
   242 <220> FEATURE:
   243 <223> OTHER INFORMATION: Description of Artificial Sequence: Synthetic
   244 oligonucleotide
246 <400> SEQUENCE: 17
   247 acagcaaauu ccaucgugu 19
250 <210> SEQ ID NO: 18
   251 <211> LENGTH: 26
   252 <212> TYPE: RNA
   253 <213> ORGANISM: Artificial Sequence
   255 <220> FEATURE:
   256 <223> OTHER INFORMATION: Description of Artificial Sequence: Synthetic
   257 oligonucleotide
259 <400> SEQUENCE: 18
   260 ggaaugagcu gaaagggacu uccaag 26
263 <210> SEQ ID NO: 19
   264 <211> LENGTH: 26
   265 <212> TYPE: RNA
   266 <213> ORGANISM: Artificial Sequence
   268 <220> FEATURE:
   269 <223> OTHER INFORMATION: Description of Artificial Sequence: Synthetic
   270 oligonucleotide
272 <400> SEQUENCE: 19
   273 cuuggaaguc ccuuucagcu cauucc 26
276 <210> SEQ ID NO: 20
   277 <211> LENGTH: 28
   278 <212> TYPE: RNA
   279 <213> ORGANISM: Artificial Sequence
   281 <220> FEATURE:
   282 <223> OTHER INFORMATION: Description of Artificial Sequence: Synthetic
   283 oligonucleotide
285 <400> SEQUENCE: 20
   286 uaaaacuaua caaccuacua ccucaucc 28
289 <210> SEQ ID NO: 21
   290 <211> LENGTH: 28
   291 <212> TYPE: RNA
   292 <213> ORGANISM: Artificial Sequence
   294 <220> FEATURE:
   295 <223> OTHER INFORMATION: Description of Artificial Sequence: Synthetic
   296 oligonucleotide
298 <400> SEQUENCE: 21
   299 cuaaacuaua caaccuacua ccucaacc 28
302 <210> SEQ ID NO: 22
   303 <211> LENGTH: 28
   304 <212> TYPE: RNA
   305 <213> ORGANISM: Artificial Sequence
   307 <220> FEATURE:
   308 <223> OTHER INFORMATION: Description of Artificial Sequence: Synthetic
   309 oligonucleotide
311 <400> SEQUENCE: 22
   312 ccaaacuaua caaccuacua ccucaccc 28
315 <210> SEQ ID NO: 23
   316 <211> LENGTH: 28
   317 <212> TYPE: RNA
   318 <213> ORGANISM: Artificial Sequence
   320 <220> FEATURE:
   321 <223> OTHER INFORMATION: Description of Artificial Sequence: Synthetic
   322 oligonucleotide
324 <400> SEQUENCE: 23
   325 ugaaaccaca caaccuacua ccucaccc 28
328 <210> SEQ ID NO: 24
   329 <211> LENGTH: 28
   330 <212> TYPE: RNA
   331 <213> ORGANISM: Artificial Sequence
   333 <220> FEATURE:
   334 <223> OTHER INFORMATION: Description of Artificial Sequence: Synthetic
   335 oligonucleotide
337 <400> SEQUENCE: 24
   338 cuaaaccaua caaccuacua ccucaacc 28

## Claims

1. A miRNA inhibitor comprising:
a first oligonucleotide comprising:
a central region having 3' and 5' ends, and having a central sequence that is from about 6 to about 37 nucleotides and having at least about 80% complementarity with a target RNA sequence, wherein the central region is single stranded, and the target RNA sequence is capable of silencing a target gene;
a 5' flanking region that comprises a duplex region; and
a 3' flanking region that comprises a duplex region
wherein the target RNA sequence is selected from the group consisting of a region of RISC-entering strand of a miRNA, a region of pre-miRNA, and a mature region and regions bordering the mature region of pri-miRNA and the miRNA inhibitor is capable of being recognised by RISC and other proteins associated with RNAi machinery.

2. A miRNA inhibitor as in claim 1, wherein the target RNA sequence is a mature miRNA sequence.

3. A miRNA inhibitor as in claim 1, wherein the duplex region within the 5' flanking region and the duplex region within the 3' flanking region, each is about 10 nucleotides to about 40 nucleotides.

4. A miRNA inhibitor as in claims 1 or 3, wherein the duplex region within the 5' flanking region and the duplex region within the 3' flanking region are each part of a hairpin structure.

5. A miRNA inhibitor as in claim 4, wherein the duplex region within each of the 5' flanking region and the 3' flanking region includes consecutive nucleotides.

6. A miRNA inhibitor as in claim 4 or claim 5, wherein the hairpin structure of each of the 5' flanking region and the 3' flanking region has a loop of about 4-15 nucleotides.

7. A miRNA inhibitor as in claim 6, wherein the central region includes about 21-28 nucleotides and the 3' and 5' flanking regions each includes a hairpin structure, wherein each duplex region is about 6 base pairs and each loop is about 4-10 nucleotides.

8. A miRNA inhibitor as in any of claims 1, or 3-7, wherein at least one of the central region, 3' flanking or 5' flanking region comprises at least one nucleotide having a 2' modification.

9. A miRNA inhibitor as in claim 8, wherein the 2' modification is a 2'-O-alkyl, 2' orthoester, or 2' ACE.

10. A miRNA inhibitor as in claim 8 or claim 9, wherein at least about 30% of nucleotides in the miRNA inhibitor have the 2' modification.

11. A miRNA inhibitor as in claim 10, wherein about 100% of nucleotides in the miRNA inhibitor have the 2' modification.

12. A miRNA inhibitor as in any of claims 8-10, wherein the central region includes a higher percentage of nucleotides with the 2' modification compared to the flanking regions.

13. A miRNA inhibitor as in any claims 1, or 3-12, wherein said 3' flanking region and 5' flanking region are comprised of not more than about 70% pyrimidine nucleotides.

14. A method of inhibiting an RNAi pathway in a cell so as to inhibit a target gene from being silenced by a target RNA sequence, the method comprising:
providing a cell capable of expressing the target RNA sequence;
introducing a miRNA inhibitor according to any of claims 1 to 13 or 15 into the cell; and
maintaining the cell under conditions in which the silencing of the target gene by the target miRNA is inhibited, wherein said method is not a method for treatment of the human body by surgery or therapy or a diagnostic method practised on the human or animal body.

15. A miRNA inhibitor comprising:
a first oligonucleotide comprising a central region having 3' and 5' ends, and having a central sequence that is from about 6 to about 37 nucleotides and having at least about 80% complementarity with a target RNA sequence, wherein the central region is single stranded and the target RNA sequence is capable of silencing a target gene and wherein the target RNA sequence is selected from the group consisting of a region of RISC-entering strand of a miRNA, a region of pre-miRNA, and a mature region and regions bordering the mature region of pri-miRNA;
a 5' flanking region that comprises a duplex with a first enhancer sequence that is greater than 8 and up to 35 nucleotides in length, and
a 3' flanking region that comprises a duplex with a second enhancer sequence that is greater than 8 and up to 35 nucleotides in length.

## Patentansprüche

1. miRNA-Hemmer, umfassend:
ein erstes Oligonukleotid, umfassend:
einen zentralen Bereich mit 3'- und 5'-Enden und mit einer zentralen Sequenz von etwa 6 bis etwa 37 Nukleotiden und mit einer Komplementarität von wenigstens etwa 80% mit einer Ziel-RNA-Sequenz, wobei der zentrale Bereich einsträngig ist, und wobei die Ziel-RNA-Sequenz ein Zielgen stummschalten kann;
einen 5' flankierenden Bereich, der einen Duplexbereich umfasst; und
einen 3' flankierenden Bereich, der einen Duplexbereich umfasst,
wobei die Ziel-RNA-Sequenz ausgewählt ist aus der Gruppe bestehend aus einem Bereich eines RISC-eintretenden Strangs einer miRNA, einem Bereich von Pre-miRNA und einem reifen Bereich und Bereichen, die an den reifen Bereich von Pri-miRNA angrenzen, und wobei der miRNA-Hemmer durch RISC und andere dem RNAi-Mechanismus assoizierte Proteine erkannt werden kann.

2. miRNA-Hemmer nach Anspruch 1, wobei die Ziel-RNA-Sequenz eine reife miRNA-Sequenz ist.

3. miRNA-Hemmer nach Anspruch 1, wobei der Duplexbereich in dem 5' flankierenden Bereich liegt, und wobei der Duplexbereich in dem 3' flankierenden Bereich liegt, wobei jeder etwa 10 Nukleotide bis etwa 40 Nukleotide aufweist.

4. miRNA-Hemmer nach Anspruch 1 oder 3, wobei der Duplexbereich in dem 5' flankierenden Bereich und der Duplexbereich in dem 3' flankierenden Bereich jeweils Teil einer Haarnadelstruktur sind.

5. miRNA-Hemmer nach Anspruch 4, wobei der Duplexbereich in dem 5' flankierenden Bereich und in dem 3' flankierenden Bereich konsekutive Nukleotide aufweist.

6. miRNA-Hemmer nach Anspruch 4 oder 5, wobei die Haarnadelstruktur des 5' flankierenden Bereichs und des 3' flankierenden Bereichs jeweils eine Schleife von etwa 4 bis 15 Nukleotiden aufweist.

7. miRNA-Hemmer nach Anspruch 6, wobei der zentrale Bereich etwa 21 - 28 Nukleotide aufweist, und wobei die 3' und 5' flankierenden Bereiche jeweils eine Haarnadelstruktur aufweisen, wobei jeder Duplexbereich etwa 6 Basenpaaren entspricht und jede Schleife etwa 4-10 Nukleotide aufweist.

8. miRNA-Hemmer nach einem der Ansprüche 1 oder 3 bis 7, wobei wenigstens ein Bereich des zentralen Bereichs, des 3' flankierenden Bereichs oder des 5' flankierenden Bereichs wenigstens ein Nukleotid mit einer 2' Modifikation umfasst.

9. miRNA-Hemmer nach Anspruch 8, wobei die 2' Modifikation ein 2'-O-Alkyl, 2'-Orthoester oder 2'-ACE ist.

10. miRNA-Hemmer nach Anspruch 8 oder 9, wobei wenigstens etwa 30% der Nukleotide in dem miRNA-Hemmer die 2' Modifikation aufweisen.

11. miRNA-Hemmer nach Anspruch 10, wobei etwa 100% der Nukleotide in dem miRNA-Hemmer die 2' Modifikation aufweisen.

12. miRNA-Hemmer nach einem der Ansprüche 8 - 10, wobei der zentrale Bereich einen höheren prozentualen Anteil an Nukleotiden mit der 2' Modifikation aufweist als die flankierenden Bereiche.

13. miRNA-hemmer nach einem der Ansprüche 1 oder 3 bis 12, wobei der 3' flankierende Bereich und der 5' flankierende Bereich nicht mehr als etwa 70% Pyrimidinnukleotide umfassen.

14. Verfahren zum Hemmen eines RNAi-Pfads in einer Zelle, um es zu hemmen, dass ein Zielgen durch eine Ziel-RNA-Sequenz stummgeschaltet wird, wobei das Verfahren folgendes umfasst:
Bereitstellen einer Zelle, welche die Ziel-RNA-Sequenz exprimieren kann;
Einführen eines miRNA-Hemmers naach einem der Ansprüche 1 bis 13 oder 15 in die Zelle; und
Aufrechterhalten der Zelle unter Bedingungen, unter denen die Stummschaltung des Zielgens durch die Ziel-miRNA gehemmt ist, wobei das Verfahren kein Verfahren zur Behandlung des menschlichen Körpers durch Chirurgie oder Behandlung oder ein diagnostisches Verfahren ist, ausgeführt an dem Körper eines Menschen oder Tieres.

15. miRNA-Hemmer, umfassend:
ein erstes Oligonukleotid, umfassend einen zentralen Bereich mit 3'- und 5'-Enden und mit einer zentralen Sequenz von etwa 6 bis etwa 37 Nukleotiden und mit einer Komplementarität von wenigstens etwa 80% mit einer Ziel-RNA-Sequenz, wobei der zentrale Bereich einsträngig ist, und wobei die Ziel-RNA-Sequenz ein Zielgen stummschalten kann, und wobei die Ziel-RNA-Sequenz ausgewählt ist aus der Gruppe bestehend aus einem Bereich eines RISC-eintretenden Strangs einer miRNA, einem Bereich von Pre-miRNA und einem reifen Bereich und Bereichen, die an den reifen Bereich von Pri-miRNA angrenzen;
einen 5' flankierenden Bereich, der einen Duplexbereich umfasst, mit einer ersten Enhancer-Sequenz, die eine Länge von mehr als 8 und bis zu 35 Nukleotiden aufweist; und
einen 3' flankierenden Bereich, der einen Duplexbereich umfasst, mit einer zweiten Enhancer-Sequenz, die eine Länge von mehr als 8 und bis zu 35 Nukleotiden aufweist.

## Revendications

1. Inhibiteur d'ARNmi comprenant :
un premier oligonucléotide comprenant :
une région centrale possédant des extrémités 3' et 5', et possédant une séquence centrale qui est comprise entre environ 6 et environ 37 nucléotides et possédant au moins une complémentarité de 80% avec une séquence d'ARN cible, la région centrale étant à simple brin, et la séquence d'ARN cible étant capable de mettre sous silence un gène cible ;
une région flanquante 5' qui comprend une région duplexe ; et
une région flanquante 3' qui comprend une région duplex la séquence d'ARN cible étant choisie parmi le groupe constitué d'une région de brin entrant RISC d'un ARNmi, une région de pré-ARNmi, et une région mature et des régions bordant la région mature du pri-ARNmi et l'inhibiteur d'ARNmi étant capable d'être reconnu par RISC et d'autres protéines associées avec la machinerie d'ARNi.

2. Inhibiteur d'ARNmi selon la revendication 1, dans lequel la séquence d'ARN cible est une séquence d'ARNmi mature.

3. Inhibiteur d'ARNmi selon la revendication 1, dans lequel la région duplexe au sein de la région flanquante 5' et la région duplexe au sein de la région flanquante 3', chaque région est comprise entre environ 10 et environ 40 nucléotides.

4. Inhibiteur d'ARNmi selon l'une quelconque des revendications 1 à 3, dans lequel la région duplexe au sein de la région flanquante 5' et la région duplexe au sein de la région flanquante 3' font chacune partie d'une structure en tête d'épingle.

5. Inhibiteur d'ARNmi selon la revendication 4, dans lequel la région duplexe au sein de chacune de la région flanquante 5' et de la région flanquante 3' inclut des nucléotides consécutifs.

6. Inhibiteur d'ARNmi selon la revendication 4 ou la revendication 5, dans lequel la structure en tête d'épingle de chacune de la région flanquante 5' et de la région flanquante 3' possède une boucle d'environ 4 à 15 nucléotides.

7. Inhibiteur d'ARNmi selon la revendication 6, dans lequel la région centrale inclut environ entre 21 et 28 nucléotides et les régions flanquantes 3' et 5' incluent chacune une structure en tête d'épingle, chaque région duplexe étant d'environ 6 paires de bases et chaque boucle étant d'environ entre 4 et 10 nucléotides.

8. Inhibiteur d'ARNmi selon l'une quelconque des revendications 1, ou de 3 à 7, dans lequel au moins une de la région centrale, de la région flanquante 3' ou de la région flanquante 5' comprend au moins un nucléotide possédant une modification 2'.

9. Inhibiteur d'ARNmi selon la revendication 8, dans lequel la modification 2' est un 2'-O-alkyle, 2'-othoester, ou 2' ACE.

10. Inhibiteur d'ARNmi selon la revendication 8 ou la revendication 9, dans lequel au moins environ 30% des nucléotides dans l'inhibiteur d'ARNmi possèdent la modification 2'.

11. Inhibiteur d'ARNmi selon la revendication 10, dans lequel environ 100% des nucléotides dans l'inhibiteur d'ARNmi possèdent la modification 2'.

12. Inhibiteur d'ARNmi selon l'une quelconque des revendications 8 à 10, dans lequel la région centrale inclut un pourcentage plus élevé de nucléotides avec la modification 2' par rapport aux régions flanquantes.

13. Inhibiteur d'ARNmi selon l'une quelconque des revendications 1, ou de 3 à 12, dans lequel lesdites région flanquante 3' et la région flanquante 5' comprennent au plus environ 70% de nucléotides pyrimidiniques.

14. Procédé d'inhibition d'un chemin d'ARNi dans une cellule de façon à inhiber un gène cible étant mis sous silence par une séquence d'ARN cible, le procédé comprenant les étapes consistant à :
fournir une cellule capable d'exprimer la séquence d'ARN cible ;
introduire un inhibiteur d'ARNmi selon l'une quelconque des revendications 1 à 13 ou 15 dans la cellule ; et
maintenir la cellule dans des conditions dans lesquelles la mise sous silence du gène cible par l'ARNmi cible est inhibée, ledit procédé n'étant pas un procédé destiné au traitement du corps humain par chirurgie ou thérapie ou une méthode de diagnostique pratiqué sur le corps humain ou animal.

15. Inhibiteur d'ARNmi comprenant :
un premier oligonucléotide comprenant une région centrale possédant des extrémités 3' et 5', et possédant une séquence centrale qui est comprise entre environ 6 et environ 37 nucléotides et possédant au moins une complémentarité d'environ 80% avec une séquence d'ARN cible, la région centrale étant à simple brin, et la séquence d'ARN cible étant capable de mettre sous silence un gène cible et la séquence d'ARN cible étant choisie parmi le groupe constitué d'une région de brin entrant RISC d'un ARNmi, une région de pré-ARNmi, et une région mature et des régions bordant la région mature du pri-ARNmi ;
une région flanquante 5' qui comprend une duplexe avec une première séquence activatrice qui comprend 8 et jusqu'à 35 nucléotides en longueur, et
une région flanquante 3' qui comprend une duplexe avec une seconde séquence activatrice qui comprend 8 et jusqu'à 35 nucléotides en longueur.
